(19) Europäisches Patentamt
European Patent Office
Office européen des brevets

(11) Veröffentlichungsnummer: **0 121 499**
**A2**

(12) # EUROPÄISCHE PATENTANMELDUNG

(21) Anmeldenummer: 84810145.7

(22) Anmeldetag: 26.03.84

(51) Int. Cl.[3]: **C 07 D 501/20**
A 61 K 31/545, C 07 D 277/48
C 07 D 263/48

(30) Priorität: 31.03.83 CH 1790/83
30.12.83 CH 6986/83

(43) Veröffentlichungstag der Anmeldung:
10.10.84 Patentblatt 84/41

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Schweizer, Ernst, Dr.
Hollenweg 59
CH-4144 Arlesheim(CH)

(72) Erfinder: Frei, Jörg, Dr.
Buechring 36
CH-4434 Hölstein(CH)

(54) **7Beta-Acylamido-3-cephem-4-carbonsäureverbindungen, Verfahren zu ihrer Herstellung, pharmazeutische Präparate, welche diese Verbindungen enthalten, und Verwendung von letzteren.**

(57) Verbindungen der Formel:

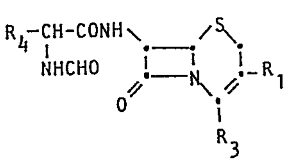

(I) ,

worin

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe, eine Azido- oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt,

Hydrate und Salze von diesen Verbindungen besitzen antibiotische Eigenschaften. Die neuen Verbindungen können z.B. in Form von pharmazeutischen Präparaten zur Behandlung von Infektionen verwendet werden.

EP 0 121 499 A2

CIBA-GEIGY AG
Basel (Schweiz)

4-14374/1÷2/+

# 7β-Acylamido-3-cephem-4-carbonsäureverbindungen, Verfahren zu ihrer Herstellung, pharmazeutische Präparate, welche diese Verbindungen enthalten, und Verwendung von letzteren

Die vorliegende Erfindung betrifft neue 7β-Acylamido-3-cephem-4-carbonsäure-Verbindungen, Verfahren zu ihrer Herstellung, pharmazeutische Präparate, welche diese Verbindungen enthalten, die Verwendung dieser Verbindungen als Arzneimittel und zur Herstellung von pharmazeutischen Präparaten sowie neue Zwischenprodukte und Verfahren zu ihrer Herstellung.

Die vorliegende Erfindung betrifft 7β-Acylamido-3-cephem-4-carbonsäure-Verbindungen der Formel

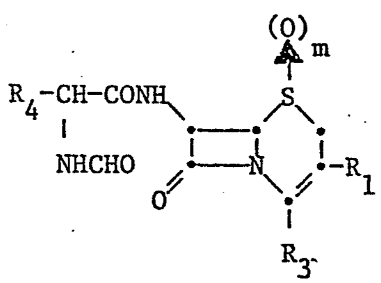

(I) ,

worin m null, eins oder zwei ist,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe, eine Azido- oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt,

sowie Stereoisomere, Mischungen von diesen Stereoisomeren, Hydrate und Salze davon, Verfahren zu ihrer Herstellung, pharmazeutische Präparate, welche diese Verbindungen enthalten, und die Verwendung dieser Verbindungen als Arzneimittel oder zur Herstellung von pharmazeutischen Präparaten.

In der Beschreibung der vorliegenden Erfindung bedeutet der Ausdruck "Nieder", der im Zusammenhang mit Gruppen oder Resten, z.B. Niederalkyl, Niederalkylen, Niederalkoxy, Niederalkanoyl etc., verwendet wird, dass die so bezeichneten Gruppen oder Reste, falls nicht ausdrücklich anders definiert, bis einschliesslich 7 und bevorzugt bis einschliesslich 4 C-Atome enthalten.

In Verbindungen der Formel I ist m in erster Linie null. Falls m eins ist, kann die 1-Oxidogruppe in α- oder β-Stellung stehen. Es kann auch ein Gemisch von Verbindungen der Formel I mit der 1-Oxidogruppe in beiden Stellungen vorliegen.

Das 2-Kohlenstoffatom der 7β-Seitenkette mit der substituierten Aminogruppe -NHCHO und dem Rest $R_4$ hat die R- oder S-Konfiguration. Von der Formel I sind die 2R-, 2S- und die 2R,S-Verbindungen umfasst, wobei die 2S-Verbindungen bevorzugt sind.

Niederalkyl $R_1$ enthält 1 - 4 C-Atome und ist beispielsweise Aethyl, Propyl, Butyl oder insbesondere Methyl.

Niederalkenyl $R_1$ enthält 1 - 4 C-Atome und ist beispielsweise Vinyl oder Allyl.

Niederalkoxy $R_1$ enthält 1 - 4 C-Atome und ist beispielsweise Aethoxy, Propoxy, Butoxy oder insbesondere Methoxy.

Halogen $R_1$ ist Fluor, Brom, Jod oder bevorzugt Chlor.

Verestertes Hydroxy oder Mercapto $R_2$ ist z.B. durch eine aliphatische Carbonsäure, eine durch Acyl, z.B. Niederalkanoyl, z.B. Acetyl, substituierte, aliphatische Carbonsäure, die Carbaminsäure oder eine substituierte Carbaminsäure veresterte Hydroxy- oder Mercaptogruppe und ist beispielsweise Niederalkanoyloxy, z.B. Formyloxy oder Acetoxy, Niederalkanoylniederalkanoyloxy, z.B. Acetacetoxy, Carbamoyloxy oder substituiertes Carbamoyloxy bzw. Niederalkanoylthio, z.B. Acetylthio, Carbamoylthio oder substituiertes Carbamoylthio.

Carbamoyloxy oder Carbamoylthio $R_2$ kann beispielsweise durch Niederalkyl, z.B. Methyl oder Aethyl, oder durch Halogen, z.B. Chlor, oder Niederalkanoyloxy, z.B. Acetoxy, substituiertes Niederalkyl, z.B. 2-Chloräthyl oder 2-Acetoxyäthyl substituiert sein und ist beispielsweise Methyl- oder Dimethylcarbamoyloxy, Aethyl- oder Diäthylcarbamoyloxy, 2-Chloräthylcarbamoyloxy, 2-Acetoxyäthylcarbamoyloxy bzw. Methyl- oder Dimethylcarbamoylthio.

Veräthertes Hydroxy oder Mercapto $R_2$ ist z.B. durch einen aliphatischen Kohlenwasserstoffrest veräthert, und ist beispielsweise Niederalkoxy mit 1-4 C-Atomen, z.B. Methoxy oder Aethoxy, oder Niederalkylthio mit 1-4 C-Atomen, z.B. Methylthio.

Veräthertes Mercapto $R_2$ ist vorzugsweise unsubstituiertes oder substituiertes, monocyclisches, aromatisches oder bicyclisches, aromatisches oder partiell gesättigtes Heterocyclylthio, welches mindestens ein C-Atom enthält und mit einem seiner C-Atome mit dem Schwefel verbunden ist, z.B. monocyclisches, aromatisches, fünf oder sechs Ringatome enthaltendes Heterocyclylthio mit 1-4 Stickstoffatomen oder mit einem Sauerstoff- oder Schwefelatom und gegebenenfalls 1-3 Stickstoffatomen oder bicyclisches, aromatisches, fünf oder sechs Atome pro Ring enthaltendes Heterocyclylthio mit 1-5 Stickstoffatomen und gegebenenfalls einem Sauerstoff- oder Schwefelatom.

Der Heterocyclus in der Heterocyclylthiogruppe $R_2$ ist insbesondere unsubstituiertes oder substituiertes, monocyclisches, aromatisches, fünf- oder sechsgliedriges Diaza-, Triaza-, Tetraza-, Thiaza-, Thiadiaza-, Thia-, Oxaza- oder Oxadiazacyclyl oder unsubstituiertes oder substituiertes bicyclisches, aromatisches, fünf oder sechs Ringatome pro Ring enthaltendes Aza-, Diaza-, Triaza-, Tetraza-, Pentaza-, Thiaza- oder Oxazabicyclyl.

Substituenten dieser Heterocyclen sind beispielsweise Niederalkyl, z.B. Aethyl, n-Propyl, Isopropyl, n-Butyl, Isobutyl oder tert-Butyl, insbesondere Methyl, welches beispielsweise durch Hydroxy, verestertes Hydroxy, z.B. Niederalkanoyloxy, z.B. Acetoxy, oder Halogen, z.B. Fluor oder Chlor, Niederalkylphosphonyl, z.B. Methyl- oder Aethylphosphonyl, Diniederalkylphosphonyl, z.B. Dimethyl- oder Diäthylphosphonyl, Carboxy, Sulfo, verestertes Carboxy, z.B. Niederalkoxycarbonyl, z.B. Aethoxycarbonyl, Sulfoamino, Sulfamoyl, Amino, Niederalkylamino, z.B. Methyl- oder Aethylamino, Diniederalkylamino, z.B. Dimethylamino oder Diäthylamino, Acylamino, z.B. Niederalkanoylamino, z.B. Acetylamino, oder durch Carboxy oder Halogen, z.B. Chlor, substituiertes Niederalkanoylamino, z.B. Carboxyacetylamino oder Chloracetylamino, Cyan, sowie Tetrazolyl, z.B. 1H-Tetrazol-5-yl, substituiert sein kann.

Solche substituierten Niederalkylreste sind beispielsweise Hydroxyniederalkyl, z.B. Hydroxymethyl (an ein C-Atom gebunden) oder 2-Hydroxyäthyl, Acetoxyniederalkyl, z.B. Acetoxymethyl oder 2-Acetoxyäthyl, Halogenniederalkyl, z.B. Chlormethyl, 2-Chloräthyl, 2,2,2-Trichloräthyl oder Trifluormethyl, Niederalkylphosphononiederalkyl, z.B. Aethylphosphonomethyl, Diniederalkylphosphononiederalkyl, z.B. Diäthylphosphonomethyl, Carboxyniederalkyl, z.B. Carboxymethyl oder 2-Carboxyäthyl, Sulfoniederalkyl, z.B. Sulfomethyl oder 2-Sulfoäthyl, Niederalkoxycarbonylniederalkyl, z.B. Aethoxycarbonylmethyl oder 2-Aethoxycarbonyläthyl, Sulfamoylniederalkyl, z.B. Sulfamyolmethyl

oder 2-Sulfamoyläthyl, Aminoniederalkyl, z.B. Aminomethyl (an ein C-Atom gebunden) oder 2-Aminoäthyl, Niederalkylaminoniederalkyl, z.B. 2-Methylaminoäthyl, Diniederalkylaminoniederalkyl, z.B. Dimethylaminomethyl oder 2-Dimethylaminoäthyl, Niederalkanoylaminoniederalkyl, z.B. 2-Acetylaminoäthyl, Carboxyniederalkanoylaminoniederalkyl, z.B. 3-Carboxypropionylaminoäthyl oder 2-Carboxyacetylaminoäthyl, oder Halogenniederalkanoylaminoniederalkyl, z.B. 3-Chlorpropionylaminoäthyl oder 2-Chloracetylaminoäthyl, sowie Tetrazolylniederalkyl, z.B. 1H-Tetrazol-5-ylmethyl oder 2-(1H-Tetrazol-5-yl)-äthyl.

Weitere Substituenten des Heterocyclus sind Niederalkenyl, z.B. Vinyl oder Allyl, Aryl, z.B. Phenyl, oder Halogen, z.B. Fluor, Chlor oder Brom, sowie an ein C-Atom des Heterocyclus gebundenes Amino, substituiertes Amino, z.B. durch Niederalkyl, z.B. Methyl oder Aethyl, mono- oder disubstituiertes Amino, z.B. Methylamino oder Dimethylamino, Acylamino, z.B. Niederalkanoylamino, z.B. Acetylamino, oder Niederalkylsulfonylamino, z.B. Mesylamino, oder durch Halogen, z.B. Chlor, oder Carboxy substituiertes Niederalkanoylamino, z.B. 3-Chlorpropionylamino oder 3-Carboxypropionylamino, Nitro, Hydroxy, Niederalkoxy, z.B. Methoxy oder Aethoxy, Carboxy, verestertes Carboxy, z.B. Niederalkoxycarbonyl, z.B. Methoxycarbonyl oder Aethoxycarbonyl, amidiertes Carbonyl, z.B. Carbamoyl, Mono- oder Diniederalkylcarbamoyl, z.B. Methylcarbamoyl oder Dimethylcarbamoyl, oder Cyan, sowie Oxo oder Oxido.

Unsubstituiertes oder substituiertes, monocyclisches, aromatisches, fünfgliedriges Heterocyclylthio ist vorzugsweise Imidazolylthio, z.B. Imidazol-2-ylthio, Triazolylthio, z.B. 1H-1,2,3-Triazol-4-ylthio oder 1H-1,2,4-Triazol-3-ylthio, durch Niederalkyl, z.B. Methyl, und/oder Phenyl substituiertes Triazolylthio, z.B. 1-Methyl-1H-1,2,3-triazol-4-ylthio, 5-Methyl-1H-1,2,4-triazol-3-ylthio oder 4,5-Dimethyl-1,2,4-

triazol-3-ylthio, Tetrazolylthio, z.B. 1H-Tetrazol-5-ylthio, durch Niederalkyl, z.B. Methyl oder Aethyl, oder substituiertes Niederalkyl, z.B. Aethyl- oder Diäthylphosphonomethyl, Carboxymethyl, 2-Carboxyäthyl, Sulfomethyl, 2-Sulfoäthyl, 2-Dimethylaminoäthyl, Cyanmethyl, oder Tetrazolylmethyl substituiertes Tetrazolylthio, z.B. 1-Methyl-1H-tetrazol-5-ylthio, 1-Aethyl- oder 1-Diäthylphosphonylmethyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Cyanmethyl-1H-tetrazol-5-ylthio oder 1-(1H-Tetrazol-5-ylmethyl)-1H-tetrazol-5-ylthio, Thiazolylthio, z.B. Thiazol-2-ylthio, oder durch Carboxyniederalkyl, z.B. Carboxymethyl, und/oder Niederalkyl, z.B. Methyl, substituiertes Thiazolylthio, z.B. 4-Methyl-5-carboxymethylthiazol-2-ylthio oder 4,5-Dimethylthiazol-2-ylthio, Isothiazolylthio, z.B. Isothiazol-3-, -4- oder -5-ylthio, Thiadiazolylthio, z.B. 1,2,3-Thiadiazol-4- oder -5-ylthio, 1,3,4-Thiadiazol-2-ylthio, 1,2,4-Thiadiazol-3- oder -5-ylthio oder 1,2,5-Thiadiazol-3-ylthio, durch Niederalkyl, z.B. Methyl, substituiertes Thiadiazolylthio, z.B. 2-Methyl-1,3,4-thiadiazol-5-ylthio, Thiatriazolylthio, z.B. 1,2,3,4-Thiatriazol-5-ylthio, Oxazolylthio, z.B. Oxazol-2-, -4- oder -5-ylthio, durch Niederalkyl, z.B. Methyl, substituiertes Oxazolylthio, z.B. 4-Methyloxazol-5-ylthio, durch Niederalkyl, z.B. Methyl, substituiertes Isoxazolylthio, z.B. 3-Methyl-5-isoxazolylthio, Oxadiazolylthio z.B. 1,2,4-Oxadiazol-5-ylthio, oder durch Niederalkyl, z.B. Methyl, substituiertes Oxadiazolylthio, z.B. 2-Methyl-1,3,4-oxadiazol-5-ylthio.

Unsubstituiertes oder substituiertes, monocyclisches, aromatisches, sechsgliedriges Heterocyclylthio ist vorzugsweise 5,6-Dioxotetrahydro-as-triazinylthio welches durch Niederalkyl, z.B. Methyl, Carboxyniederalkyl, z.B. Carboxymethyl oder durch Sulfoniederalkyl, z.B. Sulfomethyl, substituiert sein kann, z.B. 1- oder 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, 1- oder 2-Carboxymethyl-5,6-dioxo-1,2,5,6-tetrahydro-as-triazin-3-ylthio, 4-Carboxymethyl-5,6-dioxo-1,4,5,6-tetrahydro-as-triazin-3-ylthio, 1- oder 2-Sulfomethyl-5,6-dioxo-1,2,5,6-tetrahydro-as-triazin-3-ylthio oder 4-Sulfomethyl-5,6-dioxo-1,4,5,6-tetrahydro-as-triazin-3-ylthio und Tautomere davon.

Unsubstituiertes oder substituiertes, bicyclisches, aromatisches fünf- oder sechs Ringatome pro Ring enthaltendes Heterocyclylthio ist vorzugsweise Indolylthio, durch Niederalkyl, z.B. Methyl, substituiertes Indolylthio, z.B. Indol-2-ylthio oder N-Methylindol-2-ylthio, Isoindolylthio, z.B. Isoindol-2-ylthio, Chinolylthio, z.B. Chinol-2-, -4- oder -8-ylthio, Benzimidazolylthio, durch Niederalkyl, z.B. Methyl, oder Carboxyniederalkyl, z.B. Carboxymethyl, substituiertes Benzimidazolylthio, z.B. 1-Methyl-, 1-Carboxymethyl- oder 1-(2-Carboxyäthyl)-benzimidazol-2-ylthio, Benztriazolylthio, durch Niederalkyl, z.B. Methyl, oder Carboxyniederalkyl, z.B. Carboxymethyl, substituiertes Benztriazolylthio, z.B. 1-Methyl- oder 1-Carboxymethyl-1H-benzo[d]-triazol-5-ylthio, Tetrazolopyridazinylthio oder durch Niederalkyl, z.B. Methyl oder Aethyl, Carboxy, Carboxyniederalkyl, z.B. Carboxymethyl, Carbamoyl, Niederalkylcarbamoyl, z.B. Methylcarbamoyl, Diniederalkylcarbamoyl, z.B. Dimethylcarbamoyl, Amino, Niederalkylamino, z.B. Methylamino, Diniederalkylamino, z.B. Dimethylamino oder Diäthylamino, substituiertes Tetrazolopyridazinylthio, z.B. 8-Methyl-, 8-Aethyl-, 8-Carboxy-, 8-Carboxymethyl-, 8-(2-Carboxyäthyl)-, 8-Carbamoyl-, 8-Methylcarbamoyl-, 8-Dimethylcarbamoyl-, 8-Amino-, 8-Dimethylamino- oder 8-Diäthylaminotetrazolo[1,5-b]pyridazin-6-ylthio.

Eine Ammoniogruppe $R_2$ ist von einer tertiären, aliphatischen oder einer aromatischen Stickstoffbase abgeleitet, indem die betreffende Base mit ihrem Stickstoffatom an die in 3-Stellung des Cephemgerüsts befindliche Methylengruppe gebunden ist.

Eine von einer tertiären, aliphatischen Stickstoffbase abgeleitete Ammoniogruppe $R_2$ ist z.B. Triniederalkylammonio, z.B. Trimethyl- oder Triäthylammonio.

Eine von einer aromatischen Stickstoffbase abgeleitete Ammoniogruppe $R_2$ wird z.B. von einem unsubstituierten oder substituierten, aromatischen Heterocyclus mit 1-3 Stickstoffatomen und insgesamt 5 oder 6 Ringatomen gebildet und ist beispielsweise 1-Pyrazolio, durch Niederalkyl, z.B. Methyl oder Aethyl, Niederalkenyl, z.B. Vinyl oder Allyl, Carboxyniederalkyl, z.B. Carboxymethyl, Niederalkoxycarbonylniederalkyl, z.B. Methoxycarbonylmethyl, Sulfoniederalkyl, z.B. Sulfomethyl, Aminoniederalkyl, z.B. 2-Aminoäthyl, oder Diniederalkylaminoniederalkyl, z.B. 2-Dimethylaminoäthyl, in 2-Stellung substituiertes 1-Pyrazolio, z.B. 2-Methyl- oder 2-Aethyl-1-pyrazolio, 2-Allyl- oder 2-Vinyl-1-pyrazolio, 2-Sulfomethyl-1-pyrazolio, 2-(2-Aminoäthyl)-1-pyrazolio oder 2-(2-Dimethylaminoäthyl)-1-pyrazolio, 1-Triazolio, durch Niederalkyl, z.B. Methyl oder Aethyl, Carboxyniederalkyl, z.B. Carboxymethyl oder Diniederalkylaminoniederalkyl, z.B. 2-Dimethylaminoäthyl, in 3-Stellung substituiertes 1-Triazolio, z.B. 3-Methyl-1-triazolio, 3-Carboxymethyl-1-triazolio oder 3-(2-Dimethylaminoäthyl)-1-triazolio, Pyrazinio, Pyrimidinio, Pyridazinio oder durch Niederalkyl, z.B. Methyl, Halogen, z.B. Chlor, Amino, Niederalkylamino, z.B. Methylamino, Diniederalkylamino, z.B. Dimethylamino, Hydroxyniederalkylamino, z.B. 2-Hydroxyäthylamino, oder Dihydroxyniederalkylamino, z.B. Di-(2-hydroxyäthyl)-amino, substituiertes Pyrazinio, Pyrimidinio oder Pyridazinio, z.B. 2- oder 3-Methyl-, 2- oder 3-Chlor-, 2- oder 3-Amino- oder 2- oder 3-Di-(2-hydroxyäthyl)-aminopyrazinio, 2-Methylpyrimidinio, 3-Methylpyridazinio oder 3-Chlorpyridazinio.

Die von einem aromatischen Heterocyclus mit 1-3 Stickstoffatomen und insgesamt 5 oder 6 Ringatomen abgeleitete Ammoniogruppe $R_2$ ist vorzugsweise Pyridinio oder durch Niederalkyl,

z.B. Methyl, Carbamoyl, Niederalkylcarbamoyl, z.B. Methylcarbamoyl, Hydroxyniederalkyl, z.B. Hydroxymethyl, Niederalkoxyniederalkyl, z.B. Methoxymethyl, Cyanniederalkyl, z.B. Cyanmethyl, Carboxyniederalkyl, z.B. Carboxymethyl, Sulfoniederalkyl, z.B. 2-Sulfoäthyl, Carboxyniederalkenyl, z.B. 2-Carboxyvinyl, Carboxyniederalkylthio,z.B. Carboxymethylthio, Thiocarbamoyl, Halogen, z.B. Brom oder Chlor, Carboxy, Sulfo oder Cyan mono- oder disubstituiertes Pyridinio, z.B. Niederalkylpyridinio, z.B. 2-, 3- oder 4-Methylpyridinio oder 2-, 3- oder 4-Aethylpyridinio, Carbamoylpyridinio, z.B. 3- oder 4-Carbamoylpyridinio, Niederalkylcarbamoylpyridinio, z.B. 3- oder 4-Methylcarbamoylpyridinio, Diniederalkylcarbamoylpyridinio, z.B. 3- oder 4-Dimethylcarbamoylpyridinio, Hydroxyniederalkylpyridinio, z.B. 3- oder 4-Hydroxymethylpyridinio, Niederalkoxyniederalkylpyridinio, z.B. 4-Methoxymethylpyridinio, Cyanniederalkylpyridinio, z.B. 3-Cyanmethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 3- oder 4-Carboxypyridinio, Sulfopyridinio, z.B. 3- oder 4-Sulfopyridinio, Cyanpyridinio, z.B. 3-Cyanpyridinio, Carboxyniederalkyl-carbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanhalogenmethylpyridinio, z.B. 3-Cyan-4-trifluormethylpyridinio, Amino-carboxypyridinio, z.B. 2-Amino-3-carboxypyridinio, oder durch Niederalkoxyiminoniederalkyl, z.B. Methoxyiminomethyl oder 1-Methoxyiminoäthyl, monosubstituiertes Pyridinio, z.B. 3- oder 4-Methoxyiminomethylpyridinio oder 3- oder 4-(1-Methoxyiminoäthyl)-pyridinio.

Die von einer aromatischen Stickstoffbase abgeleitete Ammoniogruppe $R_2$, z.B. Pyridinio, kann auch durch Niederalkylen, z.B. Aethylen,

1,3-Propylen oder 1,4-Butylen, oder Niederalkenylen, z.B. 1,3-Propenylen oder 1,4-But-1- oder But-2-enylen, disubstituiert sein. Diese Substituenten können ihrerseits durch Hydroxy, Niederalkoxy, z.B. Methoxy, oder Halogen, z.B. Chlor substituiert sein. Eine solche Ammoniogruppe ist beispielsweise 3,4- und bevorzugt 2,3-Cyclobuteno-, -Cyclopenteno- oder -Cyclohexenopyridinio.

Die von einer aromatischen Stickstoffbase abgeleitete Ammoniogruppe $R_2$, z.B. Pyridinio, kann auch mit einem fünf- oder sechsgliedrigen, gesättigten oder ungesättigten, z.B. partiell gesättigten oder aromatischen, Heterocyclus, z.B. mit Tetrahydropyran oder -furan, Dihydropyran oder -furan, Pyran, Furan oder Thiophen, sowie mit Benzol kondensiert. sein.

Mit Benzol kondensiertes Pyridinio ist z.B. Chinolinio oder Isochinolinio, welches durch Amino, Niederalkylamino, z.B. Methylamino, Diniederalkylamino, z.B. Dimethylamino, Hydroxy, Niederalkoxy, z.B. Methoxy, Halogen, z.B. Chlor, Niederalkyl, z.B. Methyl, Cyan, Trifluormethyl, Sulfo, Sulfamoyl, Carboxy, Niederalkoxycarbonyl, z.B. Aethoxycarbonyl, Hydroxyniederalkyl, z.B. Hydroxymethyl, Niederalkanoyl, z.B. Formyl oder Acetyl, Thiocarbamoyl, Carbamoyl oder Niederalkoxyiminoniederalkyl, z.B. Methoxyiminomethyl oder 1-Methoxyiminoäthyl, substituiert sein kann, z.B. 3-, 4-, 5- oder 7-Aminochinolinio oder -isochinolinio, 3-, 4-, 5- oder 7-Hydroxychinolinio oder -isochinolinio, 3-, 4- oder 5-Methylchinolinio oder -isochinolinio, 3-, 4- oder 5-Carbamoylchinolinio oder Isochinolinio, 3-, 4- oder 5-Methoxyiminochinolinio oder -isochinolinio oder 3-, 4- oder 5-(1-Methoxyiminoäthyl)-chinolinio oder -isochinolinio.

Eine Ammoniogruppe $R_2$ ist in erster Linie Pyridinio oder durch Hydroxyniederalkyl, z.B. Hydroxymethyl, Carboxy, Carboxyniederalkyl, z.B. Carboxymethyl, Halogen, z.B. Chlor oder Brom, Carbamoyl oder Niederalkylen substituiertes sowie mit Benzol kondensiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder

4-Brompyridinio oder 3- oder 4-Carbamoylpyridinio, 2,3-Cyclopentenopyridinio oder Chinolinio.

In einer Gruppe der Formel $-CH=CH-R_2$ hat die verätherte Mercaptogruppe $R_2$ die weiter vorn genannten Bedeutungen, z.B. Heterocyclylthio, und bedeutet bevorzugt durch Niederalkoxy, z.B. Methoxy, substituiertes 5,6-Dioxotetrahydro-as-triazinylthio, z.B. 4-Methoxy-5,6-dioxo-1,4,5,6-tetrahydro-as-triazin-3-ylthio.

Geschütztes Carboxy $R_3$ ist durch eine der im folgenden beschriebenen Carboxyschutzgruppen verestertes Carboxy, insbesondere unter physiologischen Bedingungen spaltbares, verestertes Carboxy.

Eine unter physiologischen Bedingungen spaltbare, veresterte Carboxygruppe $R_3$ ist in erster Linie eine Acyloxyniederalkoxycarbonylgruppe, worin Acyl, z.B. die Acylgruppe einer organischen Carbonsäure, eines Kohlensäuremonoesters oder einer Aminosäure bedeutet, oder worin Acyloxyniederalkoxy, z.B. Acyloxymethoxy, den Rest eines Lactons bildet.

Acyl ist vorzugsweise die Acylgruppe einer gegebenenfalls verzweigten Niederalkancarbonsäure, die Acylgruppe der durch gegebenenfalls verzweigtes Niederalkyl monoveresterten Kohlensäure oder die Acylgruppe eine gegebenenfalls verzweigten α-Aminoniederalkancarbonsäure.

Unter physiologischen Bedingungen spaltbares, verestertes Carboxy $R_3$ ist vorzugsweise Niederalkanoyloxyniederalkoxycarbonyl, z.B. Niederalkanoyloxymethoxycarbonyl oder Niederalkanoyloxyäthoxycarbonyl, z.B. Acetoxymethoxycarbonyl, Pivaloyloxymethoxycarbonyl oder 1-Propionyloxyäthoxycarbonyl, Niederalkoxycarbonyloxyniederalkoxycarbonyl, z.B. 1-Aethoxycarbonyloxyäthoxycarbonyl oder tert-Butoxycarbonyloxymethoxycarbonyl, Aminoniederaklkanoyloxymethoxycarbonyl, insbesondere α-Aminoniederalkanoyloxymethoxycarbonyl, z.B. Glycyloxymethoxycarbonyl, L-Valyloxymethoxycarbonyl oder L-Leucyloxymethoxycarbonyl, ferner

Phthalidyloxycarbonyl, z.B. 2-Phthalidyloxycarbonyl, oder Indanyloxycarbonyl, z.B. 5-Indanyloxycarbonyl.

Heterocyclyl $R_4$ ist mit einem seiner C-Atome an das 2-C-Atom der 7β-Seitenkette gebunden und ist beispielsweise unsubstituiertes oder substituiertes, aromatisches, monocyclisches Oxaza-, Thiaza-, Diaza-, Thiadiaza-, Triaza- oder Tetrazacyclyl, z.B. Oxazolyl, z.B. Oxazol-4-yl, Thiazolyl, z.B. Thiazol-4-yl, Isothiazolyl, z.B. Isothiazol-3- oder -4-yl, Imidazolyl, z.B. Imidazol-4-yl, Thiadiazolyl, z.B. 1,2,4-Thiadiazol-3-yl, 1,2,5-Thiadiazol-3-yl, oder 1,3,4-Thiadiazol-2-yl, Triazolyl, z.B. 1,2,4-Triazol-3-yl, oder Tetrazolyl, z.B. Tetrazol-5-yl.

Substituenten des Heterocyclylrestes $R_4$ sind die gleichen weiter vorn für die Heterocyclylthiogruppe $R_2$ genannten Substituenten.

Heterocyclyl $R_4$ ist bevorzugt Aminooxazolyl, z.B. 2-Aminooxazol-4-yl, Aminothiazolyl, z.B. 2-Aminothiazol-4-yl, Aminoimidazolyl, z.B. 2-Aminoimidazol-4-yl, Aminothiadiazolyl, z.B. 5-Amino-1,2,4-thiadiazol-3-yl, Hydroxythiadiazolyl, z.B. 4-Hydroxy-1,2,5-thiadiazol-3-yl, oder Aminotriazolyl, z.B. 5-Amino-1,2,4-triazol-3-yl.

Die in Verbindungen der Formel I vorhandenen funktionellen Gruppen, insbesondere die Carboxy-, Amino-, Hydroxy- und Sulfogruppen, sind gegebenenfalls durch solche Schutzgruppen (conventional protecting groups) geschützt, die üblicherweise in der Penicillin-, Cephalosporin- und Peptidchemie verwendet werden und bei deren Abspaltung die 2-Formamidogruppe erhalten bleibt. Diese Schutzgruppen sollen die betreffenden funktionellen Gruppen gegen unerwünschte Nebenreaktionen wie Acylierungen, Verätherungen, Veresterungen, Oxydationen, Solvolyse etc. schützen und unter schonenden Reaktionsbedingungen leicht abspaltbar sein, das heisst unter Vermeidung von Spaltung z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I Georg Thieme Verlag, Stuttgart 1974, beschrieben.

Eine Carboxygruppe, z.B. die Carboxygruppe $R_3$, ferner eine in $R_2$ vorhandene Carboxygruppe, ist üblicherweise in veresterter Form geschützt, wobei die Estergruppe unter schonenden Bediungungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine in 1-Stellung verzweigte Niederalkylgruppe verestert ist, ist beispielsweise tert-Niederalkoxycarbonyl, z.B. tert-Butoxycarbonyl, Arylmethoxycarbonyl, mit einem oder zwei Arylresten, worin Aryl vorzugsweise unsubstituiertes oder z.B. durch Niederalkyl, z.B. tert-Niederalkyl, z.B. tert-Butyl, Niederalkoxy, z.B. Methoxy, Hydroxy, Halogen, z.B. Chlor, und/oder Nitro, mono-, di- oder trisubstituiertes Phenyl bedeutet, beispielsweise Benzyloxycarbonyl, durch die genannten Substituenten substituiertes Benyloxycarbonyl, z.B. 4-Nitrobenzyloxycarbonyl oder 4-Methoxybenzyloxycarbonyl, Diphenylmethoxycarbonyl oder die genannten Substituenten substituiertes Diphenylmethoxycarbonyl, z.B. Di-(4-methoxyphenyl)-methoxycarbonyl.

Eine geschützte Carboxygruppe, welche durch eine in 1- oder 2-Stellung durch geeignete Substituenten substituierte Niederalkylgruppe verestert ist, ist beispielsweise 1-Niederalkoxyniederalkoxycarbonyl, z.B. Methoxymethoxycarbonyl, 1-Methoxyäthoxycarbonyl oder 1-Aethoxymethoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, sowie 2-Halogenniederalkoxycarbonyl, z.B. 2,2,2—Trichloräthoxycarbonyl, 2-Bromäthoxycarbonyl oder 2-Jodäthoxycarbonyl.

Eine Carboxygruppe kann auch als organische Silyloxycarbonylgruppe geschützt sein. Eine organische Silyloxycarbonylgruppe ist beispielsweise Triniederalklysilyloxycarbonyl z.B. Trimethylsilyloxycarbonyl. Das Siliciumatom der Silyloxycarbonylgruppe kann auch durch zwei Niederalkylgruppen, z.B. Methylgruppen, und die Carboxygruppe oder Aminogruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine geschützte Carboxygruppe ist bevorzugt tert-Niederalkoxycarbonyl-z.B. tert-Butoxycarbonyl, Benzyloxycarbonyl, 4-Nitrobenzyloxycarbonyl und Diphenylmethoxycarbonyl.

Eine Aminogruppe, z.B. eine in $R_2$ und $R_4$ vorhandene Aminogruppe, kann z.B. in Form einer unter schonenden Bedingungen selektiv spaltbaren Acylamino-, verätherten Mercaptoamino- oder Silylaminogruppe oder als Azidogruppe geschützt sein.

In einer leicht spaltbaren Acylaminogruppe ist Acyl beispielsweise die Acylgruppe von einer organischen Carbonsäure mit bis zu 10 Kohlenstoffatomen, insbesondere einer unsubstituierten oder einer, z.B. durch Halogen oder Aryl, substituierten Niederalkancarbonsäure oder der unsubstituierten oder, z.B. durch Halogen, Niederalkoxy oder Nitro, substituierten Benzoesäure oder eines Kohlensäurehalbesters.

Eine solche Acylgruppe ist beispielsweise Halogenniederalkanoyl, z.B. 2-Halogenacetyl, insbesondere 2-Chlor-, 2-Brom-, 2-Jod-, 2,2,2-Trifluor- oder 2,2,2-Trichloracetyl, Benzoyl oder z.B. durch Halogen, z.B. Chlor, Niederalkoxy, z.B. Methoxy, oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestrs verzweigtes oder in 1- oder 2-Stellung durch geeignete Substituenten substituiertes Niederalkoxycarbonyl.

In 1-Stellung des Niederalkylrests verzweigtes Niederalkoxycarbonyl ist beispielsweise tert-Niederalkoxycarbonyl, z.B. tert-Butoxycarbonyl (BOC), Arylmethoxycarbonyl mit einem oder zwei Arylresten, worin Aryl vorzugsweise unsubstituiertes oder z.B. durch Niederalkyl, insbesondere tert—Niederalkyl, z.B. tert—Butyl, Niederalkoxy, z.B. Methoxy, Hydroxy, Halogen, z.B. Chlor, und/oder Nitro, mono-, di- oder trisubstituiertes Phenyl bedeutet, beispielsweise Diphenylmethoxycarbonyl oder Di-(4-methoxyphenyl)-methoxycarbonyl.

In 1- oder 2-Stellung durch geeignete Substituenten substituiertes Niederalkoxycarbonyl ist beispielsweise Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Silylgruppe durch organische Reste, z.B. Niederalkyl, Phenylniederalkyl oder Phenyl substituiert ist, beispielsweise 2-Triniederalkylsilyläthoxycarbonyl, z.B. 2-Trimethylsilyläthoxycarbonyl oder 2-(Di-n-butylmethylsilyl)-äthoxycarbonyl, oder 2-Triphenylsilyläthoxycarbonyl.

In einer verätherten Mercaptoaminogruppe ist die verätherte Mercaptogruppe in erster Linie Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten

Moleküls der Formel I substituiert sein. Verbindungen mit solchen Schutzgruppen lassen sich z.B. bei Verwendung von Dimethyldichlorsilan als Silylierungsmittel herstellen.

Eine Aminogruppe kann auch in protonierter Form geschützt sein. Als Anionen sind in erster Linie die Anionen von starken anorganischen Säuren, wie von Halogenwasserstoffsäuren, z.B. das Chlor- oder Bromanion, oder von organischen Sulfonsäuren, wie p-Toluolsulfonsäure, geeignet.

Eine geschützte Aminogruppe ist bevorzugt tert—Butoxycarbonylamino (BOC), 4-Nitrobenzyloxycarbonylamino, Diphenylmethoxycarbonylamino, oder 2-Halogenniederalkoxycarbonylamino, z.B. 2,2,2-Trichloräthoxycarbonylamino.

Eine Hydroxygruppe, z.B. eine in $R_2$ vorhandene Hydroxygruppe, kann beispielsweise durch eine Acylgruppe, z.B. durch Halogen,substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, ein organischer Silylrest mit den weiter vorn genannten Substituenten, z.B. Trimethylsilyl oder Dimethyl-n-butylsilyl, ferner eine leicht abspaltbare, veräthernde Gruppe, wie tert.-Niederalkyl, z.B. tert-Butyl, ein oxa- oder ein thiaaliphatischer oder -cycloaliphatischer Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5 - 7 Ringatomen, z.B. 2-Tetrahydrofuryl oder 2-Tetrahydropyranyl, oder ein entsprechendes Thiaanaloges, sowie 1-Phenylniederalkyl, z.B. Benzyl oder Diphenylmethyl, wobei die Phenylreste beispielsweise durch Halogen, z.B. Chlor, Niederalkoxy, z.B. Methoxy, und/oder Nitro substituiert sein können.

Eine Sulfogruppe, z.B. eine in $R_2$ vorhandene Sulfogruppe, ist vorzugsweise durch eine tert-Niederalkylgruppe, z.B. tert-Butyl, oder durch eine Silylgruppe, z.B. durch Triniederalkylsilyl, geschützt. Eine Sulfogruppe kann z.B. durch dieselben Schutzgruppen wie die Carboxygruppen geschützt sein.

Salze sind in erster Linie die pharmazeutisch annehmbaren oder verwendbaren, nicht-toxischen Salze von Verbindungen der Formel I.

Solche Salze werden beispielsweise von den in Verbindungen der Formel I vorhandenen sauren Gruppen, z.B. Carboxy- oder Sulfogruppen, gebildet und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche Ammoniak oder geeigneten organischen Aminen gebildet werden, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen. Solche Basen sind beispielsweise Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethylpiperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

Die in Verbindungen der Formel I vorhandenen basischen Gruppen, z.B. Aminogruppen, können Säureadditionssalze, z.B. mit anorganischen Säuren, z.B. Mineralsäuren, wie Salzsäure, Schwefelsäure oder Phosphorsäure, oder mit geeigneten organischen Carbon- oder Sulfonsäuren, z.B. ungesättigten Carbonsäuren, z.B. Fumarsäure oder Maleinsäure, Hydroxysäuren, z.B. Milchsäure, Weinsäure oder Citronensäure, oder aromatischen Säuren, z.B. Salicylsäure, sowie mit Aminosäuren, wie Arginin und Lysin, bilden.

Liegen in Verbindungen der Formel I mehrere saure Gruppen, z.B. zwei Carboxygruppen, oder mehrere basische Gruppen, z.B. zwei Aminogruppen, vor, können Mono- oder Polysalze gebildet werden. Wenn die Verbindungen der Formel I mindestens eine saure Gruppe, z.B. die freie Carboxygruppe $R_3$, und mindestens eine basische Gruppe, z.B. eine Aminogruppe in $R_4$ besitzen, können diese in Form von inneren Salzen, d.h. in zwitterionischer Form, vorliegen. In Verbindungen der Formel I können eine saure und eine basische Gruppe in Form eines inneren Salzes und zusätzlich saure und/oder basische Gruppen beispielsweise als Säureadditions- und/oder Baseadditionssalz vorliegen.

Zur Isolierung oder Reinigung können auch pharmazeutisch ungeeignete Salze Verwendung finden. Zur therapeutischen Anwendung gelangen nur pharmazeutisch verwendbare, nicht toxische Salze, die deshalb bevorzugt sind.

Die Verbindungen der Formel I, worin die funktionellen Gruppen in freier Form und die Carboxylgruppen gegebenenfalls in physiologisch spaltbarer veresterter Form vorliegen, und ihre pharmazeutisch verwendbaren, nichttoxischen Salze sind wertvolle, antibiotische Wirkstoffe, die insbesondere als antibakterielle Antibiotika verwendet werden können.

Beispielsweise sind sie in vitro gegen grampositive und gramnegative Mikroorganismen, inklusive β-Lactamase produzierende Stämme, z.B. gegen Kokken wie Staphylococcus aureus, Streptococcus pneumoniae, Streptococcus pyogenes und Neisseria gonorrhoeae in Minimalkonzentrationen von ca. 0,001 bis ca. 16 µg/ml, gegen Enterobakterien, z.B. gegen Escherichia coli, Salmonella typhimurium, Klebsiella pneumoniae, Proteus spp., Enterobacter cloacae, Serratia marcescens, Haemophilus influenzae und Pseudomonas aeruginosa, und gegen anaerobe grampositive und gramnegative Bakterien, z.B. Bacteroides fragilis oder Clostridium perfringens, in Minimalkonzentrationen von ca. 0,001 bis ca. 8 µg/ml wirksam.

Im folgenden Versuchsbericht wird anhand von ausgewählten Verbindungen die Wirksamkeit von Verbindungen der Formel I gezeigt.

Versuchsbericht

Testverbindungen:

1. 3-Acetoxymethyl-7β-[(2R,S)-2-(2-aminothiazol-4-yl)-2-formamidoacetamido]-3-cephem-4-carbonsäurenatriumsalz.

2. 3-Carbamoyloxymethyl-7β-[(2S)-2-(2-aminothiazol-4-yl)-2-formamidoacetamido]-3-cephem-4-carbonsäurenatriumsalz.

3. 3-(1-Methyl-1H-tetrazol-5-ylthiomethyl)-7β-[(2R,S)-2-(2-aminothiazol-4-yl)-2-formamidoacetamido]-3-cephem-4-carbonsäurenatriumsalz.

4. 3-(1,2,3-Thiadiazol-5-ylthiomethyl)-7β-[(2S)-2-(2-aminothiazol-4-yl)-2-formamidoacetamido]-3-cephem-4-carbonsäurenatriumsalz.

II. Experimentelles:

A. Die antibiotische Aktivität der Testverbindung in vitro wurde durch die Agarverdünnungsmethode nach Ericsson, H.M., und Sherris, S.C., 1971, Acta Path. Microb. Scand. Section B, Suppl. No. 217, Bd. 1 - 90, in DST Agar ermittelt. Die gefundenen, das Wachstum der Testorganismen noch hemmenden Minimalkonzentrationen (MIC = minimum inhibitory concentration) werden in Mikrogramm pro Mililiter (μg/ml) für die geprüften Verbindungen in der Tabelle 1 angegeben.

III. Versuchsergebnisse:

Tabelle: Antibiotische Aktivität (in vitro)

| Testverbindung | MIC [μg/ml] | | | | |
|---|---|---|---|---|---|
| | Neisseria gonorrhoeae 1317/4 | Escherichia coli 205 | Klebsiella pneumoniae 327 | Proteus mirabilis 774 | Staphylococcus aureus 10B |
| 1 | 0,001 | 0,002 | 0,002 | 0,01 | 1 |
| 2 | 0,001 | 0,005 | 0,005 | 0,01 | 1 |
| 3 | 0,001 | 0,002 | 0,01 | 0,01 | 2 |
| 4 | 0,002 | 0,002 | 0,002 | 0,002 | 0,2 |

Verbindungen der Formel I, worin die funktionellen Gruppen geschützt sind, werden als Ausgangsmaterialien zur Herstellung von Verbindungen der Formel I verwendet, worin funktionelle Gruppen in freier Form oder in physiologisch spaltbarer Form vorliegen.

Die vorliegende Erfindung betrifft bevorzugt solche Verbindungen der Formel I, worin funktionelle Gruppen in freier Form oder in physiologisch spaltbarer geschützter Form vorliegen, und deren pharmazeutisch annehmbare Salze, da hauptsächlich diese Verbindungen die angegebene Wirksamkeit besitzen und für den angegebenen Zweck verwendet werden können.

Die Erfindung betrifft bevorzugt Verbindungen der Formel I, worin m null ist, $R_1$ Wasserstoff, Niederalkyl, Niederalkoxy, Halogen oder eine Gruppe der Formel $-CH_2-R_2$, worin $R_2$ Niederalkanoyloxy, Carbamoyloxy, unsubstituiertes oder substituiertes, monocyclisches, aromatisches oder bicyclisches, aromatisches oder partiell gesättigtes Heterocyclylthio, welches mindestens ein C-Atom enthält und mit einem seiner C-Atome mit dem Schwefel verbunden ist, Azido oder von einer tertiären, aliphatischen oder von einer aromatischen Stickstoffbase abgeleitetes Ammonio bedeutet, $R_3$ Carboxy oder unter physiologischen Bedingungen spaltbares, verestertes Carboxy und $R_4$ unsubstituiertes oder substituiertes, monocyclisches, aromatisches Oxaza-, Thiaza-, Diaza-, Thiadiaza-, Triaza-, oder Tetrazacyclyl darstellt, sowie pharmazeutisch annehmbare Salze von solchen Verbindungen mit salzbildenden Gruppen.

Die Erfindung betrifft insbesondere Verbindungen der Formel I, worin m null ist und $R_1$ Wasserstoff, Niederalkyl, z.B. Methyl, Niederalkoxy, z.B. Methoxy, Halogen, z.B. Chlor, oder eine Gruppe der Formel $-CH_2-R_2$, worin $R_2$ Niederalkanoyloxy, z.B. Acetoxy, Carbamoyloxy, unsubstituiertes oder substituiertes, monocyclisches, aromatisches, fünf oder sechs Ringatome enthaltendes Heterocyclylthio mit 1-4 Stickstoffatomen oder mit einem Sauerstoff- oder Schwefelatom und gegebenenfalls 1-3 Stickstoffatomen oder bicyclisches, aromatisches, fünf oder sechs Atome pro Ring enthaltendes Heterocyclylthio mit 1-5 Stickstoffatomen und gegebenenfalls einem Sauerstoff- oder Schwefelatom, Azido oder eine von einem unsubstituierten oder substituierten aromatischen Heterocyclus mit 1-3 Stickstoffatomen und insgesamt 5 oder 6 Ringatomen gebildete Ammoniogruppe bedeutet, $R_3$ Carboxy oder Acyloxyniederalkoxycarbonyl und $R_4$ unsubstituiertes oder substituiertes Oxazolyl, Thiazolyl, Isothiazolyl, Imidazolyl, Thiadiazolyl, Triazolyl oder Tetrazolyl darstellt, sowie pharmazeutisch annehmbare Salze von solchen Verbindungen mit salzbildenden Gruppen.

Hervorzuheben sind Verbindungen der Formel I, worin m null ist und $R_1$ Wasserstoff oder eine Gruppe der Formel $-CH_2-R_2$, worin $R_2$ Niederalkanoyloxy, z.B. Acetoxy, Carbamoyloxy, Diaza-, Triaza-, Tetraza-, Thiaza-, Thiadiaza-, Oxaza- oder Oxadiazacyclylthio, welche durch Niederalkyl z.B. Methyl, Diniederalkylaminoniederalkyl, z.B. Dimethylaminomethyl oder 2-Dimethylaminoäthyl, Sulfoniederalkyl, z.B. Sulfomethyl oder 2-Sulfoäthyl, Carboxyniederalkyl, z.B. Carboxymethyl oder 2-Carboxyäthyl, Carbamoyl, Cyanniederalkyl, z.B. Cyanmethyl, Tetrazolylniederalkyl, z.B. 1H-Tetrazol-5-ylmethyl, oder durch Oxo substiuiert sein können, Azido, Pyridinio, durch Niederalkyl, z.B. Methyl, Carbamoyl, Niederalkylcarbamoyl, z.B. Methylcarbamoyl, Hydroxyniederalkyl, z.B. Hydroxymethyl, Niederalkoxyniederalkyl, z.B. Methoxymethyl, Cyanniederalkyl, z.B. Cyanmethyl, Carboxyniederalkyl, z.B. Carboxymethyl, Sulfoniederalkyl, z.B. 2-Sulfoäthyl, Carboxyniederalkenyl, z.B. 2-Carboxyvinyl, Carboxyniederalkylthio, z.B. Carboxymethylthio, Thiocarbamoyl, Halogen, z.B. Brom oder Chlor, Carboxy, Sulfo oder Cyan mono- oder disubstiuiertes Pyridinio, durch Niederalkoxyiminoniederalkyl, z.B. Methoxyiminomethyl oder 1-Methoxyiminoäthyl, oder Niederalkylen, z.B. Aethylen, 1,3-Propylen oder 1,4-Butylen, monosubstituiertes Pyridinio oder mit Benzol kondensiertes Pyridinio, $R_3$ Carboxy, Niederalkanoyloxyniederalkoxycarbonyl, z.B. Niederalkanoyloxymethoxycarbonyl, oder Niederalkanoyloxyäthoxycarbonyl, z.B. Acetoxymethoxycarbonyl, Pivaloyloxymethoxycarbonyl oder 1-Propionyloxyäthoxycarbonyl, Niederalkoxycarbonyloxyniederalkoxycarbonyl, z.B. 1-Aethoxycarbonyloxyäthoxycarbonyl oder tert-Butoxycarbonyl-oxymethoxycarbonyl, $R_4$ Aminooxazolyl, z.B. 2-Aminooxyazol-4-yl, Aminothiazolyl, z.B. 2-Aminothiazol-4-yl, Aminothiadiazolyl, z.B. 5-Amino-1,2,4-thiadiazol-3-yl, Hydroxythiadiazolyl, z.B. 4-Hydroxy-1,2,5-thiadiazol-3-yl, oder Aminotriazolyl, z.B. 5-Amino-1,2,4-triazol-3-yl, bedeutet, und pharmazeutisch verwendbare Salze von solchen Verbindungen.

Die vorliegende Erfindung betrifft hauptsächlich Verbindungen der Formel I, worin m null ist und $R_1$ Wasserstoff oder eine Gruppe der Formel $-CH_2-R_2$, worin $R_2$ Niederalkanoyloxy, z.B. Acetoxy, Carbamoyloxy, Imidazolylthio, z.B. Imidazol-2-ylthio, Triazolylthio, z.B. 1H-1,2,3-Triazol-4-ylthio oder 1H-1,2,4-Triazol-3-ylthio, durch Niederalkyl, z.B. Methyl, substituiertes Triazolylthio, z.B. 1-Methyl-1H-1,2,3-triazol-4-ylthio, 5-Methyl-1H-1,2,4-triazol-3-ylthio oder 4,5-Dimethyl-1,2,4,triazol-3-ylthio, Tetrazolylthio, z.B. 1H-Tetrazol-5-ylthio, durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl oder 2-Sulfoäthyl, Carboxyniederalkyl, z.B. Carboxymethyl oder 2-Carboxyäthyl, Carbamoyl, Cyanniederalkyl oder durch Tetrazolylniederalkyl, z.B. 1H-Tetrazol-5-ylmethyl, substituiertes Tetrazolylthio, z.B. 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Cyanmethyl-1H-tetrazol-5-ylthio, oder 1-(1H-Tetrazol-5-ylmethyl)-1H-tetrazol-5-ylthio, Thiazolylthio, z.B. Thiazol-2-ylthio, oder durch Carboxyniederalkyl, z.B. Carboxymethyl, und/oder Niederalkyl, z.B. Methyl, substituiertes Thiazolylthio, z.B. 4-Methyl-5-carboxymethylthiazol-2-ylthio oder 4,5-Dimethylthiazol-2-ylthio, Isothiazolylthio, z.B. Isothiazol-3-, -4- oder -5-ylthio, Thiadiazolylthio, z.B. 1,2,3-Thiadiazol-4- oder -5-ylthio, 1,3,4-Thiadiazol-2-ylthio, 1,2,4-Thiadiazol-3- oder -5-ylthio oder 1,2,5-Thiadiazol-3-ylthio, durch Niederalkyl, z.B. Methyl, substituiertes Thiadiazolylthio, z.B. 2-Methyl-1,3,4-thiadiazol-5-ylthio,

Thiatriazolylthio, z.B. 1,2,3,4-Thiatriazol-5-ylthio, Oxazolylthio, z.B. Oxazol-2-, -4- oder -5-ylthio, durch Niederalkyl, z.B. Methyl, substituiertes Oxazolylthio, z.B. 4-Methyloxazol-5-ylthio, durch Niederalkyl, z.B. Methyl, substituiertes Isoxazolylthio, z.B. 3-Methylisoxazol-5-ylthio, Oxadiazolylthio, z.B. 1,2,4-Oxadiazol-5-ylthio, oder durch Niederalkyl, z.B. Methyl, substituiertes Oxadiazolylthio, z.B. 2-Methyl-1,3,4-oxadiazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Azido, Pyridinio oder durch Hydroxyniederalkyl, z.B. Hydroxymethyl, Carboxy, Carboxyniederalkyl, z.B. Carboxymethyl, Halogen, z.B. Chlor oder Brom, Carbamoyl oder Niederalkylen, z.B. 1,3-Propylen, substituiertes oder mit Benzol kondensiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio, 3- oder 4-Carbamoylpyridinio, 2,3-Cyclopentenopyridinio oder Chinolinio, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Acetoxymethoxycarbonyl, Pivaloyloxymethoxycarbonyl oder 1-Propionyloxyäthoxycarbonyl, Niederalkoxycarbonyloxyniederalkoxycarbonyl, z.B. 1-Aethoxycarbonyloxyäthoxycarbonyl oder tert-Butoxycarbonyloxymethoxycarbonyl, und $R_4$ Aminooxazolyl, z.B. 2-Aminooxazol-4-yl, Aminothiazolyl, z.B. 2-Aminothiazol-4-yl, Aminothiadiazolyl, z.B. 5-Amino-1,2,4-thiadiazol-3-yl, oder Aminotriazolyl, z.B. 5-Amino-1,2,4-triazol-3-yl, bedeutet, Stereoisomere, Mischungen von diesen Stereoisomeren und pharmazeutisch verwendbare Salze von solchen Verbindungen.

Die vorliegende Erfindung betrifft in erster Linie Verbindungen der Formel I, worin m null ist und $R_1$ Wasserstoff oder eine Gruppe der Formel $-CH_2-R_2$, worin $R_2$ Niederalkanoyloxy, z.B. Acetoxy, Carbamoyloxy, Triazolylthio, z.B. 1H-1,2,3-Triazol-4(5)-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-

ylthio, 1-(2-Dimethylaminoäthyl)-1H-tetrazol-5-ylthio, 1-Sulfomethyl-1H-tetrazol-5-ylthio oder 1-Carboxymethyl-1H-tetrazol-5-ylthio, Thiadiazolylthio, z.B. 1,2,3-Thiadiazol-4- oder -5-ylthio, 1,3,4-Thiadiazol-2-ylthio, 1,2,4-Thiadiazol-3- oder -5-ylthio, oder 1,2,5-Thiadiazol-3-ylthio, durch Niederalkyl, z.B. Methyl, substituiertes 5,6-Dioxo-tetrahydro-as-triazinylthio, z.B. 2-Methyl-5,6-dioxo-1,2,5-6-tetrahydro-as-triazin-3-ylthio oder 4-Methyl-5,6-dioxo-1,4,5,6-tetrahydro-as-triazin-3-ylthio, Azido, Pyridinio oder durch Hydroxyniederalkyl, z.B. Hydroxymethyl, Carboxy, Carboxyniederalkyl, z.B. Carboxymethyl, Halogen, z.B. Chlor oder Brom, Carbamoyl oder Niederalkylen, z.B. 1,3-Propylen, substituiertes oder mit Benzol kondensiertes Pyridinio, z.B. 3- oder 4-Hydroxymethyl-pyridinio, 4-Carboxypyridinio, 3- oder 4-Carboxymethylpyridinio, 3- oder 4-Chlorpyridinio, 3- oder 4-Brompyridinio, 3- oder 4-Carbamoyl-pyridinio, 2,3-Cyclopentenopyridinio oder Chinolinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 1-Propionyloxyäthoxycarbonyl, oder Niederalkoxycarbonyloxyniederalkoxycarbonyl, z.B. 1-Aethoxycarbonyloxyäthoxycarbonyl oder tert-Butoxycarbonyloxymethoxycarbonyl, und $R_4$ Aminooxazolyl, z.B. 2-Aminooxazol-4-yl, Aminothiazolyl, z.B. 2-Aminothiazol-4-yl, oder Aminothiadiazolyl, z.B. 5-Amino-1,2,4-thiadiazol-3-yl bedeutet, Stereoisomere, Mischungen von diesen Stereoisomeren, Hydrate und pharamzeutisch verwendbare Salze von solchen Verbindungen.

Die Erfindung betrifft ganz besonders die in den Beispielen beschriebenen Verbindungen der Formel I, deren pharmazeutisch verwendbare Salze, sowie die dort beschriebenen Ausgangsstoffe und Zwischenprodukte.

Herstellungsverfahren:

Verbindungen der Formel I, worin die Carboxygruppen in freier Form vorliegen oder in physiologisch spaltbarer Form verestert sind, Hydrate und Salze von solchen Verbindungen, die eine salzbildende Gruppe aufweisen, werden beispielsweise hergestellt, indem man

a) in einer Verbindung der Formel

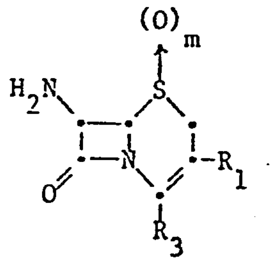

(II),

worin m und $R_1$ die unter Formel I genannten Bedeutungen haben, $R_3$ geschütztes Carboxy bedeutet, eine in $R_1$ vorhandene funktionelle Gruppe geschützt ist und die 7β-Aminogruppe gegebenenfalls durch eine die Acylierungsreaktion erlaubende Gruppe geschützt ist, die 7β-Aminogruppe durch Umsetzung mit einem den Acylrest einer Carbonsäure der Formel

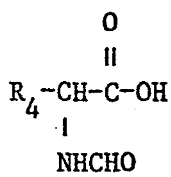

(III) ,

einführenden Acylierungsmittel, worin $R_4$ die unter Formel I genannte Bedeutung hat, und eine in $R_4$ vorhandene funktionelle Gruppe gegebenenfalls in geschützter Form vorliegt, acyliert oder

b) in einer Verbindung der Formel

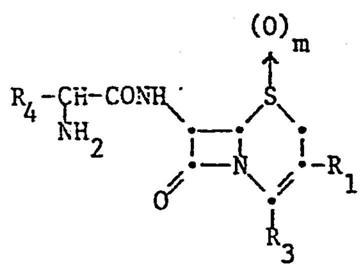

(IV) ,

worin m, $R_1$, $R_3$ und $R_4$ die unter Formel I genannten Bedeutungen haben, und eine in $R_1$ und/oder $R_4$ vorhandene funktionelle Gruppe gegebenenfalls geschützt ist, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt oder

c) eine 2-Cephem-Verbindung der Formel

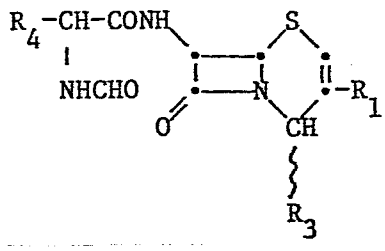

(V) ,

worin $R_1$, $R_3$ und $R_4$ die unter Formel I genannten Bedeutungen haben und eine in $R_1$ und/oder $R_4$ vorhandene funktionelle Gruppe gegebenenfalls in geschützter Form vorliegt, zur entsprechenden 3-Cephem-Verbindung der Formel I isomerisiert oder

d) zur Herstellung einer Verbindung der Formel I, worin $R_4$ 2-Amino-oxazol-4-yl oder 2-Aminothiazol-4-yl bedeutet, eine Verbindung der Formel

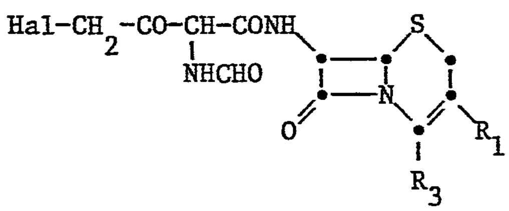

(VI),

worin Hal Halogen bedeutet, $R_1$ und $R_3$ die unter Formel I genannten Bedeutungen haben und eine in $R_1$ vorhandene funktionelle Gruppe gegebenenfalls geschützt ist, mit Harnstoff bzw. Thioharnstoff kondensiert oder

e) zur Herstellung einer Verbindung der Formel I, worin $R_4$ 5-Amino-1,2,4-thiadiazol-3-yl bedeutet, in einer Verbindung der Formel:

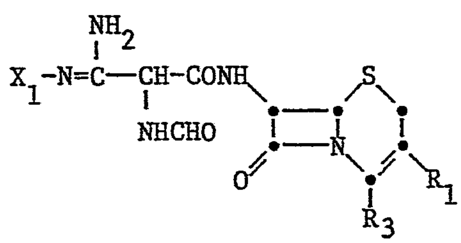

(VII),

worin $X_1$ Wasserstoff, Halogen oder Hydroxy bedeutet, $R_1$ und $R_3$ die unter Formel I genannten Bedeutungen haben und eine in $R_1$ vorhandene

funktionelle Gruppe gegebenenfalls geschützt ist, mit einem Salz der Rhodanwasserstoffsäure oder, falls $X_1$ Wasserstoff ist, mit Dirhodan umsetzt oder

f) zur Herstellung einer Verbindung der Formel I, worin $R_1$ eine Gruppe der Formel $-CH_2-R_2$ darstellt und $R_2$ veräthertes Mercapto bedeutet, eine Verbindung der Formel II, worin m und $R_3$ die unter Formel I genannten Bedeutungen haben, und $R_1$ die Gruppe der Formel $-CH_2-R_2$ darstellt, worin $R_2$ eine nukleofuge Abgangsgruppe bedeutet, mit einer Verbindung der Formel

NHCOH
|
$R_4$-CH-C-$R_2$'     (VIII),
||
O

worin $R_4$ die unter Formel I genannten Bedeutungen hat, und $R_2$' Niederalkylthio oder Heterocyclylthio bedeutet, umsetzt und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

Verfahren a) (Acylierung):

In einem Ausgangsmaterial der Formel II ist eine in $R_1$ vorhandene funktionelle Gruppe, z.B. eine Carboxy-, Amino- oder Hydroxygruppe, durch eine der weiter vorn genannten Schutzgruppen geschützt.

Die 7β-Aminogruppe in einem Ausgangsmaterial der Formel II ist gegebenenfalls durch eine die Acylierungsreaktion erlaubende Gruppe geschützt. Eine solche Gruppe ist beispielsweise eine organische Silylgruppe, ferner eine Ylidengruppe, die zusammen mit der Aminogruppe eine Schiff'sche Base bildet. Eine organische Silylgruppe ist z.B. eine solche Gruppe, die auch mit der Carboxygruppe $R_3$ eine geschützte Carboxygruppe bilden kann, in erster Linie Triniederalkylsilyl, insbesondere Trimethylsilyl. Bei der Silylierungsreaktion zum Schutz einer 4-Carboxygruppe in einem Ausgangsmaterial der Formel II kann bei Verwendung eines Ueberschusses des Silylierungsmittels die Aminogruppe ebenfalls silyliert werden. Eine Ylidengruppe ist in erster Linie eine 1-Arylniederalkyliden-, insbesondere eine 1-Arylmethylengruppe, worin Aryl besonders für einen carbocyclischen, in erster Linie monocyclischen, Arylrest steht, z.B. für gegebenenfalls durch Niederalkyl, Hydroxy, Niederalkoxy und/oder Nitro substituiertes Phenyl.

Ein den Acylrest einer Carbonsäure der Formel III einführendes Acylierungsmittel ist die Carbonsäure der Formel III in Gegenwart eines Kondensationsmittels oder ein reaktionsfähiges, funktionelles Derivat oder Salz dieser Carbonsäure.

Es können Carbonsäuren der Formel III mit 2R- oder 2S-Konfiguration oder reaktionsfähige, funktionelle Derivate davon oder 2R,S-Verbindungen bei der Acylierung eingesetzt werden. Bevorzugt wird die Acylierung mit 2S-Verbindungen durchgeführt.

In einem Ausgangsmaterial der Formel III ist eine in $R_4$ vorhandene funktionelle Gruppe, z.B. eine Aminogruppe am Heterocyclus, durch eine der weiter vorn genannten Schutzgruppen z.B. Aminoschutzgruppen, z.B. BOC, geschützt.

In einem Ausgangsmaterial der Formel III kann diese Aminogruppe auch in ionischer Form geschützt sein, z.B. in Form eines Säureadditionssalzes, welches beispielsweise mit einer starken anorganischen Säure, z.B. einer Halogenwasserstoffsäure, z.B. Salzsäure oder Schwefelsäure, oder mit einer organischen Säure z.B. p-Toluolsulfonsäure, gebildet wird.

Falls eine freie Säure der Formel III zur Acylierung eingesetzt wird, wird die Reaktion üblicherweise in Gegenwart von geeigneten Kondensationsmitteln wie Carbodiimiden, beispielsweise Diäthyl-, Dipropyl, Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert-Butyl-5-methyl-1,2-oxazoliumperchlorat, oder einer geeigneten Acylaminoverbindung, z.B. 2-Aethoxy-1-äthoxycarbonyl-1,2-dihydrochinolin, durchgeführt.

Die Kondensationsreaktion wird vorzugsweise in flüssiger Phase, vorzugsweise in Gegenwart eines Lösungsmittels, z.B. Methylenchlorid, Dimethylformamid, Acetonitril oder Tetrahydrofuran, gegebenenfalls unter Kühlen oder Erwärmen, z.B. in einem Temperaturbereich von etwa -40°C bis etwa +100°C, bevorzugt von etwa -20°C bis etwa +50°C, und gegebenenfalls unter Inertgas-, z.B. Stickstoffatmosphäre, durchgeführt.

Ein reaktionsfähiges, funktionelles Derivat einer Carbonsäure der Formel III ist zur Bildung der Carboxamid-Funktion -CONH- befähigt und ist in erster Linie ein Anhydrid der Carbonsäure der Formel III, vorzugsweise ein gemischtes Anhydrid. Ein gemischtes Anhydrid wird beispielsweise durch Kondensation der Carbonsäure der Formel III mit einer anderen Säure, z.B. einer anorganischen Säure, z.B. einer

Halogenwasserstoffsäure, gebildet und ist beispielweise das entsprechende Carbonsäurehalogenid, z.B. das Carbonsäurechlorid oder -bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung des gemischten Anhydridrids eignen, sind Phosphor-haltige Säuren, z.B. Phosphorsäure, Diäthylphosphorsäure und phosphorige Säuren, Schwefel-haltige Säuren, z.B. Schwefelsäure oder Cyanwasserstoffsäure. Ein reaktionsfähiges, funktionelles Derivat einer Carbonsäure der Formel III wird ausserdem durch Kondensation mit einer organischen Carbonsäure gebildet, z.B. mit einer unsubstituierten oder durch Halogen, z.B. Fluor oder Chlor, substituierten Niederalkancarbonsäure, z.B. Pivalinsäure oder Trifluoressigsäure, mit einem Niederalkylhalbester der Kohlensäure, z.B. dem Aethyl- oder Isobutylhalbester der Kohlensäure, oder mit einer organischen, z.B. aliphatischen oder aromatischen, Sulfonsäure, z.B. Methansulfonsäure oder p-Toluolsulfonsäure.

Ein reaktionsfähiges funktionelles Derivat einer Carbonsäure der Formel III ist ebenfalls ein aktivierter Ester der Carbonsäure der Formel III, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel III und z.B. Dimethyl-(1-chloräthyliden)-iminiumchlorid der Formel $[(CH_3)_2N^+=C(Cl)CH_3]Cl^-$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. der Pentachlor-, 4-Nitrophenyl- oder 2,3-Dinitrophenylester, ein N-heteroaromatischer Ester, z.B. der N-Bentriazolester, oder ein N-Diacyliminoester, z.B. der N-Succinylimino- oder N-Phthalyliminoester.

Die Acylierung mit einem reaktionsfähigen funktionellen Derivat der Carbonsäure der Formel III, z.B. mit einem entsprechenden Anhydrid,

insbesondere einem Säurehalogenid, wird bevorzugt in Anwesenheit einer geeigneten Base durchgeführt. Eine geeignete Base ist beispielsweise ein Amin, z.B. ein tertiäres Amin, z.B. Triniederalkylamin, z.B. Trimethylamin, Triäthylamin oder Aethyl-di-isopropylamin, oder N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin oder Chinolin. Eine geeignete Base ist ferner eine anorganische Base, beispielsweise ein Alkalimetall- oder Erdalkalimetallhydroxid, -carbonat oder -hydrogencarbonat, z.B. Natrium-, Kalium- oder Calciumhydroxid, -carbonat oder -hydrogencarbonat, oder ist ein Oxiran, beispielsweise ein 1,2-Niederalkylenoxid, wie Aethylenoxid oder Propylenoxid, ferner ein Silylamid, z.B. Trimethylsilylacetamid, ein Carbonsäureamid, z.B. Dimethylformamid, Harnstoff oder ein Nitril, z.B. Acetonitril.

Die Acylierung mit einem reaktionsfähigen, funktionellen Derivat der Carbonsäure der Formel III wird vorzugsweise in einem inerten, vorzugsweise wasserfreien, Lösungsmittel oder Lösungsmittelgemisch vorgenommen, beispielsweise in einem Carbonsäureamid, z.B. Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls in Gegenwart eines Alkohols, z.B. Methanol oder Aethanol, oder Wasser, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegegebenenfalls unter Inertgas-, z.B. Stickstoffatmosphäre.

Die Acylierung einer Verbindung der Formel II kann auch bei Verwendung eines geeigneten reaktionsfähigen, funktionellen Derivats der Säure der Formel III in Gegenwart einer geeigneten Acylase erfolgen. Solche

Acylasen sind bekannt und können durch eine Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung wird insbesondere ein Amid, Ester oder Thioester, wie ein Niederalkyl-, z.B. Methyl- oder Aethylester, der Carbonsäure der Formel III als reaktionsfähiges, funktionelles Derivat eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Ein bei der Acylierungsreaktion eingesetztes, reaktionsfähiges funktionelles Derivat einer Säure der Formel III kann, wenn erwünscht, in situ gebildet werden. So kann man z.B. ein gemischtes Anhydrid in situ herstellen, indem man eine Säure der Formel III, worin funktionelle Gruppen gegebenenfalls geschützt sind, oder ein geeignetes Salz davon, z.B. ein Ammoniumsalz, welches z.B. mit einer organischen Base, wie Pyridin oder 4-Methylmorpholin gebildet wird, oder ein Metallsalz, z.B. ein Alkalimetallsalz, z.B. Natriumsalz, mit einem geeigneten Derivat einer anderen Säure, beispielsweise einem Säurehalogenid, einer unsubstituierten oder durch Halogen, z.B. Chlor, substituierten Niederalkancarbonsäure, z.B. Trichloracetylchlorid, einem Halbester eines Kohlensäurehalbhalogenids, z.B. Chlorameisensäureäthylester, oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung bei der Acylierungsreaktion verwenden.

Verfahren b) (Formylierung):

In einem Ausgangsmaterial der Formel IV kann eine in $R_1$ und/oder $R_4$ vorhandene funktionelle Gruppe, z.B. eine Carboxy-, Amino- oder Hydroxygruppe, durch eine der weiter vorn genannten Schutzgruppen geschützt sein.

Die 2-Aminogruppe in einem Ausgangsmaterial der Formel IV ist gegebenenfalls durch eine die Formylierungsreaktion erlaubende Gruppe geschützt. Eine solche Gruppe ist beispielsweise eine organische Silylgruppe, z.B. eine Triniederalkylsilylgruppe, z.B. Trimethylsilyl oder eine Ylidengruppe, welche zusammen mit der Aminogruppe eine Schiff'sche Base bildet, und ist dieselbe Gruppe, durch welche die 7β-Aminogruppe in einem Ausgangsmaterial der Formel II gegebenenfalls substituiert ist, und welche die Acylierungsreaktion gemäss Verfahren a) erlaubt.

Eine in $R_4$ vorhandene funktionelle Gruppe, z.B. die Aminogruppe der 2-Aminothiazol-4-yl-, 2-Aminooxazol-4-yl oder 5-Amino-1,2,4-thiadiazol-3-yl-Gruppe ist durch übliche Schutzgruppen, z.B. übliche Aminoschutzgruppen, z.B. tert-Butoxycarbonyl (BOC) geschützt, bei deren Abspaltung die Formamidogruppe erhalten bleibt.

Geeignete Formylierungsmittel sind die Ameisensäure oder die Ester davon, z.B. ein Niederalkylester, z.B. der Methyl- oder Aethylester, ein Arylester, z.B. ein durch Halogen, z.B. Chlor, und/oder Nitro substituierter Phenylester, z.B. der Pentachlor-, 4-Nitrophenyl-

oder 2,3-Dinitrophenylester, oder ein N-heteroaromatischer Ester, z.B. N-Benztriazolester, gemischte Anhydride der Ameisensäure mit anderen Carbonsäuren oder deren Derivaten, z.B. Säurechloriden, z.B. Essigsäure, Trichlor- oder Trifluoressigsäure oder Propionsäure oder Säurechloride, z.B. Acetylchlorid, gemischte Anhydride mit Sulfonsäuren oder Sulfochloriden, z.B. Methansulfonsäure oder Mesylchlorid, Formamid, Formylfluorid, Chloral oder Kohlenmonoxid in Gegenwart eines Katalysators.

Falls die freie Ameisensäure bei der Formylierung eingesetzt wird, führt man die Formylierung üblicherweise in Gegenwart desselben Kondensationsmittels durch, welches man bei der Acylierung der 7β-Aminogruppe einer Verbindung der Formel II mit einer freien Carbonsäure der Formel III gemäss Verfahren a) verwendet, z.B. in Gegenwart von einem Carbodiimid, z.B. N,N'-Dicyclohexylcarbodiimid.

Bei der Formylierung mit der Ameisensäure werden die gleichen Lösungsmittel verwendet und die gleichen Reaktionsbedingungen eingehalten wie bei der Acylierung mit einer Carbonsäure der Formel III gemäss Verfahren a).

Bei der Formylierung mit Ameisensäureestern und gemischten Anhydriden der Ameisensäure werden die gleichen Lösungsmittel, z.B. Essigsäure oder Essigsäureanhydrid, verwendet und die gleichen Reaktionsbedingungen eingehalten wie bei der Acylierung mit einem reaktionsfähigen, funktionellen Derivat einer Carbonsäure der Formel III gemäss Verfahren a).

Die Formylierung mit Formamid erfolgt bei erhöhter Temperatur, z.B. ca. 70° - 100°, z.B. in einem Ueberschuss Formamid. Bei der Formylierung mit Chloral setzt man das Chloral im allgemeinen unter Kühlen, z.B. unter 0°, zu und erhitzt anschliessend langsam, z.B. bis zur Siedetemperatur des Reaktionsgemisches. Die Formylierungen mit Formamid oder Chloral erfolgen im allgemeinen analog den in "Houben-Weyl, Methoden der Organischen Chemie", Band 11/2 auf S. 27 - 30 genannten Reaktionsbedingungen.

Bei der Formylierung mit Kohlenmonoxid werden als Katalysatoren Metalle, z.B. Cobalt und Nickel, oder Metallsalze, z.B. Kupfer-I- oder Kupfer-II-chlorid, verwendet. Man arbeitet bei erhöhtem Druck, z.B. über 150 at.

Verfahren c) (Isomerisierung):

In einem 2-Cephem-Ausgangsmaterial der Formel V weist die gegebenenfalls geschützte 4-Carboxygruppe vorzugsweise die α-Konfiguration auf.

Die Isomerisierung einer 2-Cephem-Verbindung der Formel V zur entsprechenden 3-Cephem-Verbindung der Formel I erfolgt, indem man die 2-Cephem-Verbindung der Formel V in 1-Stellung mit einem geeigneten Oxydationsmittel oxydiert und, wenn erwünscht, ein gegebenenfalls erhältliches Isomerengemisch der 1-Oxide trennt und ein so erhältliches 1-Oxid der 3-Cephem-Verbindung der Formel I, worin m 1 bedeutet, zur 3-Cephem-Verbindung, worin m null bedeutet, reduziert.

Als Oxydationsmittel für die Oxydation des Schwefelatoms in 1-Stellung einer 2-Cephem-Verbindung der Formel V eignen sich organische Persäure oder Gemische aus Wasserstoffperoxid und Säuren, insbesondere organische Carbonsäuren mit einer Dissoziationskonstante von wenigstens $10^{-5}$. Organische Persäuren sind beispielsweise Percarbon- und Persulfonsäuren, die als Persäure zugesetzt oder durch Verwendung von mindestens einem Aequivalent Wasserstoffperoxid und einer Carbonsäure in situ gebildet werden können. Bei der in-situ Bildung der Persäure setzt man zweckmässig einen grossen Ueberschuss der Carbonsäure, z.B. Essigsäure, als Lösungsmittel zu. Geeignete organische Persäuren sind vorzugsweise Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxydation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der betreffenden Säure einsetzen kann. Dabei hängt die oxydative Wirksamkeit des Gemisches in erster Linie von der Stärke der Säure ab. Geeignete Gemische sind z.B. Wasserstoffperoxid mit Essigsäure oder Trifluoressigsäure.

Die obige Oxydation kann in Gegenwart von geeigneten sauren Katalysatoren durchgeführt werden. So kann z.B. die Oxydation mit einer Percarbonsäure in Gegenwart einer Säure mit einer Dissoziationskonstante von wenigstens $10^{-5}$ katalysiert werden, wobei ihre katalytische Wirksamkeit von ihrer Säurestärke abhängt. Als Katalysatoren geeignete Säuren sind z.B. Essigsäure, Perchlorsäure und Trifluoressigsäure. Ueblicherweise verwendet man mindestens äquimolare Mengen des Oxydationsmittels, vorzugsweise einen geringen Ueberschuss von etwa 10 % bis etwa 20 %, wobei man auch grössere Ueberschüsse, d.h. bis zur 10fachen Menge des Oxydationsmittels oder darüber verwenden kann. Die Oxydation wird unter milden Bedingungen, z.B. bei Temperaturen von etwa -50°C bis etwa +100°C, vorzugsweise von etwa -10°C bis etwa 40°C durchgeführt.

Die Reduktion eines 1-Oxids der 3-Cephem-Verbindung, d.h. einer 3-Cephem-Verbindung der Formel I, worin m Null bedeutet, kann in an sich bekannter Weise durch Behandeln mit einem geeigneten Reduktionsmittel, wenn notwendig in Anwesenheit eines aktivierenden Mittels, durchgeführt werden. Geeignete Reduktionsmittel sind beispielsweise: Reduzierend wirkende Zinn-, Eisen-, Kupfer- oder Mangankationen, welche in Form ihrer Salze, z.B. als Zinn-II-chlorid, -acetat oder -formiat, Eisen-II-chlorid, -sulfat, -oxalat, oder Ammoniumeisensulfat, Kupfer-I-chlorid oder -oxid, oder Mangan-II-chlorid, -sulfat, -acetat oder -oxid, oder als organische oder anorganische Komplexe, z.B. mit Aethylendiamintetraessigsäure oder Nitrolotriessigsäure, verwendet werden; reduzierend wirkende Dithionit-, Jod- oder Eisen-II-cyanid-anionen, welche in Form ihrer anorganischen oder organischen Salze, z.B. als Alkalimetall-, z.B. Natrium- oder Kaliumdithionit, Natrium- oder Kaliumjodid oder Natrium- oder Kaliumeisen-II-cyanid verwendet werden; Jodwasserstoffsäure, reduzierend wirkende trivalente, anorganische oder organische Phosphorverbindungen, wie Phosphine, ferner Ester, Amide und Halogenide der phosphonigen, phosphinigen oder phosphorigen Säure, sowie diesen Phosphor-Sauerstoffverbindungen entsprechende Phosphor-Schwefelverbindungen, worin in diesen Verbindungen organische Reste in erster Linie aliphatische, aromatische oder araliphatische Reste, z.B. gegebenenfalls substituiertes Niederalkyl, Phenyl oder Phenylniederalkyl, darstellen, z.B. Triphenylphosphin, Diphenylphosphonigsäuremethylester, Diphenylchlorphosphin, Phenyldichlorphosphin, Benzolphosphonigsäuredimethylester, Phosphorigsäuretriphenylester, Phosphorigsäuretrimethylester, Phosphortrichlorid, Phosphortribromid, ferner Phosphorigsäuretriphenylester-Halogenaddukte, z.B. Chlor- oder Bromaddukte, worin die Phenylreste gegebenenfalls durch Niederalkyl, z.B. Methyl, Niederalkoxy, z.B. Methoxy, oder durch Halogen, z.B. Chlor, substituiert sind, etc.; reduzierend wirkende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, beispielsweise Diphenylchlorsilan oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, etc.; reduzierend wirkende quaternäre Chlormethylen-iminiumsalze, insbesondere -chloride oder -bromide, worin die Iminiumgruppen durch einen bivalenten oder zwei monovalente organische Reste, wie gegebenenfalls substituiertes Niederalkylen bzw. Niederalkyl substituiert ist, wie N-Chlormethylen-N,N-dimethyliminiumchlorid oder N-Chlormethylen-pyrrolidiniumchlorid; oder komplexe Metallhydride, wie Natriumborhydrid, in Gegenwart von geeigneten Aktivierungsmitteln, wie Kobalt-II-chlorid, sowie Borandichlorid.

Aktivierende Mittel verwendet man zusammen mit solchen Reduktionsmitteln, welche keine oder nur schwache Lewissäure-Eigenschaften aufweisen. Diese werden in erster Linie zusammen mit Dithionit-, Jod- oder Eisen-II-cyanidsalzen und nicht halogenhaltigen trivalenten

Phosphor-Reduktionsmitteln eingesetzt und sind insbesondere organische Carbon- und Sulfonsäurchalogenide, z.B. Phosgen, Oxalylchlorid, Essigsäurechlorid oder -bromid, oder Chloressigsäurechlorid.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, z.B. gegebenenfalls substituierte, z.B. halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel kein Wasser enthalten.

Dabei arbeitet man gewöhnlicherweise bei Temperaturen von etwa -20°C bis etwa 100°C, wobei man bei Verwendung von sehr reaktionsfähigen Reduktionsmitteln bzw. Aktivierungsmitteln die Reaktion auch bei tieferen Temperaturen durchführt und gegebenenfalls unter Inertgasatmosphäre, z.B. Stickstoffatmosphäre.

Verfahren d) (Ringschluss):

In einem Ausgangsmaterial der Formel VI ist eine in $R_1$ vorhandene funktionelle Gruppe, z.B. eine Amino- oder Carboxygruppe, gegebenenfalls durch eine der weiter vorn genannten Schutzgruppen geschützt. Hal bedeutet Halogen, bevorzugt Chlor, ferner Brom, Jod oder Fluor. Wenn im Produkt der Formel I $R_4$ 2-Aminooxazol-4-yl bedeuten soll, wird Harnstoff, und wenn $R_4$ 2-Aminothiazol-4-yl bedeutet, Thioharnstoff in äquivalenter Menge oder im Ueberschuss eingesetzt.

Die Zyklisierung wird im allgemeinen in einem Lösungsmittel, wie Wasser oder einem organischen, inerten Lösungsmittel oder einer Mischung davon, durchgeführt. Als organische Lösungsmittel geeignet sind Alkohole, wie Methanol, Aethanol oder Isopropanol, Ketone, wie Aceton, Aether, wie Dioxan oder Tetrahydrofuran, Nitrile, wie Acetonitril, halogenierte Kohlenwasserstoffe, wie Methylenchlorid, Chloroform oder Tetrachlorkohlenstoff, Ester, wie Aethylacetat, oder Amide, wie Dimethylformamid oder Dimethylacetamid, und ähnliche. Die Reaktion kann in Gegenwart einer unter Verfahren a) genannten Base durchgeführt werden. Die Reaktionstemperatur liegt zwischen Raumtemperatur und der Siedetemperatur des Reaktionsgemisches, vorzugsweise zwischen 60°C und der Siedetemperatur des Reaktionsgemisches.

Die Zyklisierung kann auch stufenweise erfolgen, indem zunächst ein offenes Zwischenprodukt, z.B. der Teilformel $NH_2-C(=NH)-O-CH_2-CO-\overset{|}{C}H-NHCO$ bzw. $NH_2-C(=NH)-S-CH_2-CO-\overset{|}{C}H-NHCO$ entsteht, das dann in der zweiten Stufe durch Erwärmen dehydratisiert wird.

Verfahren e) (Ringschluss):

In einem Ausgangsmaterial der Formel VII ist eine in $R_1$ vorhandene funktionelle Gruppe, z.B. eine Amino- oder Carboxygruppe, gegebenenfalls durch eine der weiter vorn genannten Schutzgruppen geschützt. $X_1$ mit der Bedeutung Halogen ist bevorzugt Brom, ferner Fluor, Chlor oder Jod. Geeignete Salze der Rhodanwasserstoffsäure sind beispielsweise Alkali-, Erdalkali- oder Schwermetallrhodanide, z.B. Natrium-, Kalium-, Magnesium- oder Bleirhodanid, oder auch Ammoniumrhodanide, worin die Ammoniumgruppe von Ammoniak oder von organischen Stickstoffbasen abgeleitet ist, z.B. Ammonium- oder Mono-, Di-, Tri- oder Tetraniederalkylammoniumrhodanid, worin Niederalkyl z.B. Methyl, Aethyl oder Isopropyl bedeutet.

Die Reaktion wird bevorzugt in einem inerten Lösungsmittel, wie einem Niederalkanol, z.B. Methanol, Aethanol, Propanol oder Isopropanol,

einem Aether, wie Diäthyläther, Dioxan oder Tetrahydrofuran, einem Amid, wie Dimethylformamid, einem aliphatischen oder aromatischen Kohlenwasserstoff, wie Hexan, Benzol oder Toluol, oder auch in Mischungen davon, und, wenn notwendig, unter Kühlen oder Erwärmen, d.h. bei Temperaturen zwischen etwa -80°C bis etwa +100°C, bevorzugt zwischen etwa -20°C und Raumtemperatur,und/oder in einer Inertgasatmosphäre durchgeführt.

Das Ausgangsmaterial der Formel VII, worin $X_1$ Halogen ist, kann in situ hergestellt werden, gegebenenfalls in Gegenwart des Rhodanwasserstoffsalzes, beispielsweise indem man eine Verbindung der Formel VII, worin $X_1$ Wasserstoff ist, in einem der genannten Lösungsmittel mit einem Hypohalogenit, z.B. Natrium- oder Kaliumhypobromit, oder mit einem Halogen und einer Alkalimetallbase, wie einem Hydroxid, Carbonat oder Niederalkanolat, z.B. mit Brom und Natriummethylat oder Kaliumcarbonat, behandelt. Die Halogenierung findet bevorzugt unter Kühlen, z.B. bei einer Temperatur von etwa -15°C bis etwa 0°C, statt.

Das Ausgangsmaterial der Formel VII, worin X Hydroxy ist, kann ebenfalls in situ hergestellt werden, beispielsweise indem man eine Verbindung der Formel VII, worin X Wasserstoff ist, mit einem Oxydationsmittel, wie Wasserstoffperoxid, behandelt.

Die Reaktion des Ausgangsmaterials der Formel VII, worin X Wasserstoff ist, mit Dirhodan wird vorzugsweise ebenfalls in einem der genannten inerten Lösungsmittel und unter ähnlichen Reaktionsbedingungen durchgeführt. Gegebenenfalls kann auch ein Säureadditionssalz einer Verbindung der Formel VII eingesetzt werden, das in situ in die freie Verbindung übergeführt wird, oder das Dirhodan kann in situ gebildet werden, z.B. aus einem Rhodanid, z.B. Bleirhodanid, und einem Halogen, z.B. Brom.

Verfahren f) (Umsetzung des Thiocarbonsäureesters):

In einem Ausgangsmaterial der Formel II ist die 7β-Aminogruppe durch eine der unter Verfahren a) genannten, die Acylierungsreaktion erlaubenden Gruppen, z.B. Trimethylsilyl, und die 4-Carboxygruppe $R_3$ durch eine der genannten üblichen Carboxyschutzgruppen, z.B. Diphenylmethyl, gegebenenfalls geschützt.

Die nukleofuge Abgangsgruppe $R_2$ ist z.B. Halogen, z.B. Chlor, Azido, Benzimidazol-2-ylthio oder vorzugsweise Acetoxy.

In einer Verbindung der Formel VIII ist $R_2'$ Niederalkylthio, z.B. Methylthio, oder, vorzugsweise, Heterocyclylthio, z.B. 1H-1,2,3-Triazol-4(5)-ylthio, 1H-1,2,4-Triazol-3-ylthio, 1H-Tetrazol-5-ylthio, 1-Methyl-1H-tetrazol-5-ylthio, 1-Aethyl- oder 1-Diäthylphosphonylmethyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Cyanmethyl-1H-tetrazol-5-ylthio, 1-(1H-Tetrazol-5-ylmethyl)-1H-tetrazol-5-ylthio, Thiazol-2-ylthio, Isothiazol-3-, -4- oder -5-ylthio, 1,2,3-Thiadiazol-4- oder -5-ylthio, 1,3,4-Thiadiazol-2-ylthio, 1,2,4-Thiadiazol-3- oder -5-ylthio, 1,2,5-Thiadiazol-3-ylthio, 2-Methyl-1,3,4-Thiadiazol-5-ylthio, 1,2,3,4-Thiatriazol-5-ylthio, Oxazol-2-, -4- oder -5-ylthio, 1- oder 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, 1- oder 2-Carboxymethyl-5,6-dioxo-1,2,5,6-tetrahydro-as-triazin-3-ylthio, 4-Carboxymethyl-5,6-dioxo-1,4,5,6-tetrahydro-as-triazin-3-ylthio, 1- oder 2-Sulfomethyl-5,6-dioxo-1,2,5,6-tetrahydro-as-triazin-3-ylthio oder 4-Sulfomethyl-5,6-dioxo-1,4,5,6-tetrahydro-as-triazin-3-ylthio und Tautomere davon.

Die Umsetzung des Thiocarbonsäureesters der Formel VIII mit einer Verbindung der Formel II, worin die nukleofuge Abgangsgruppe $R_2$ Halogen, z.B. Chlor, Azido oder Acetoxy bedeutet, erfolgt unter neutralen oder

schwach alkalischen Bedingungen, vorzugsweise bei einem pH-Wert von ca. 7 bis 8, indem man z.B. eine wässrige Natrium- oder Kaliumhydrogencarbonat-Lösung zur Reaktionsmischung hinzufügt. Die Reaktion mit einer Verbindung der Formel II, worin die nukleofuge Abgangsgruppe die Benzimidazolylthiogruppe ist, erfolgt vorzugsweise unter sauren Bedingungen, indem man z.B. 0,05 bis 1 Aequivalent einer wässrigen Mineralsäure, z.B. verdünnter Chlorwasserstoff- oder Schwefelsäure, Sulfonsäure, z.B. Methan- oder p-Toluolsulfonsäure, oder Carbonsäure, z.B. Essigsäure, hinzufügt.

Pro Mol eingesetzten Thiocarbonsäureesters der Formel VIII lässt man vorzugsweise 1 bis 2 Aequivalente der Verbindung der Formel II reagieren.

Die Umsetzung erfolgt analog den weiter vorn unter Verfahren a) - Umsetzung einer Carbonsäure der Formel III mit einer 7β-Aminoverbindung der Formel II - genannten Reaktionsbedingungen und in einem der dort genannten Lösungsmittel.

Nachoperationen:

In einer erhaltenen Verbindung der Formel I können auf übliche, an sich bekannte Weise noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

$R_1$-Umwandlungen:

In einer erhaltenen Verbindung der Formel I, worin funktionelle Gruppen gegebenenfalls geschützt sind, kann man in an sich bekannter Weise eine Gruppe $R_1$ durch einen anderen Rest $R_1$ ersetzen oder in einen anderen Rest $R_1$ umwandeln. So kann man beispielsweise in einer Verbindung der Formel I, worin $R_1$ eine Gruppe der Formel $-CH_2-R_2$ bedeutet und $R_2$ z.B. einen, durch nucleophile Substituenten ersetzbaren Rest darstellt, oder in einem Salz davon durch Behandeln mit einer Mercaptanverbindung, z.B. einer Heterocyclylmercaptan-Verbindung, oder mit einer Thiocarbonsäureverbindung einen solchen Rest $R_2$ durch eine verätherte Mercaptogruppe, z.B. Heterocyclylmercaptogruppe, bzw. eine veresterte Mercaptogruppe $R_2$ ersetzen.

Ein geeigneter, durch nucleophile Substituenten, z.B. eine verätherte Mercaptogruppe, ersetzbarer Rest ist beispielsweise eine durch eine niederaliphatische Carbonsäure veresterte Hydroxygruppe. Eine solche veresterte Hydroxygruppe ist insbesondere Acetyloxy und Acetoacetoxy.

Die Reaktion einer solchen Verbindung der Formel I mit einer geeigneten Mercaptanverbindung, z.B. Heterocyclylmercaptan-Verbindung, kann unter sauren, neutralen oder schwach basischen Bedingungen durchgeführt werden. Bei sauren Bedingungen arbeitet man in Gegenwart von konzentrierter Schwefelsäure, welche gegebenenfalls durch ein anorganisches Lösungsmittel, z.B. Polyphosphorsäure, verdünnt wird. Bei neutralen oder schwach basischen Bedingungen führt man die Reaktion in Gegenwart von Wasser und gegebenenfalls einem mit Wasser mischbaren organischen Lösungsmittel durch. Auch Pufferlösungen können als Reaktionsmedium verwendet werden.

Die basischen Bedingungen können beispielsweise durch Zugabe einer anorganischen Base, wie eines Alkalimetall- oder Erdalkalimetallhydroxids, -carbonats oder -hydrogencarbonats, z.B. durch Zugabe von

Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, z.B. Dimethylformamid, oder Nitrile, z.B. Niederalkansäurenitrile, z.B. Acetonitril, verwendet werden.

In einer Verbindung der Formel I, worin $R_1$ eine Gruppe der Formel $-CH_2-R_2$ bedeutet, worin $R_2$ freies Hydroxy bedeutet, kann man die freie Hydroxygruppe durch den Acylrest einer gegebenenfalls N-substituierten Carbaminsäure verestern. Die Veresterung der freien Hydroxygruppe mit einer Isocyanat-Verbindung, z.B. Halogensulfonylisocyanat, z.B. Chlorsulfonylisocyanat, oder mit einem Carbaminsäurehalogenid, z.B. Carbaminsäurechlorid, führt zu N-unsubstituierten 3-Carbamoyloxymethyl-Cephalosporinen der Formel I. Die Veresterung der freien Hydroxygruppe mit einer N-substituierten Isocyanat-Verbindung oder mit einer N-mono- oder N,N-disubstituierten Carbaminsäure-Verbindung, z.B. einem entsprechend substituierten Carbaminsäurehalogenid, z.B. einem N-mono- oder N,N-disubstituierten Carbaminsäurechlorid, führt zu N-mono- oder N,N-disubstituierten 3-Carbamoyloxymethyl-Cephalosporinen der Formel I. Man arbeitet üblicherweise in Gegenwart eines Lösungs- oder Verdünnungsmittels und, wenn notwendig, unter Kühlen oder Erwärmen, in einem geschlossenen Gefäss und gegebenenfalls unter Inertgas-, z.B. Stickstoffatmosphäre. Die Verbindung der Formel I, worin $R_1$ eine Gruppe der Formel $-CH_2-R_2$ bedeutet, worin $R_2$ freies Hydroxy bedeutet, kann man aus einer Verbindung der Formel IV durch Abspaltung des Acetylrests aus einer Acetoxygruppe $R_2$ herstellen, z.B. durch Hydrolyse in schwach-basischem Medium, z.B. in einer wässrigen Natriumhydroxydlösung bei pH 9 - 10, oder durch Behandeln mit einer geeigneten Esterase, wie einem entsprechenden Enzym aus Rhizobium tritolii, Rhizobium lupinii, Rhizobium japonicum oder Bacillus subtilis, oder einer geeigneten Citrus-Esterase, z.B. aus Orangenschalen, und anschliessende Formylierung gemäss Verfahren b).

Ferner kann man eine Verbindung der Formel I, worin $R_1$ eine Gruppe $-CH_2-R_2$ bedeutet, wobei $R_2$ z.B. den oben definierten, durch nucleophile Substituenten ersetzbaren Rest, z.B. Acetoxy oder Acetacetoxy, darstellt, mit einer organischen Base, insbesondere einer tertiären, Stickstoff-haltigen Base, beispielsweise einem tertiären, aliphatischen Amin, oder vorzugsweise einer tertiären, heterocyclischen, aromatischen Stickstoffbase, z.B. Pyridin oder Pyrimidin mit den weiter vorn genannten Substituenten, unter neutralen oder schwach sauren Bedinngungen, bevorzugt bei einem pH-Wert von etwa 6,5, in Gegenwart von Wasser und gegebenenfalls in einem mit Wasser mischbaren organischen Lösungsmittel umsetzen. Man gelangt so zu Verbindungen der Formel I, worin $R_1$ den Rest der Formel-$CH_2-R_2$ und $R_2$ eine weiter vorn definierte Ammoniogruppe darstellt. Schwach saure Bedingungen können durch Zugabe einer geeigneten organischen oder anorganischen Säure, beispielsweise Essigsäure, Chlorwasserstoffsäure, Phosphorsäure oder Schwefelsäure, eingestellt werden. Als organische Lösungsmittel können beispielsweise die vorstehend genannten, mit Wasser mischbaren Lösungsmittel, verwendet werden. Zur Erhöhung der Ausbeute können der Reaktionsmischung Salze zugesetzt werden, beispielsweise Alkalimetall-, wie Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder von organischen Säuren, wie Niederalkancarbonsäuren, z.B. Essigsäure. Geeignete Salze sind beispielsweise Natriumjodid, Kaliumjodid und Kaliumthiocyanat. Auch Salze von bestimmten Anionenaustauschern, z.B. flüssige Ionenaustauscher in Salzform, wie z.B. Amberlite LA-1 (flüssige sekundäre Amine mit einem Molekulargewicht von 351-393; Oel-löslich und wasserunlöslich; mAeq./g = 2,5-2,7, z.B. in Acetatform), mit Säuren, z.B. Essigsäure, können für diesen Zweck verwendet werden.

Umwandlung zum 1-Oxid, 1-Dioxid und 1-Sulfid:

Eine Verbindung der Formel I, worin der Index m 0 bedeutet, kann mit den unter Verfahren c) beschriebenen Oxydationsmitteln in das entsprechende 1-Oxid, worin der Index m den Wert 1 hat, umgewandelt werden.

1-Oxide der Formel I, welche in α- oder β-Konfiguration vorliegen, können in an sich bekannter Weise gemäss dem aus der Deutschen Offenlegungsschrift 30 13 996 bekannten Verfahren und zwar durch Oxydation einer Verbindung der Formel II, worin m null ist und die 7β-Aminogruppe beispielsweise durch Ylidengruppen geschützt ist, die zusammen mit der Aminogruppe eine Schiff'sche Base bilden, mit einer Percarbonsäure, z.B. m-Chlorperbenzoesäure, chromatographischer Trennung der erhaltenen α- und β-1-Oxide der Formel II und anschliessender Acylierung mit einer Carbonsäure der Formel III hergestellt werden.

Eine Verbindung der Formel I, worin der Index m 0 oder 1 bedeutet, kann durch Umsetzung mit Sulfid- oder Sulfoxidgruppen in Sulfongruppen überführenden Oxydationsmitteln in das entsprechende 1-Dioxid, worin m den Wert 2 hat, überführt werden.

Solche Oxydationsmittel sind insbesondere Wasserstoffperoxid oder organische Persäuren, insbesondere aliphatische Percarbonsäuren, z.B. Peressigsäure, Perbenzoesäure, Chlorperbenzoesäure, z.B. m-Chlorperbenzoesäure, oder Monoperphthalsäure. Die Oxydation wird bevorzugt in einem geeigneten nicht-wässrigen, inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Amid, z.B. Dimethylformamid, oder einer flüssigen organischen Carbonsäure, z.B. Essigsäure, oder einem Gemisch dieser Lösungsmittel bei Raumtemperatur, unter Kühlen oder leichtem Erwärmen, d.h. bei etwa -50°C bis etwa +40°C, bevorzugt bei etwa -20°C bis etwa 0°C, durchgeführt. Die Oxydation kann auch stufenweise durchgeführt werden, indem zunächst bei niederer Temperatur, d.h. bei etwa -20°C bis etwa -10°C, bis zur Sulfoxidstufe (m = 1), das gegebenenfalls isoliert wird, oxydiert wird, worauf in einem zweiten Schritt, bevorzugt bei höherer Temperatur, z.B. -10° bis 0°, das Sulfoxid zum Sulfon (m = 2) oxydiert wird.

Zur Aufarbeitung kann gegebenenfalls noch vorhandenes überschüssiges Oxydationsmittel durch Umsetzung mit einem Reduktionsmittel, z.B. einem Thiosulfat, z.B. Natriumthiosulfat, zerstört werden.

Ein 1-Oxid der Formel I, worin der Index m den Wert 1 hat, sowie ein 1-Dioxid, worin der Index m den Wert 2 hat, kann mit den unter Verfahren c) beschriebenen Reduktionsmitteln in das entsprechende 1-Sulfid, worin der Index m den Wert 0 hat, umgewandelt werden.

Abspaltung von Schutzgruppen:

In einer erhältlichen Verbindung der Formel I, worin eine oder mehrere funktionelle Gruppen geschützt sind, können diese Schutzgruppen, z.B. geschützte Carboxy-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse, selektiv freigesetzt werden. Bei der Abspaltung der Schutzgruppen sind Reaktionsbedingungen zu vermeiden, die zur Spaltung der Formamidogruppe führen. Die einzuhaltenden Reaktionsbedingungen sind in dem Uebersichtsartikel von Lakshmi P.U. und Ramachandran L.K., J.Sci. Ind. Res. 30 [12] 680 - 688 (1971) beschrieben.

Eine geschützte Carboxylgruppe setzt man in an sich bekannter und je nach Art der Schutzgruppen in verschiedenartiger Weise, vorzugsweise mittels Solvolyse oder Reduktion, frei.

So kann man tert—Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol, Anisol oder Aethylenthioglykol, in freies Carboxy überführen. Geeignet substituiertes Benzyloxycarbonyl, wie 4-Nitrobenzyloxycarbonyl, kann man durch Reduktion in freies Carboxy überführen, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffionen-abgebenden Mittels, das zusammen mit dem Metall oder Metallsalz nascierenden Wasserstoff zu

erzeugen vermag, wie einer Säure, in erster Linie einer geeigneten Carbonsäure, wie einer gegebenenfalls, z.B. durch Hydroxy substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt. Durch Behandeln mit einem reduzierenden Metall oder Metallsalz kann man, wie oben beschrieben, auch 2-Halogenniederalkoxycarbonyl, gegebenenfalls nach Umwandlung einer 2-Bromniederalkoxycarbonylgruppe in die entsprechende 2-Jodniederalkoxycarbonylgruppe, oder Aroylmethoxycarbonyl in freies Carboxy umwandeln, wobei Aroylmethoxycarbonyl ebenfalls durch Behandeln mit einem nucleophilen, vorzugsweise salzbildenden, Reagenz, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann üblicherweise solvolytisch, z.B. durch Behandeln mit Wasser, einem Alkohol oder einer Säure, freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino, gegebenenfalls nach Umwandlung einer 2-Bromniederalkoxycarbonylaminogruppe in die 2-Jodniederalkoxycarbonylaminogruppe, Aroylmethoxycarbonylamino oder 4-Nitrobenzyloxycarbonylamino können z.B. durch Behandeln mit einem geeigneten chemischen Reduktionsmittel, wie Zink in Gegenwart einer geeigneten Carbonsäure, wie wässriger Essig-

säure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert-Niederalkoxycarbonylamino, z.B. BOC, oder 2-trisubstituiertes Silyläthoxycarbonylamino oder 4-Nitrophenylthio kann durch Behandeln mit einer geeigneten Säure, z.B. Ameisen- oder Trifluoressigsäure, und eine mit einer organischen Silylgruppe geschützte Aminogruppe, z.B. mittels Hydrolyse oder Alkoholyse, gespalten werden. Eine durch 2-Halogenacetyl, z.B. 2-Chloracetyl, geschützte Aminogruppe kann durch Behandeln mit Thioharnstoff in Gegenwart einer Base und anschliessende Solvolyse, wie Alkoholyse oder Hydrolyse, des entstandenen Kondensationsproduktes gespalten werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe übergeführt werden.

In Form einer Azidogruppe geschütztes Amino wird z.B. durch Reduktion in freies Amino übergeführt, durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse freigesetzt, während eine durch tert-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer halogenierten Carbonsäure, z.B. Trifluoressigsäure, freigesetzt wird.

Eine geschützte, insbesondere veresterte, Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, und gegebenenfalls in einer Inertgas-, z.B. Stickstoffatomosphäre.

Bevorzugt werden bei Vorhandensein von mehreren geschützten funktionellen Gruppen, z.B. einer geschützten Amino- und einer geschützten Carboxygruppe, die Schutzgruppen so gewählt, dass diese gleichzeitig abgespalten werden können ohne dass die Formamidogruppe gespalten wird, beispielsweise acidolytisch, wie durch Behandeln mit Trifluoressigsäure oder Ameisensäure, oder reduktiv, wie durch Behandeln mit Zink und Essigsäure.

Veresterung einer freien Carboxygruppe: Die Umwandlung einer freien Carboxygruppe, z.B. der freien Carboxygruppe $R_3$, in eine veresterte Carboxygruppe, insbesondere in eine Carboxygruppe, die unter physiologischen Bedingungen spaltbar ist, erfolgt nach an sich bekannten Veresterungsmethoden. Beispielsweise setzt man eine Verbindung der Formel I, worin die zu veresternde Carboxygruppe in freier Form und andere funktionelle Gruppen, z.B. Amino- oder Hydroxygruppen, in geschützter Form vorliegen, oder eine Verbindung der Formel I, worin die zu veresternde Carboxygruppe in Form eines reaktionsfähigen, funktionellen Derivates vorliegt, oder ein Salz einer Verbindung der Formel I mit dem entsprechenden Alkohol oder einem reaktionsfähigen, funktionellen Derivat dieses Alkohols um.

Bei der Veresterung einer Verbindung der Formel I, worin die zu veresternde Carboxygruppe in freier Form vorliegt, mit dem gewünschten Alkohol werden die gleichen Kondensationsmittel, z.B. Carbodiimide, die gleichen Lösungsmittel verwendet und die gleichen Reaktionsbedingungen eingehalten wie bei der Acylierung gemäss Verfahren a).

Eine Verbindung der Formel I, worin die zu veresternde Carboxygruppe in Form eines reaktionsfähigen, funktionellen Derivates vorliegt, ist beispielsweise ein gemischtes Anhydrid oder ein aktivierter Ester, welcher in der unter Verfahren a) (Acylierung) geschilderten Weise durch Kondensation der Carbonsäure der Formel I mit einer anorganischen Säure, einer Carbonsäure, mit einem Halbester der Kohlensäure, einer Sulfonsäure oder durch Kondensation mit einem vinylogen Alkohol erhalten werden kann.

Ein reaktionsfähiges, funktionelles Derivat des zu veresternden Alkohols ist in erster Linie der Ester, welcher durch Kondensation mit einer starken anorganischen oder organischen Säure gebildet wird, beispielsweise das entsprechende Halogenid, z.B. Chlorid, Bromid oder Jodid, oder die entsprechende Niederalkan-, z.B. die Methansulfonyloxy-Verbindung.

Bei der Veresterung einer Verbindung der Formel I, worin die zu veresternde Carboxylgruppe in Form eines reaktionsfähigen funktionellen Derivats vorliegt, mit dem entsprechenden Alkohol oder bei der Veresterung einer Verbindung der Formel I, worin die zu veresternde Carboxylgruppe in freier Form vorliegt, mit einem reaktionsfähigen, funktionellen Derivat des entsprechenden Alkohols werden die gleichen Lösungsmittel verwendet und die gleichen Reaktionsbedingungen eingehalten wie bei der Acylierung mit einem reaktionsfähigen, funktionellen Derivat einer Carbonsäure der Formel III gemäss Verfahren a).

Eine Verbindung der Formel I, worin die zu veresternde Carboxylgruppe in Form eines reaktionsfähigen, funktionellen Derivats vorliegt,

kann man auch analog zu der im Verfahren a) (Acylierung) beschriebenen Methode in situ herstellen und ohne Isolierung mit dem entsprechenden Alkohol umsetzen.

Salzbildung:
Salze von Verbindungen der Formel I können in an sich bekannter Weise hergestellt werden. So kann man Salze von Verbindungen der Formel I nach der in den Beispielen beschriebenen Methode oder z.B. durch Reaktion der sauren Gruppen mit Metallverbindungen, wie Alkalimetallsalzen von geeigneten Carbonsäuren, z.B. dem Natriumsalz der α-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Bei sämtlichen weiter vorn genannten Umsetzungen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Gemische von Diastereomeren, z.B. 2R,S-Verbindungen, der Formel I können in an sich bekannter Weise, z.B. durch fraktionierte Kristallisation eines Zwischenprodukts, Chromatographie, etc. in die einzelnen Isomere aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Pharmazeutische Präparate:

Die pharmakologisch verwendbaren Verbindungen der Formel I, deren Hydrate oder Salze können zur Herstellung von pharmazeutischen Präparaten verwendet werden.

Pharmazeutische Präparate enthalten eine wirksame Menge des reinen Wirkstoffs der Formel I selbst oder eine wirksame Menge des Wirkstoffs der Formel I im Gemisch mit anorganischen oder organischen, festen oder flüssigen, pharmazeutisch verwendbaren Trägerstoffen, die sich zur parenteralen oder peroralen Verabreichung eignen.

Beispielsweise verwendet man die Wirkstoffe der Formel I der vorliegenden Erfindung in Form von injizierbaren, z.B. intravenös verabreichbaren, Präparaten oder von Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebrauch hergestellt werden können. Pharmazeutischen Präparate

zur peroralen Anwendung sind sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe, z.B. andere Wirkstoffe, enthalten können, enthalten etwa 0,1 % bis 100 %, insbesondere etwa 1 % bis zu 100 %, des Wirkstoffes.

Die pharmazeutischen Präparate werden in an sich bekannter Weise, z.B. mittels konventioneller Lösungs- oder Lyophilisierungsverfahren, hergestellt.

Verwendung:
Verbindungen der Formel I, deren Hydrate oder pharmazeutisch verwendbare Salze können als antibiotisch wirksame Mittel in Form von pharmazeutischen Präparaten in einem Verfahren zur therapeutischen Behandlung des menschlichen oder tierischen Körpers angewendet werden, z.B. zur Behandlung von Infektionen, welche durch grampositive, z.B. Staphylococcus aureus, oder gramnegative Bakterien und Kokken, z.B. durch Enterobakterien, z.B. Escherichia coli, Klebsiella pneumoniae oder Proteus spp., verursacht werden.

Je nach Art der Infektionen und Zustand des infizierten Organismus verwendet man tägliche Dosen von etwa 0,5 g bis etwa 5 g s.c. i.v., i.m. oder p.o. zur Behandlung von Personen oder Warmblütern von etwa 70 kg Gewicht.

Ausgangsstoffe: Die im Verfahren zur Herstellung der Verbindungen der vorliegenden Erfindung verwendeten Ausgangsmaterialien der Formeln II und IV sind bekannt oder, können, falls sie neu sind, in an sich bekannter Weise hergestellt werden.

Verbindungen der Formel III, welche für die Herstellung von Verbindungen der Formel I entwickelt worden sind, sind neu und ebenfalls

Gegenstand der vorliegenden Verbindung. Diese werden beispielsweise hergestellt, indem man in einem Aminosäureester der Formel

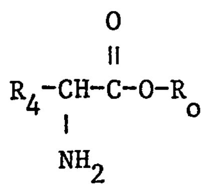

(IX) ,

worin $R_4$ die unter Formel I genannte Bedeutung hat und $R_o$ eine veresternde Gruppe darstellt und eine in $R_4$ vorhandene funktionelle Gruppe gegebenenfalls in geschützter Form vorliegt, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt und die veresternde Gruppe $R_o$ abspaltet und, wenn erwünscht, ein erhältliches racemisches Gemisch von 2R- und 2S-Verbindungen auftrennt und die 2R- oder 2S-Verbindungen isoliert und/oder die erhältliche Carbonsäure in die unter Verfahren a) genannten reaktionsfähigen, funktionellen Derivate überführt.

In einem Aminosäurederivat ist eine veresternde Gruppe $R_o$ beispielsweise Niederalkoxy, z.B. Methoxy, Aethoxy, n-Propoxy, verzweigtes Niederalkoxy, z.B. tert-Butoxy, substituiertes Niederalkoxy, z.B. Benzyloxy, 4-Nitrobenzyloxy, 4-Methoxybenzyloxy oder Diphenylmethoxy, oder Sulfonyloxy, z.B. Niederalkylsulfonyloxy, z.B. Mesyloxy, oder Arylsulfonyloxy, z.B. Tosyloxy.

Eine in $R_4$ vorhandene funktionelle Gruppe, z.B. die Aminogruppe der 2-Aminothiazol-4-yl-, 2-Aminooxazol-4-yl- oder 5-Amino-1,2,4-thiadiazol-3-yl-Gruppe, ist durch übliche Schutzgruppen, z.B. übliche Aminoschutzgruppen, z.B. tert-Butoxycarbonyl (BOC) geschützt.

Eine die Formylierungsreaktion erlaubende Schutzgruppe der 2-Aminogruppe ist weiter vorn unter Verfahren b) beschrieben und ist z.B. Triniederalkylsilyl, z.B. Trimethylsilyl.

Die Umwandlung der 2-Aminogruppe in die 2-Formamidogruppe erfolgt mit den unter Verfahren b) genannten Formylierungsmitteln, z.B. einem gemischten Anhydrid der Ameisensäure mit Mesylchlorid, unter den dort genannten Reaktionsbedingungen.

Die Abspaltung der vorstehenden Gruppe $R_o$ erfolgt in an sich bekannter Weise, z.B. durch Verseifung mit einem basischen Mittel, z.B. Natrium- oder Kaliumhydroxid.

Es können Aminosäurederivate der Formel IX mit 2R- oder 2S-Konfiguration oder 2R,S-Verbindungen bei der Formylierung eingesetzt werden.

Die Trennung eines erhältlichen Gemisches der 2R- und 2S-Verbindungen und die Isolierung der optisch reinen 2R- oder der 2S-Verbindung der Formel (III) kann nach einem der zahlreichen in der Literatur für die Aufspaltung von racemischen Gemischen beschriebenen Verfahren erfolgen.

Beispielsweise können Carbonsäuren der Formel III, worin das 2-C-Atom die S-Konfiguration hat, analog der von Roper J.M. und Bauer D.P. in Synthesis, S. 1041-1043 (1983) angegebenen Methode enzymatisch durch Umsetzung des Isomerengemisches des Niederalkylesters der Formel III, z.B. des Aethylesters, mit einer nicht spezifischen Serinproteinase, z.B. Subtilisin Carlsberg, wobei nur die S-Carbonsäure der Formel III gebildet wird, und Trennung der S-Carbonsäure der Formel III vom R-Ester, erhalten werden.

Vorzugsweise setzt man ein erhältliches racemisches Gemisch der 2R,S-Verbindung der Formel III in methanolischer Lösung mit (S)-1-Phenyläthylamin um. Dabei kristallisiert das Salz der 2S-Verbindung (III) mit (S)-1-Phenyläthylamin aus. Dieses Salz wird gereinigt und nach Umsetzen mit einer Säure, z.B. verdünnter, wässriger Mineralsäure, z.B. wässriger HCl-Lösung, die 2S-Carbonsäure der Formel III erhalten. Das in der methanolischen Mutterlauge gebliebenen 2R-Carbonsäuresalz kann man nach Bedarf durch Ausfällen mit Aether isolieren. Nach Umsetzung dieses Salzes mit einer Mineralsäure erhält man die R-Carbonsäure der Formel III.

Die Ueberführung in die unter Verfahren a) genannten reaktionsfähigen funktionellen Derivate erfolgt mit für Carbonsäuren bekannten Derivatisierungsverfahren.

Verbindungen der Formel IV und ihre Herstellung sind bekannt und z.B. in der US-Patentschrift 4,098,888 beschrieben oder können falls sie neu sind durch Umsetzung einer Verbindung der Formel II mit einer Aminosäure der Formel IX ($R_o$ = H) oder einem reaktionsfähigen, funktionellen Derivat davon, z.B. dem Säurechlorid analog Verfahren a) hergestellt werden.

2-Cephem-Verbindungen der Formel V sind neu. Man kann sie analog dem Acylierungsverfahren a) oder analog dem Formylierungsverfahren b) ausgehend von einer bekannten oder auf an sich bekannte Weise herstellbaren 2-Cephem-Verbindung der Formel

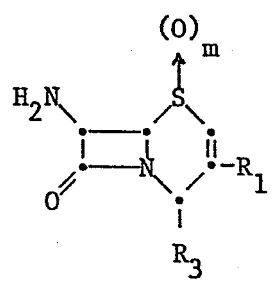

(X)

herstellen. Ausserdem können 2-Cephem-Verbindungen der Formel V als Nebenprodukte bei dem Verfahren a) gebildet werden, insbesondere wenn unter basischen Bedingungen gearbeitet wird.

Verbindungen der Formel VI sind neu und können beispielsweise in an

sich bekannter Weise hergestellt werden, indem man in einer Verbindung der Formel II, worin die 7β-Aminogruppe und die 4-Carboxygruppe gegebenenfalls durch eine die Acylierungsreaktion erlaubende Gruppe geschützt sind, die 7β-Aminogruppe durch Umsetzung mit einem den Acylrest einer Carbonsäure der Formel

$$\begin{array}{l} Hal\text{-}CH_2\text{-}CO\text{-}\underset{\displaystyle NHCHO}{\underset{|}{CH}}\text{-}COOH \end{array} \qquad (XI)$$

worin Hal Halogen bedeutet, einführenden Acylierungsmittel acyliert.

Die Acylierung einer Verbindung der Formel II mit der Carbonsäure der Formel XI oder einem reaktionsfähigen, funktionellen Derivat dieser Carbonsäuren, z.B. dem Säurechlorid, erfolgt analog Verfahren a).

Verbindungen der Formel VII sind neu und können in an sich bekannter Weise hergestellt werden, indem man in einer Verbindung der Formel II worin die 7β-Aminogruppe und die 4-Carboxygruppe gegebenenfalls durch eine die Acylierungsreaktion erlaubende Gruppe geschützt ist, die 7β-Aminogruppe durch Umsetzung mit einem den Acylrest einer Carbonsäure der Formel:

$$X_1\text{-}N\text{=}\underset{\displaystyle NH_2}{\underset{|}{C}}\text{---}\underset{\displaystyle NHCHO}{\underset{|}{CH}}\text{-}COOH \qquad (XII)\ ,$$

worin $X_1$ Wasserstoff, Halogen oder Hydroxy bedeutet, einführenden Acylierungsmittel acyliert.

Die Acylierung einer Verbindung der Formel II mit der Carbonsäure der Formel XII oder einem reaktionsfähigen, funktionellen Derivat dieser Carbonsäure z.B. dem Säurechlorid, erfolgt analog Verfahren a).

Verbindungen der Formel VIII sind neu und können in an sich bekannter Weise nach dem in der Europäischen Patentanmeldung 47014 angegebenen Verfahren durch Umsetzung einer Verbindung der Formel III oder einem reaktionsfähigen, funktionellen Derivat davon mit einem Mercaptan der Formel $R_2$'-H hergestellt werden.

Verbindungen der Formeln IX und X sind bekannt.

Verbindungen der Formeln XI und XII lassen sich beispielsweise durch Formylierung analog Verfahren b) der entsprechenden Aminosäuren herstellen.

Die folgenden Beispiele dienen zur Illustration der Efindung; Temperaturen sind in Celsiusgraden, Wellenlängen der UV-Spektren in Nanometern (nm), $\varepsilon$-Werte in Klammern und für IR-Spektren Wellenzahlen ($cm^{-1}$) angegeben.

In den Beispielen werden folgende Abkürzungen verwendet:

BOC: tert-Butoxycarbonyl

Smp.: Schmelzpunkt

$[\alpha] = [\alpha]_D^{20}$.

Beispiel 1:

a) 3-Acetoxymethyl-7β-[(2R,S)-2-(2-aminooxazol-4-yl)-2-formamidoacetamido]-3-cephem-4-carbonsäurenatriumsalz

Eine Lösung von 4,4 g 3-Acetoxymethyl-7β-[(2R,S)-2-(2-BOC-aminooxazol-4-yl)-2-formamidoacetamido]-3-cephem-4-carbonsäurediphenylmethylester in 20 ml Methylenchlorid wird 1 Stunde mit 19,7 ml Trifluoressigsäure und 3,3 ml Anisol gerührt, mit 250 ml eiskaltem Toluol versetzt und eingedampft. Nach Digerieren des Rückstandes mit Aether, Abnutschen und Trocknen erhält man das Trifluoressigsäuresalz der Titelverbindung als beiges Pulver. Dieses wird in 20 ml Wasser suspendiert und die

Lösung bei 0° mit 1 N Natriumhydroxid-Lösung auf pH 7,2 gestellt und über ein Adsorberharz, z.B. Amberlite ER-180®-Adsorberharz (Hersteller: Rohm+ Haas, Milano), chromatographiert. Man erhält die Titelverbindung vom Rf-Wert 0,9 (Kieselgel 60; Acetonitril/Wasser 1:1); UV (Wasser): 218 (11500) und 265 (10100); [α] = +77 ± 1° (0,969 % in $H_2O$).

b) 3-Acetoxymethyl-7β-[(2R,S)-2-(2-BOC-aminooxazol-4-yl)-2-formamido-acetamido]-3-cephem-4-carbonsäurediphenylmethylester

Zu einer Lösung von 2,8 g 2-(2-BOC-Aminooxazol-4-yl)-2-formamido-essigsäure, 4,4 g 3-Acetoxymethyl-7β-amino-3-cephem-4-carbonsäure-diphenylmethylester und 1,8 g 1-Hydroxybenztriazol in 30 ml Tetrahydrofuran wird bei -5° eine Lösung von 2,5 g N,N'-Dicyclohexylcarbodiimid in 10 ml Tetrahydrofuran zugetropft. Man lässt 1 Stunde bei 0° und 2 Stunden bei ca. 25° weiterrühren. Zur Aufarbeitung filtriert man den Niederschlag ab, engt das Reaktionsgemisch ein, nimmt den Rückstand in Essigsäureäthylester auf und schüttelt nacheinander mit kalter, gesättigter Natriumhydrogencarbonatlösung, ca. 2N Citronensäure und nochmals mit gesättigter Natriumhydrogencarbonat- und gesättigter Kochsalzlösung aus. Nach Trocknen der organischen Phase über Natriumsulfat und Eindampfen erhält man die Titelverbindung b) als Rohprodukt, welche man durch Säulenchromatographie (Kieselgel M+N; Toluol/Essigsäureäthylester) reinigt; Rf-Wert 0,85 (Kieselgel 60 (Merck); Methylenchlorid/Aethanol 9:1)

Man stellt die Ausgangsmaterialien folgendermassen her:

c) 2-(2-BOC-Aminooxazol-4-yl)-2-formamidoessigsäure

Man versetzt 2,8 g 2-(2-Di-BOC-aminooxazol-4-yl)-2-formamidoessigsäuremethylester in 50 ml Aethanol mit einer Lösung von 2,2 g Kaliumhydroxid in 20 ml Wasser und rührt 30 Minuten bei Raumtemperatur. Anschliessend engt man das Reaktionsgemisch im Vakuum ein, verdünnt mit Essigsäureäthylester und säuert mit 4N Salzsäure auf pH 1,5 an. Man trennt

die organische Phase ab, schüttelt mit gesättigter Kochsalzlösung aus, trocknet über Natriumsulfat und dampft ein. Nach Digerieren des Rückstandes mit Aether erhält man die Titelverbindung c) mit einem Schmelzpunkt von 107-109°.

d) 2-(2-Di-BOC-aminooxazol-4-yl)-2-formamidoessigsäuremethylester

Zu einer Lösung von 5,5 g des Formiatsalzes des 2-(Di-BOC-aminooxazol-4-yl)-2-aminoessigsäuremethylesters in 15 ml Methylenchlorid tropft man bei -5° bis 0° 4 ml Triäthylamin und dann eine Lösung von 1,6 g Methansulfochlorid in 7 ml Methylenchlorid zu. Man lässt noch 90 Minuten weiterrühren. Zur Aufarbeitung wird das Reaktionsgemisch eingeengt, der Rückstand in Essigsäureäthylester gelöst und nacheinander mit gesättigter Natriumhydrogencarbonatlösung, Wasser, 1N Salzsäure und gesättigter Kochsalzlösung ausgeschüttelt. Nach Trocknen der organischen Phase über Natriumsulfat und Eindampfen erhält man die Titelverbindung d) als Rohprodukt, das ohne Reinigung verarbeitet wird.

e) 2-(2-Di-BOC-aminooxazol-4-yl)-2-aminoessigsäuremethylesterformiat

2 g 2-(2-Di-BOC-aminooxazol-4-yl)-2-Z-methoxyiminoessigsäuremethylester werden in 12,5 ml Ameisensäure, 12,5 ml Methanol und 1,25 ml Wasser gelöst. Bei ca. 0-10° werden 30 Minuten lang 15,9 g Zinkstaub portionsweise eingetragen. Nach 30-minütigem Rühren bei dieser Temperatur wird der Feststoff abgenutscht und das Filtrat eingeengt. Man löst den Rückstand in Wasser und schüttelt mit Aether aus. Nach Lyophilisieren der wässerigen Phase erhält man die Titelverbindung e) als Formiatsalz.

f) 2-(2-Di-BOC-aminooxazol-4-yl)-2-Z-methoxyiminoessigsäuremethylester

9,9 g 2-(2-Aminooxazol-4-yl)-2-Z-methoxyiminoessigsäuremethylester werden in 32,7 g Di-tert-butyldicarbonat suspendiert. Nach Zugabe

von 0,5 g 4-Dimethylaminopyridin wird 30 Minuten bei Raumtemperatur gerührt, wobei allmählich Lösung eintritt. Die Lösung wird mit Essigsäureäthylester verdünnt und nacheinander mit gesättigter Kochsalzlösung, 0,1 N Salzsäure und erneut mit gesättigter Kochsalzlösung ausgeschüttelt. Die organische Phase wird über Natriumsulfat getrocknet und im Vakuum eingedampft. Der Rückstand wird mit Hexan kristallisiert. Die Titelverbindung f) schmilzt bei 85 - 87°.

g) 2-(2-Aminooxazol-4-yl)-2-Z-methoxyiminoessigsäuremethylester

Man lässt 47,6 g 4-Brom-2-methoxyiminoacetessigsäuremethylester und 120 g Harnstoff in 480 ml Dimethylformamid 1 Stunde auf 100° erwärmen, versetzt mit zusätzlichen 30 g Harnstoff und lässt eine weitere Stunde bei 100° reagieren. Man giesst das dunkle Reaktionsgemisch auf Eiswasser, extrahiert mit Essigsäureäthylester erschöpfend, schüttelt die organische Phase dreimal mit Wasser und zweimal mit gesättigter Kochsalz-Lösung und trocknet über Natriumsulfat. Nach Eindampfen im Vakuum kristallisiert man die Titelverbindung g) aus einem Essigsäureäthylester-Diäthyläther Gemisch aus. Schmelzpunkt: 142 - 147°.

Beispiel 2: 3-Acetoxymethyl-7β-(2R,S)-2-(2-aminooxazol-4-yl)-2-formamidoacetamido]-3-cephem-4-carbonsäurepivaloyloxymethylester

Zu einer Lösung von 2,4 g 3-Acetoxymethyl-7β-[(2R,S)-2-(2-aminooxazol-4-yl)-2-formamidoacetamido]-3-cephem-4-carbonsäure-natriumsalz in 15 ml Dimethylformamid werden bei 0° in 5 Minuten 1,8 ml Iodmethylpivalat zugetropft und eine Stunde bei Raumtemperatur weitergerührt. Das Reaktionsgemisch wird mit Essigsäureäthylester verdünnt, die Lösung mit gesättigter Kochsalzlösung ausgeschüttelt, über Natriumsulfat getrocknet und im Vakuum eingedampft. Der Rückstand wird an Kieselgel 60 mit Methylenchlorid-Essigsäureäthylester 1:1 und Essigsäureäthylester chromatographiert und ergibt die Titelverbindung.

Beispiel 3:

a) 3-Acetoxymethyl-7β-[(2R,S)-2-(2-aminothiazol-4-yl)-2-formamidoacetamido]-3-cephem-4-carbonsäurenatriumsalz

Der gemäss Beispiel 3b) erhältliche rohe 3-Acetoxymethyl-7β-[(2R,S)-2-(2-BOC-aminothiazol-4-yl)-2-formamidoacetamido]-3-cephem-4-carbonsäurediphenylmethylester wird in 2 ml Anisol suspendiert. Nach Zugabe von 10 ml Trifluoressigsäure rührt man das Reaktionsgemisch eine halbe Stunde bei Raumtemperatur und dampft im Vakuum unterhalb von 40° ein. Man versetzt den öligen Rückstand unter Rühren mit 100 ml Aether. Der dabei gebildete kristalline Niederschlag wird abfiltriert, mit Aether gewaschen, in einem Gemisch von 5 ml Wasser und 25 ml Methanol gelöst und die Lösung mit 1 N Natriumhydroxid-Lösung auf einen pH-Wert von 7,2 eingestellt. Man engt die Lösung im Vakuum ein und chromatographiert das Konzentrat an einem Adsorberharz, z.B. Amberlite ER-180®-Adsorberharz. Die mit Wasser eluierten produkthaltigen Fraktionen werden vereinigt, im Vakuum eingeengt und anschliessend im Hochvakuum lyophilisiert. Die erhaltene Titelverbindung enthält 5,3 % Wasser und liegt als Diastereomerengemisch vor, in welchem das S- gegenüber dem R-Diastereomeren im Verhältnis von ca. 3,5:1 überwiegt; IR (Nujol): 3310, 3200, 1765, 1670, 1610, 1520, 1235.

b) 3-Acetoxymethyl-7β-[(2R,S)-2-(2-BOC-aminothiazol-4-yl)-2-formamidoacetamido]-3-cephem-4-carbonsäurediphenylmethylester

Zu einer auf -5° gekühlten Lösung von 1,5 g (0,005 Mol) (2R,S)-2-(2-BOC-Aminothiazol-4-yl)-2-formamidoessigsäure, 2,19 g (0,005 Mol) 3-Acetoxymethyl-7β-amino-3-cephem-4-carbonsäurediphenylmethylester und 0,90 g (0,006 Mol) 1-Hydroxybenztriazol (Wassergehalt ca. 10 %) in 30 ml Tetrahydrofuran tropft man eine Lösung von 1,13 g (0,0055 Mol) Dicyclohexylcarbodiimid in 15 ml Tetrahydrofuran. Das Reaktionsgemisch wird eine Stunde bei 0° und zwölf Stunden bei Raumtemperatur gerührt, anschliessend filtriert und das Filtrat im Vakuum eingedampft. Den Rückstand versetzt man mit Aethylacetat, so dass die Titelverbindung b) ausfällt.

c) (2R,S)-2-(2-BOC-aminothiazol-4-yl)-2-formamidoessigsäure

Zu einer auf 0° gekühlten Suspension von 10 g (0,0366 Mol) (2R,S)-2-Amino-2-(2-BOC-aminothiazol-4-yl)-essigsäure in einem Gemisch von 100 ml Tetrahydrofuran und 100 ml Eisessig tropft man unter Rühren innerhalb von 5 Minuten 80 ml Ameisenessigsäureanhydrid. Das allmählich in Lösung gehende Reaktionsgemisch wird noch eine Stunde bei 0° gerührt und anschliessend im Vakuum unterhalb von 40° eingedampft. Man löst den Kolbeninhalt in 100 ml Toluol, dampft erneut im Vakuum ein und löst den Rückstand in 100 ml Aethylacetat. Beim Abkühlen auf 0° kristallisiert die Titelverbindung c) aus, die nach Waschen mit Aethylacetat und Aether bei 127° unter Zersetzung schmilzt.

d) (2R,S)-2-(2-BOC-aminothiazol-4-yl)-2-formamidoessigsäureäthylester und (2R,S)-2-(2-BOC-aminothiazol-4-yl)-2-formamidoessigsäure

33 ml Ameisenessigsäureanhydrid werden innerhalb von 10 Minuten bei 5-10° zu einer gut gerührten Lösung von 10g(0,0332 Mol) (2R,S)-2-Amino-2-(2-BOC-aminothiazol-4-yl)-essigsäureäthylester in 30 ml Essigsäure gegeben. Nach 20 Minuten entfernt man das Kühlbad, rührt das Reaktionsgemisch noch eine halbe Stunde weiter und dampft es dann im Vakuum unterhalb von 40° ein. Der Rückstand wird zwischen Aethylacetat und eiskalter wässriger Natriumbicarbonatlösung verteilt, die organische Phase nacheinander mit 2N Citronensäure, wässriger Natriumbicarbonatlösung und gesättigter wässriger Natriumchloridlösung gewaschen, anschliessend über Natriumsulfat getrocknet und eingedampft. Das Rohprodukt reinigt man durch Chromatographie über Kieselgel der Korngrösse 0,063-0,200 mm unter Verwendung von Methylenchlorid und Methylenchlorid/Methanol (30:1) als Elutionsmittel. Die produkthaltigen

Fraktionen werden eingedampft und aus Aether/Hexan umkristallisiert. Der erhaltene (2R,S)-2-(2-BOC-aminothiazol-4-yl)-2-formamidoessigsäureäthylester, der bei 110-114° schmilzt, wird in einem Gemisch von 10 ml Methanol und 30 ml 1N Natronlauge gelöst. Man rührt 20 Minuten bei Raumtemperatur, gibt dann 30 ml 1N Salzsäure zu und extrahiert das Reaktionsgemisch mit Aethylacetat. Die mit wenig Wasser gewaschene organische Phase wird über Natriumsulfat getrocknet und eingeengt. Dabei kristallisiert die (2R,S)-2-(2-BOC-aminothiazol-4-yl)-2-formamidoessigsäure aus, die nach Waschen mit Aether bei 125° unter Zersetzung schmilzt.

Beispiel 4:

a) 3-Acetoxymethyl-7β-[(2R)-2-(2-aminothiazol-4-yl)-2-formamidoacetamido]-3-cephem-4-carbonsäurenatriumsalz

3,4 g (0,0047 Mol) 3-Acetoxymethyl-7β-[(2R)-2-(2-BOC-aminothiazol-4-yl)-2-formamidoacetamido]-3-cephem-4-carbonsäurediphenylmethylester werden in 3 ml Anisol suspendiert. Nach Zutropfen von 15 ml Trifluoressigsäure innerhalb von fünf Minuten rührt man das Reaktionsgemisch noch eine halbe Stunde bei Raumtemperatur, dampft unterhalb von 40° im Vakuum ein und gibt unter Rühren 100 ml Aether zum öligen Rückstand. Der dabei gebildete Niederschlag wird abfiltriert, mit Aether gewaschen, in einem Gemisch von 40 ml Methanol und 10 ml Wasser gelöst und die Lösung mit 1 N Natriumhydroxid-Lösung auf einen pH-Wert von 7,2 eingestellt. Man engt die Lösung im Vakuum auf ein Volumen von ca. 10 ml ein und chromatographiert das Konzentrat an einem Adsorberharz, z.B. Amberlite ER-180®-Adsorberharz. Die mit Wasser und einem Wasser/Isopropanol-Gemisch (92,5:7,5) eluierten produkthaltigen Fraktionen werden vereinigt, im Vakuum eingeengt und im Hochvakuum lyophilisiert. Die erhaltene Titelverbindung enthält 4,8 % Wasser; [α] = +36 ± 1° (0,517 % in $H_2O$); IR (Nujol): 3310, 3200, 1765, 1670, 1610, 1520, 1235.

b) 3-Acetoxymethyl-7β-[(2S)-2-(2-aminothiazol-4-yl)-2-formamido-acetamido]-3-cephem-4-carbonsäurenatriumsalz

Analog Beispiel 4a) erhält man ausgehend von 3,3 g 3-Acetoxymethyl-7β-[(2S)-2-(2-BOC-aminothiazol-4-yl)-2-formamidoacetamido]-3-cephem-4-carbonsäurediphenylmethylester nach Zugabe von 3 ml Anisol und 15 ml Trifluoressigsäure die Titelverbindung b) mit einem Wassergehalt von 4,8 %; [α] = +102 ± 1° (0,366 % in $H_2O$); IR (Nujol): 3310, 3200, 1765, 1670, 1610, 1520, 1235.

c) 3-Acetoxymethyl-7β-[(2R)-2-(2-BOC-aminothiazol-4-yl)-2-formamido-acetamido]-3-cephem-4-carbonsäurediphenylmethylester und 3-Acetoxy-methyl-7β-[(2S)-2-(2-BOC-aminothiazol-4-yl)-2-formamidoacetamido]-3 cephem-4-carbonsäurediphenylmethylester

Zu einer auf -5° gekühlten Lösung von 3,01 g (0,01 Mol) (2R,S)-2-(2-BOC-Aminothiazol-4-yl)-2-formamidoessigsäure, 4,38 g (0,01 Mol) 3-Acetoxymethyl-7β-amino-3-cephem-4-carbonsäurediphenylmethylester und 1,8 g (0,012 Mol) 1-Hydroxybenztriazol (Wassergehalt ca. 10 %) in 70 ml Tetrahydrofuran tropft man eine Lösung von 2,26 g (0,011 Mol) Dicyclohexylcarbodiimid in 20 ml Tetrahydrofuran. Das Reaktionsgemisch wird eine Stunde bei 0° und zwei Stunden bei Raumtemperatur gerührt, nach Stehenlassen über Nacht filtriert und das Filtrat im Vakuum eingedampft. Man löst den Rückstand in 100 ml Tetrahydrofuran, gibt zur Lösung 50 g Kieselgel der Korngrösse 0,063-0,200 mm und dampft die Aufschlämmung im Rotationsverdampfer unterhalb 40° ein. Die Mischung wird mit 100 ml Hexan versetzt, eingedampft, in 100 ml Hexan suspendiert und auf eine mit 600 g Kieselgel der Korngrösse 0,04-0,063 mm beschickte Flash-Säule gebracht. Man trennt mit einem Aethylacetat/Hexan-Gemisch (4:1) bei einem Druck von 0,4 bar. Man erhält die R-Titelverbindung, Smp. 128-132°, und die S-Titelverbindung, Smp. 163°-167°.

Beispiel 5: 3-(1-Methyl-1H-tetrazol-5-ylthiomethyl)-7β-[(2R,S)-2-(2-aminothiazol-4-yl)-2-formamidoacetamido]-3-cephem-4-carbonsäure-natriumsalz

Analog Beispiel 3b) erhält man durch Umsetzung einer Lösung von 2,26 g (0,0075 Mol) (2R,S)-2-(2-BOC-Aminothiazol-4-yl)-2-formamido-essigsäure, 3,71 g (0,0075 Mol), 3-(1-Methyl-1H-tetrazol-5-ylthio-methyl)-7β-amino-3-cephem-4-carbonsäurediphenylmethylester und 1,24 g (0,0083 Mol) 1-Hydroxybenztriazol in 60 ml Tetrahydrofuran, sowie einer Lösung von 1,63 g (0,0079 Mol) Dicyclohexylcarbodiimid in 20 ml Tetrahydrofuran den 3-(1-Methyl-1H-tetrazol-5-ylthiomethyl)-7β-[(2R,S)-2-(2-BOC-aminothiazol-4-yl)-2-formamidoacetamido]-3-cephem-4-carbonsäure-diphenylmethylester; Smp. 182° (unter Zersetzung).

Dieser wird analog Beispiel 3a) mit einem Gemisch von 15 ml Trifluoressigsäure und 3 ml Anisol behandelt. Die Isolierung des Produkts erfolgt chromatographisch an einem Adsorberharz, z.B. Amberlite ER-180®-Adsorberharz, wobei als Eluiermittel Wasser und ein Gemisch Wasser/Isopropanol (19:1) verwendet werden. Die nach Lyophilisation erhaltene Titelverbindung enthält 4,1 % Wasser; [α] = -19 ± 1° (0,516 % in $H_2O$); IR (Nujol): 3315, 3200, 1768, 1670, 1610, 1520.

Beispiel 6:

a) 3-Carbamoyloxymethyl-7β-[(2R)-2-(2-aminothiazol-4-yl)-2-formamido-acetamido]-3-cephem-4-carbonsäurenatriumsalz

Analog Beispiel 4a) erhält man ausgehend von 3,3 g 3-Carbamoyloxy-methyl-7β-[(2R)-2-(2-BOC-aminothiazol-4-yl)-2-formamidoacetamido]-3-cephem-4-carbonsäurediphenylmethylester nach Zugabe von 3 ml Anisol und 15 ml Trifluoressigsäure die Titelverbindung a) mit einem Wassergehalt von 6,6 %; [α] = +29 ± 1° (0,552 % in $H_2O$); IR (Nujol): 3318, 3200, 1765, 1668, 1610, 1518.

b) 3-Carbamoyloxymethyl-7β-[(2S)-2-(2-aminothiazol-4-yl)-2-formamidoacetamido]-3-cephem-4-carbonsäurenatriumsalz

Analog Beispiel 4a) erhält man ausgehend von 3,3 g 3-Carbamoyloxymethyl-7β-[(2S)-2-(2-BOC-aminothiazol-4-yl)-2-formamidoacetamido]-3-cephem-4-carbonsäurediphenylmethylester nach Zugabe von 3 ml Anisol und 15 ml Trifluoressigsäure die Titelverbindung b) mit einem Wassergehalt von 6,5 %; [α] = +92 ± 1°; IR (Nujol): 3318, 3200, 1765, 1668, 1610, 1518.

c) 3-Carbamoyloxymethyl-7β-[(2R)-2-(2-BOC-aminothiazol-4-yl)-2-formamidoacetamido]-3-cephem-4-carbonsäurediphenylmethylester und 3-Carbamoyloxymethyl-7β-[(2S)-2-(2-BOC-aminothiazol-4-yl)-2-formamidoacetamido]-3-cephem-4-carbonsäurediphenylmethylester

Zu einer auf -5° gekühlten Lösung von 1,13 g (0,00375 Mol) (2R,S)-2-(2-BOC-Aminothiazol-4-yl)-2-formamidoessigsäure, 1,65 g (0,00375 Mol) 3-Carbamoyloxymethyl-7β-amino-3-cephem-4-carbonsäurediphenylmethylester und 0,62 g (0,00413 Mol) 1-Hydroxybenztriazol (Wassergehalt ca. 10 %) in 30 ml Tetrahydrofuran tropft man eine Lösung von 0,81 g (0,00393 Mol) Dicyclohexylcarbodiimid in 10 ml Tetrahydrofuran. Das Reaktionsgemisch wird eine Stunde bei 0° und 12 Stunden bei Raumtemperatur gerührt, filtriert und das Filtrat im Vakuum eingedampft. Den Rückstand verteilt man zwischen Aethylacetat und eiskalter wässriger Natriumbicarbonatlösung, wäscht die organische Phase nacheinander mit 2 N Citronensäure, wässriger Natriumbicarbonatlösung und gesättigter wässriger Natriumchloridlösung, trocknet über Natriumsulfat und dampft im Vakuum ein. Man reinigt den Rückstand mittels Flash-Chromatographie an 280 g Kieselgel der Korngrösse 0,04 - 0,06 mm. Bei einem Druck von 0,25 bar werden mit einem Aethylacetat/Hexan-Gemisch (2:1) die R-Titelverbindung und mit Aethylacetat die S-Titelverbindung eluiert.

Beispiel 7:

a) 7β-[(2R)-2-(2-Aminothiazol-4-yl)-2-formamidoacetamido]-3-cephem-4-carbonsäurenatriumsalz

2,5 g (0,00385 Mol) 7β-[(2R)-2-(2-BOC-aminothiazol-4-yl)-2-formamidoacetamido]-3-cephem-4—carbonsäurediphenylmethylester werden in 3 ml Anisol suspendiert. Nach Zugabe von 15 ml Trifluoressigsäure rührt man das Reaktionsgemisch 15 Minuten bei Raumtemperatur, dampft unterhalb von 40° im Vakuum ein und gibt unter Rühren 100 ml Aether zum öligen Rückstand. Der dabei gebildete Niederschlag (Trifluoressigsäuresalz der Titelverbindung) wird abfiltriert, mit Aether gewaschen und mit einem Gemisch von 30 ml Wasser und 30 ml Methanol versetzt. Man engt dann die mit 1N Natriumhydroxid-Lösung auf einen pH-Wert von 7,2 eingestellte Lösung im Vakuum ein und chromatographiert das Konzentrat an einem Adsorberharz, z.B. Amberlite ER-180®-Adsorberharz. Die mit Wasser eluierten produkthaltigen Fraktionen werden vereinigt, im Vakuum eingeengt und anschliessend im Hochvakuum lyophilisiert. Die erhaltene Titelverbindung a) enthält 6,3 % Wasser; [α] = +95 ± 1° (0,559 % in $H_2O$); IR (Nujol): 3290, 3185, 1755, 1680, 1645, 1595, 1515.

b) 7β-[(2S)-2-(2-Aminothiazol-4-yl)-2-formamidoacetamido]-3-cephem-4-carbonsäurenatriumsalz

Analog Beispiel 7a) erhält man ausgehend von 2,1 g (0,00323 Mol) 7β-[(2S)-2-(2-BOC-aminothiazol-4-yl)-2-formamidoacetamido]-3-cephem-4-carbonsäurediphenylmethylester die Titelverbindung b) mit einem Wassergehalt von 5,4 %; [α] = +160 ± 1° (c = 0,584 % in $H_2O$); IR (Nujol): 3270, 3190, 1755, 1650, 1600, 1520.

c) 7β-[(2R,S)-2-(2-Aminothiazol-4-yl)-2-formamidoacetamido]-3-cephem-4-carbonsäurenatriumsalz

7,14 g (0,011 Mol) 7β-[(2R,S)-2-(2-BOC-aminothiazol-4-yl)-2-formamido-

acetamido]-3-cephem-4-carbonsäurediphenylmethylester werden in 6 ml Anisol suspendiert und bei Raumtemperatur unter Rühren innerhalb von 5 Minuten mit 30 ml Trifluoressigsäure versetzt. Man rührt 20 Minuten weiter und dampft dann das Reaktionsgemisch bei ca. 30° im Vakuum ein. Nach Zugabe von 150 ml Aether zum öligen Rückstand wird der gebildete Niederschlag (Trifluoressigsäuresalz der Titelverbindung) abfiltriert, mit Aether gewaschen und in einem Gemisch von 30 ml Methanol und 7,5 ml Wasser gelöst. Man engt dann die mit 1N-Natronlauge auf einen pH-Wert von 7,2 gestellte Lösung im Vakuum ein und chromatographiert das Konzentrat an einem Adsorberharz, z.B. Amberlite ER-180®-Adsorberharz, wobei man zunächst mit Wasser, dann mit einem Gemisch Wasser/Isopropanol (19:1) eluiert. Die produkthaltigen Fraktionen werden vereinigt, im Vakuum eingedampft und im Hochvakuum lyophilisiert. Die erhaltene Titelverbindung c) enthält 7,1 % Wasser und liegt als Gemisch von Diastereomeren vor, in dem die R-Komponente gegenüber der S-Komponente im Verhältnis von ca. 4:3 überwiegt; $[\alpha] = +107 \pm 1°$ (0,487 % in $H_2O$); IR (Nujol): 3300, 3195, 1760, 1665, 1600, 1515.

d) <u>7β-[(2R)-2-(2-BOC-aminothiazol-4-yl)-2-formamidoacetamido]-3-cephem-4-carbonsäurediphenylmethylester</u> und <u>7β-[(2S)-2-(2-BOC-aminothiazol-4-yl)-2-formamidoacetamido]-3-cephem-4-carbonsäurediphenylmethylester</u>

Analog Beispiel 4c) setzt man 10 g (0,0332 Mol) (2R,S)-2-(2-BOC-aminothiazol-4-yl)-2-formamidoessigsäure mit 12,16 g (0,0332 Mol) 7β-Amino-3-cephem-4-carbonsäurediphenylmethylester um. Die Auftrennung des gebildeten Diastereomerengemischs erfolgt mittels Flash-Chromatographie an 1000 g Kieselgel der Korngrösse 0,04-0,063 mm, wobei durch Elution mit Aethylacetat/Hexan (3:1) die (R)-Titelverbindung und mit Aethylacetat/Hexan (4:1) die (S)-Titelverbindung als Rohprodukte erhalten werden.

e) 7β-[(2R,S)-2-(2-BOC-aminothiazol-4-yl)-2-formamidoacetamido]-3-cephem-4-carbonsäurediphenylmethylester

Analog Beispiel 4c) setzt man 5 g (0,0166 Mol) (2R,S)-2-(2-BOC-aminothiazol-4-yl)-2-formamidoessigsäure und 6,08 g (0,0166 Mol) 7β-Amino--3-cephem-4-carbonsäurediphenylmethylester um. Die Reinigung des Produkts erfolgt mittels Flash-Chromatographie an 600 g Kieselgel der Korngrösse 0,04-0,063 mm, wobei durch Elution mit einem Gemisch Methylenchlorid/Methanol (25:1) die Titelverbindung e) als Rohprodukt erhalten wird.

Beispiel 8:

a) 7β-[(2R)-2-(2-Aminothiazol-4-yl)-2-formamidoacetamido]-3-cephem-4-carbonsäurepivaloyloxymethylester

Zu einer Suspension von 0,746 g (0,00172 Mol) 7β-[(2R)-2-(2-Aminothiazol-4-yl)-2-formamidoacetamido]-3-cephem-4-carbonsäurenatriumsalz (Wassergehalt: 6,3 %) in 7 ml Dimethylformamid gibt man bei 5° 0,52 g (0,00215 Mol) Jodmethylpivalat. Das Reaktionsgemisch wird eine Stunde bei 10° gerührt und dann zwischen 50 ml Aethylacetat und 20 ml Wasser verteilt. Man wäscht die organische Phase nacheinander mit eiskalter gesättigter Natriumbicarbonatlösung, Wasser und gesättigter Kochsalzlösung, dampft im Vakuum ein und versetzt den Rückstand unter Rühren mit 30 ml Aether. Dabei fällt die gewünschte Verbindung in kristalliner Form an. Die weitere Reinigung erfolgt mittels Flash-Chromatogrpahie an Kieselgel der Korngrösse 0,04-0,063 mm. Zur Elution verwendet man ein Methylenchlorid/Methanol-Gemisch (15:1). Die erhaltene (R)-Titelverbindung schmilzt bei 175-180°; [α] = +52 ± 1° (0,465 % in $H_2O$).

b) 7β-[(2S)-2-(2-Aminothiazol-4-yl)-2-formamidoacetamido]-3-cephem-4-carbonsäurepivaloyloxymethylester

Analog Beispiel 8a) erhält man ausgehend von 7β-[(2S)-2-(2-Aminothiazol-4-yl)-2-formamidoacetamido]-3-cephem-4-carbonsäurenatriumsalz und Jodmethylpivalat die S-Titelverbindung, Smp. 173 - 175°; [α] = + 105 ± 1° (0,464 % in $H_2O$).

Beispiel 9: 3-(1,2,3-Thiadiazol-5-ylthiomethyl)-7β-[(2R,S)-2-(2-aminothiazol-4-yl)-2-formamidoacetamido]-3-cephem-4-carbonsäurenatriumsalz

Eine auf pH 7 eingestellte Lösung von 1,6 g (0,00319 Mol) 3-Acetoxymethyl-7β-[(2R)-2-(2-aminothiazol-4-yl)-2-formamidoacetamido]-3-cephem-4-carbonsäurenatriumsalz und 0,64 g Natrium-1,2,3-thiadiazol-5-thiolat-dihydrat (0,00363 Mol) in 20 ml Wasser wird 5 Stunden bei 75° gerührt. Durch sukzessive Zugabe (in Zeitabständen von 45 Minuten) von 0,1 N Natronlauge zum Reaktionsgemisch werden neutrale Reaktionsbedingungen eingehalten. Das Reaktionsgemisch wird dann auf ein Volumen von ca. 5 ml eingeengt und an einem Adsorberharz, z.B. Amberlite ER-180®-Adsorberharz chromatographiert, wobei man zunächst mit Wasser, dann mit einem Gemisch Wasser/Isopropanol (19:1) eluiert. Die produkthaltigen Fraktionen werden vereinigt, im Vakuum eingeengt und anschliessend im Hochvakuum lyophilisiert. Die erhaltene Titelverbindung enthält 8 % Wasser und liegt als Gemisch von Diastereomeren vor, in dem die R-Komponente gegenüber der S-Komponente im Verhältnis von ca. 2:1 überwiegt; [α] = -30 ± 1° (0,582 % in $H_2O$); IR (Nujol): 3300, 3190, 1760, 1660, 1600, 1515.

Beispiel 10:

a) 3-(1,2,3-Thiadiazol-5-ylthiomethyl)-7β-[(2R)-2-(2-aminothiazol-4-yl)-2-formamidoacetamido]-3-cephem-4-carbonsäurenatriumsalz

Eine Lösung von 5,4 g (0,00692 Mol) 3-(1,2,3-Thiadiazol-5-ylthiomethyl)-7β-[(2R)-2-(2-BOC-aminothiazol-4-yl)-2-formamidoacetamido]-3-cephem-4-carbonsäurediphenylmethylester in 7,5 ml Anisol und 37,5 ml Trifluoressigsäure wird eine halbe Stunde bei Raumtemperatur gerührt und anschliessend im Vakuum bei maximal 40° eingedampft. Nach Zugabe von 160 ml Aether zum öligen Rückstand bildet sich beim Rühren ein Niederschlag, den man abfiltriert, mit Aether wäscht, trocknet und in einem Gemisch von 40 ml Methanol und 20 ml Wasser suspendiert. Die Suspension wird mit 2N Natriumhydroxid-Lösung auf einen pH-Wert von 7,2 eingestellt, die dabei resultiertende Lösung im Vakuum bei maximal 40° eingeengt und das erhaltene Konzentrat an einem Adsorberharz, z.B. Amberlite ER-180®-Adsorberharz (Säulendurchmesser 3 cm; Säulenhöhe 30 cm) chromatographiert. Man eluiert zunächst mit Wasser, dann mit einem Gemisch Wasser/Isopropanol (19:1). Die noch leicht verunreinigten produkthaltigen Fraktionen werden vereinigt, im Vakuum bei maximal 40° eingeengt und im Hochvakuum lyophilisiert. Zur weiteren Reinigung wird das Lyophilisat (2,1 g) in 8 ml Wasser gelöst und an 150 g silyliertem Kieselgel, z.B. OPTI-UP $C_{12}$® Kieselgel der Korngrösse 0,04-0,063 mm (Art. Nr. 2067, Antec AG, CH-4431 Bennwil) chromatographiert. Man eluiert zuerst mit Wasser (500 ml) und anschliessend mit einem Gemisch (2000 ml) Wasser/Acetonitril (97:3). Die produkthaltigen Fraktionen werden vereinigt, im Vakuum bei 40° eingeengt und im Hochvakuum lyophilisiert. Die erhaltene Titelverbindung b) enthält 5,9 % Wasser; [α] = -55 ± 1° (0,491 % in $H_2O$); IR (Nujol): 3305, 3190, 1765, 1668, 1605, 1505.

b) <u>3-(1,2,3-Thiadiazol-5-ylthiomethyl)-7β-[(2S)-2-(2-aminothiazol-4-yl)-2-formamidoacetamido]-3-cephem-4-carbonsäurenatriumsalz</u>

Eine Lösung von 3,1 g (0,00397 Mol) 3-(1,2,3-Thiadiazol-5-ylthiomethyl)-7β-[(2S)-2-(2-BOC-aminothiazol-4-yl)-2-formamidoacetamido]-3-cephem-4-carbonsäurediphenylmethylester in 5 ml Anisol und 15 ml Trifluoressigsäure wird eine halbe Stunde bei Raumtemperatur gerührt und anschliessend im Vakuum bei maximal 40° eingedampft. Nach Zugabe von ca. 50 ml Aether zum öligen Rückstand bildet sich beim Rühren ein Niederschlag,

den man abfiltriert, mit Aether wäscht, trocknet und in einem Gemisch von 30 ml Methanol und 10 ml Wasser suspendiert. Die Suspension wird mit 2 N Natriumhydroxid-Lösung auf einen pH-Wert von 7,2 eingestellt. Dabei resultiert eine nahezu klare Lösung, die man filtriert und im Vakuum bei maximal 40° auf ein Volumen von ca. 10 ml einengt. Das Konzentrat wird an einem Adsorberharz, z.B. Amberlite ER-180®-Adsorberharz (Säulendurchmesser 3 cm; Säulenhöhe 30 cm) chromatographiert, wobei man zuerst mit Wasser, dann mit einem Gemisch Wasser/Isopropanol (97:3) eluiert. Die produkthaltigen Fraktionen werden vereinigt, im Vakuum bei maximal 40° eingeengt und im Hochvakuum lyophilisiert. Um noch Spuren von Verunreinigungen zu entfernen, löst man das Lyophilisat in ca. 15 ml Wasser und reinigt es mittels Flashchromatographie an 90 g syliertem Kieselgel, z.B. OPTI-UP $C_{12}$®-Kieselgel der Korngrösse 0,04 - 0,063 mm, wobei man zuerst mit Wasser (500 ml) und anschliessend mit einem Gemisch (1000 ml) Wasser/Acetonitril (98:2) eluiert. Die produkthaltigen Fraktionen werden vereinigt, im Vakuum bei maximal 40° eingeengt und im Hochvakuum lyophilisiert. Die erhaltene Titelverbindung c) enthält 6,4 % Wasser; [α] = +2 ± 1° (0,421 % in $H_2O$); IR (Nujol): 3305, 3200, 1762, 1668, 1605, 1515.

c) 3-(1,2,3-Thiadiazol-5-ylthiomethyl)-7β-[(2R)-2-(2-BOC-aminothiazol-4-yl)-2-formamidoacetamido]-3-cephem-4-carbonsäurediphenylmethylester und 3-[1,2,3-Thiadiazol-5-ylthiomethyl)-7β-[(2S)-2-(2-BOC-aminothiazol-4-yl)-2-formamidoacetamido]-3-cephem-4-carbonsäurediphenylmethylester

Zu einer auf -5° gekühlten Lösung von 9,0 g (0,0181 Mol) 3-(1,2,3-Thiadiazol-5-ylthiomethyl)-7β-amino-3-cephem-4-carbonsäurediphenylmethylester [Herstellung siehe J. Med. Chem. 22, 1214 (1979)], 5,46 g (0,0181 Mol (2R,S)-2-(2-BOC-aminothiazol-4-yl)-2-formamidoessigsäure und 3,26 g (0,0217 Mol) 1-Hydroxybenztriazol (Wassergehalt ca. 10 %) in 100 ml Tetrahydrofuran tropft man innerhalb von fünfzehn Minuten eine Lösung von 4,12 g (0,02 Mol) Dicyclohexylcarbodiimid in 40 ml Tetrahydrofuran. Das Reaktionsgemisch wird eine Stunde bei 0° und vierzehn Stunden bei Raumtemperatur gerührt, anschliessend filtriert

und das Nutschgut mit Tetrahydrofuran gewaschen. Nach Versetzen des Filtrats mit 60 g Kieselgel der Korngrösse 0,06 - 0,2 mm dampft man die Aufschlämmung am Rotationsverdampfer bei maximal 40° zur Trockne ein und transferiert das mit dem Substanzgemisch beladene Kieselgel auf eine mit 1 kg Kieselgel der Korngrösse 0,04 - 0,06 mm (in Aethylacetat/Hexan 2:1) beschickte Flash-Säule. Durch Elution mit einem Aethylacetat/Hexan-Gemisch (Mischungsverhältnis Aethylacetat/Hexan im Verlauf der Chromatographie zunehmend von 2:1 bis 9:1) werden nacheinander die rohe (R-Titelverbindung mit einem Rf-Wert von 0,18 (DC-Fertigplatten, Merck, Kieselgel 60 $F_{254}$, Aethylacetat/Hexan 4:1) und einem Schmelzpunkt (unter Aufschäumen) von ca. 120°, ein Gemisch der R- und der S-Titelverbindung sowie die rohe S-Titelverbindung mit einem Rf-Wert von 0,11 eluiert. Nach Umkristallisation der Mischfraktion aus Methanol/Aethylacetat wird nochmals S-Titelverbindung erhalten. Die beiden Fraktionen der durch Flash-Chromatographie und durch Umkristallisation erhaltenen S-Titelverbindung werden vereinigt und in ca. 40 ml Methanol suspendiert. Man rührt drei Stunden bei Raumtemperatur, filtriert und wäscht das Nutschgut mit wenig Methanol und Aether. Die auf diese Weise erhaltene S-Titelverbindung schmilzt bei 164 - 166° (unter Aufschäumen).

d) Der 3-[1,2,3-Thiadiazol-5-ylthiomethyl)-7β-[(2S)-2-(2-BOC-aminothiazol-4-yl)-2-formamidoacetamino]-4-cephem-4-carbonsäurediphenylmethylester kann auch folgendermassen erhalten werden:

Zu einer auf -5° gekühlten Lösung von 4,96 g (0,01 Mol) 3-(1,2,3-Thiadiazol-5-ylthiomethyl)-7β-amino-3-cemphem-4-carbonsäurediphenylmethylester, 3,01 g (0,01 Mol) (2S)-2-(2-BOC-aminothiazol-4-yl)-2-formamidoessigsäure und 1,80 g (0,012 Mol) 1-Hydroxybenztriazol (Wassergehalt ca. 10 %) in 75 ml Tetrahydrofuran tropft man innerhalb von 10 Minuten eine Lösung von 2,27 g (0,011 Mol) Dicyclohexylcarbondiimid in 25 ml Tetrahydrofuran. Das Reaktionsgemisch wird eine Stunde bei 0° und 5 Stunden bei Raumtemperatur gerührt, anschliessend filtriert und das Filtrat im Vakuum eingedampft. Man verteilt den Rückstand zwischen Aethylacetat und eiskalter wässriger Natriumbicarbonatlösung, wäscht die organische Phase mit gesättigter wässriger Natriumchloridlösung, trocknet über Natriumsulfat und dampft im Vakuum ein. Nach Umkristallisation des Rückstandes aus Methanol/Aethylacetat erhält man die Titelverbindung, Smp. 164-166° (unter Aufschäumen). Sie ist identisch mit der gemäss Beispiel 10 d) erhaltenen (S)-Titelverbindung.

Beispiel 11:
(2S)-2-(2-BOC-aminothiazol-4-yl)-2-formamidoessigsäure

Zu einer Lösung von 21,09 g (0,07 Mol) (2RS)-2-(2-BOC-aminothiazol-4-yl)-2-formamidoessigsäure in 250 ml Methanol gibt man unter Rühren bei Raumtemperatur eine Lösung von 8,48 g (0,07 Mol) S(-)-1-Phenyläthylamin in 50 ml Methanol. Bereits nach ca. 2 Minuten setzt Kristallisation ein, worauf man den Kristallbrei noch eine halbe Stunde bei Raumtemperatur weiterrührt. Nach dem Abnutschen und zweimaligen Waschen des Filterrückstandes mit je 50 ml Methanol wird das rohe S(-)-1-Phenyl-äthylammoniumsalz der (2S)-2-(2-BOC-aminothiazol-4-yl)-2-formamidoessigsäure in 400 ml Aethanol suspendiert und die Suspension 45 Minuten unter Rückfluss gekocht. Man kühlt auf Raumtemperatur, filtriert und wäscht mit 100 ml kaltem Aethanol. Der Rückstand wird noch einmal in 400 ml Aethanol suspendiert, 15 Minuten unter Rückfluss gekocht, nach Abkühlen auf Raumtemperatur filtriert und mit 100 ml kaltem Aethanol gewaschen. Das bei 60° im Hochvakuum getrocknete S(-)-1-Phenyl-äthylammonium (2S)-2-(2-BOC-aminothiazol-4-yl)-2-formamidoacetat schmilzt unter Zersetzung bei 201-202°.

11,5 g (0,027 Mol) dieses Salzes werden unter starkem Rühren zwischen einem Gemisch von 186 ml Wasser plus 37 ml 1N Salzsäure und 310 ml

eines Gemischs von Aethylacetat/Methanol (9:1) verteilt. Man trennt die organische Phase ab und wäscht die wässrige Phase dreimal mit je 50 ml Aethylacetat. Die vereinigten organischen Phasen werden mit 50 ml gesättigter wässriger Natriumchloridlösung gewaschen, über Natriumsulfat getrocknet und im Vakuum eingedampft. Den Rückstand kristallisiert man zweimal aus je 200 ml Acetonitril aus. Nach Trocknen bei 60° im Hochvakuum schmilzt die Titelverbindung unter Zersetzung bei 115-120°; [α] = +169,9 ± 1,8 ° (0,562 % in Methanol).

Beispiel 12:

a) 3-(1H-1,2,3-Triazol-4(5)-ylthiomethyl)-7β-[(2R,S)-2-(2-aminothiazol-4-yl)-2-formamidoacetamido]-3-cephem-4-carbonsäurenatriumsalz

0,53 g (0,695 mMol) 3-(1H-1,2,3-Triazol-4(5)-ylthiomethyl)-7β-[(2R,S)-2-(2-BOC-aminothiazol-4-yl)-2-formamidoacetamido]-3-cephem-4-carbonsäurediphenylmethylester werden in einem Gemisch von 2 ml Anisol und 10 ml Trifluoressigsäure eine halbe Stunde bei Raumtemperatur gerührt. Man dampft dann das Reaktionsgemisch bei maximal 30° im Vakuum ein. Nach Zugabe von ca. 30 ml Aether zum öligen Rückstand bildet sich beim Rühren ein Niederschlag, den man abfiltriert, mit Aether wäscht, trocknet, und in einem Gemisch von 4 ml Methanol und 2 ml Wasser suspendiert. Die Suspension wird mit 1 N Natriumhydroxid-Lösung auf einen pH-Wert von 7,2 eingestellt, die dabei gebildete Lösung im Vakuum eingeengt und das Konzentrat an einem Adsorberharz, z.B. Amberlite ER-180®-Adsorberharz, chromatographiert, wobei man zunächst mit Wasser, dann mit einem Wasser/Isopropanolgemisch (97,5:2,5) eluiert. Die produkthaltigen Fraktionen werden vereinigt, im Vakuum eingeengt und im Hochvakuum lyophilisiert. Die erhaltene Titelverbindung a) enthält 6,2 % Wasser; [α] = -27° ± 1° (0,537 % in $H_2O$); IR (Nujol): 3305, 3192, 1760, 1670, 1600, 1518.

b) 3-(1H-1,2,3-Trizaol-4(5)-ylthiomethyl)-7β-[(2R,S)-2-(2-BOC-aminothiazol-4-yl)-2-formamidoacetamido]-3-cephem-4-carbonsäurediphenylmethylester

Zu einer auf -5° gekühlten Lösung von 0,301 g (0,001 Mol) (2R,S)-2-(2-BOC-aminothiazol-4-yl)-2-formamidoessigsäure, 0,48 g (0,001 Mol) 3-(1H-1,2,3-Triazol-4(5)-ylthiomethyl)-7β-amino-3-cephem-4-carbonsäure-diphenylmethylester und 0,18 g (0,0012 Mol) 1-Hydroxybenztriazol (Wassergehalt ca. 10 %) in 7,5 ml Tetrahydrofuran tropft man eine Lösung von 0,226 g (0,0011 Mol) Dicyclohexylcarbodiimid in 5 ml Tetrahydrofuran. Das Reaktionsgemisch wird eine Stunde bei 0° und fünf Stunden bei Raumtemperatur gerührt, anschliessend filtriert und das Filtrat im Vakuum eingedampft. Man nimmt den Rückstand in Aethylacetat auf, wäscht die organische Phase mit wässriger Natriumbicarbonat-Lösung und gesättigter, wässriger Natriumchlorid-Lösung, trocknet über Natriumsulfat und dampft im Vakuum ein. Man reinigt den Rückstand mittels Flash-Chromatographie an Kieselgel der Korngrösse 0,04 - 0,063 mm, wobei durch Elution mit Aethylacetat die Titelverbindung b) als Rohprodukt erhalten wird.

Beispiel 13:

a) 3-Azidomethyl-7β-[(2R,S)-2-(2-aminothiazol-4-yl)-2-formamido-acetamido]-3-cephem-4-carbonsäurenatriumsalz

Analog Beispiel 12a) erhält man ausgehend von 0,4 g 3-Azidomethyl-7β-[(2R,S)-2-(2-BOC-aminothiazol-4-yl)-2-formamidoacetamido]-3-cephem-4-carbonsäurediphenylmethylester die Titelverbindung a) mit einem Wassergehalt von 6,2 %; [α] = +76 ± 1° (0,415 % in $H_2O$); IR (Nujol): 3310, 3195, 2112, 1765, 1672, 1610, 1520.

b) 3-Azidomethyl-7β-[(2R)-2-(2-aminothiazol-4-yl)-2-formamidoacet-amido]-3-cephem-4-carbonsäurenatriumsalz

Analog Beispiel 12a) erhält man ausgehend von 0,8 g 3-Azidomethyl-7β-[(2R)-2-(2-BOC-aminothiazol-4-yl)-2-formamido-acetamido]-3-cephem-4-carbonsäurediphenylmethylester die Titelverbindung b) mit einem Wassergehalt von 3,8 %; [α] = +34 ± 1° (0,494 % in $H_2O$); IR (Nujol) : 3305, 3192, 2110, 1763, 1670, 1605, 1518.

c) 3-Azidomethyl-7β-[(2S)-2-(2-aminothiazol-4-yl)-2-formamidoacetamido]-3-cephem-4-carbonsäurenatriumsalz

Analog Beispiel 12a) erhält man ausgehend von 0,8 g 3-Azidomethyl-7β-[(2S)-2-(2-BOC-aminothiazol-4-yl)-2-formamidoacetamido]-3-cephem-4-carbonsäurediphenylmethylester die Titelverbindung c) mit einem Wassergehalt von 7,3 %; [α] = +100 ± 1° (0,517 % in $H_2O$); IR (Nujol): 3305, 3190, 2110, 1762, 1668, 1606, 1516.

d) 3-Azidomethyl-7β-[(2R)-2-(2-BOC-aminothiazol-4-yl)-2-formamidoacetamido]-3-cephem-4-carbonsäurediphenylmethylester und 3-Azidomethyl-7β-[(2S)-2-(2-BOC-aminothiazol-4-yl)-2-formamidoacetamido]-3-cephem-4-carbonsäurediphenylmethylester

Analog Beispiel 12b) setzt man 2,14 g (0,0071 Mol) (2R,S)-2-(2-BOC-aminothiazol-4-yl)-2-formamidoessigsäure mit 3 g (0,0071 Mol) 3-Azidomethyl-7β-amino-3-cephem-4-carbonsäure-diphenylmethylester um. Die Auftrennung des gebildeten Diastereomerengemischs erfolgt mittels Flash-Chromatographie an Kieselgel der Korngrösse 0.04- 0.063 mm, wobei durch Elution mit einem Hexan/Aethylacetat-Gemisch (Mischungsverhältnis Hexan/Aethylacetat im Verlauf der Chromatographie abnehmend von 2:1 bis 1:4) nacheinander die (R)-Titelverbindung, Smp. 151 - 160° (Zers.) und die (S)-Titelverbindung, Smp. 161 - 166°(Zers.) als Rohprodukte erhalten werden.

e) 3-Azidomethyl-7β-[(2R,S)-2-(2-BOC-aminothiazol-4-yl)-2-formamidoacetamido]-3-cephem-4-carbonsäurediphenylmethylester

Analog Beispiel 12b) setzt man 0,461 g (0,00153 Mol) (2R,S)-2-(2-BOC-aminothiazol-4-yl)-2-formamidoessigsäure mit 0,645 g (0,00153 Mol) 3-Azidomethyl-7β-amino-3-cephem-4-carbonsäurediphenylmethylester um. Die Reinigung des Produkts erfolgt mittels Flash-Chromatographie an

Kieselgel der Korngrösse 0,04 - 0,063 mm, wobei durch Elution mit Aethylacetat/Hexan (2:1) die Titelverbindung e) als Rohprodukt erhalten wird.

Beispiel 14:

3-(2,3-Cyclopentenopyridiniomethyl)-7β-[(2R,S)-2-(2-aminothiazol-4-yl)-2-formamidoacetamido]-3-cephem-4-carboxylat

Zu einer Lösung von 0,477 g (0,001 Mol) 3-Acetoxymethyl-7β-[(2R)-2-(2-aminothiazol-4-yl)-2-formamidoacetamido]-3-cephem-4-carbonsäurenatriumsalz und 2,25 g (0,015 Mol) Natriumjodid in 2 ml Wasser gibt man 0,238 g (0,002 Mol) 2,3-Cyclopentenopyridin. Das Reaktionsgemisch wird 2,5 Stunden bei 75° gerührt und anschliessend an einem Adsorberharz, z.B. Amberlite ER-180®-Adsorberharz, chromatographiert, wobei als Eluiermittel Wasser und ein Gemisch Wasser/Isopropanol (19:1) verwendet werden. Die produkthaltigen Fraktionen werden vereinigt, im Vakuum eingeengt und im Hochvakuum lyophilisiert. Die erhaltene Titelverbindung enthält 10,0 % Wasser; IR(Nujol®):3305, 3200, 1769, 1666, 1615, 1515.

Beispiel 15: Analog Beispiel 1 - 14 werden folgende Verbindungen erhalten:

12.1. 7β-[(2R,S)-2-(2-Aminooxazol-4-yl)-2-formamidoacetamido]-3-cephem-4-carbonsäurenatriumsalz, IR(Nujol): 3300, 3195, 1760, 1658, 1602, 1520.

12.2. 3-(1H-1,2,3-Triazol-4(5)-ylthiomethyl)-7β-[(2R,S)-2-(2-aminooxazol-4-yl)-2-formamidoacetamido]-3-cephem-4-carbonsäurenatriumsalz, IR(Nujol): 3310, 3195, 1760, 1662, 1600, 1515.

12.3. 3-(1,2,3-Thiazol-5-ylthiomethyl)-7β-[(2R,S)-2-(2-aminooxazol-4-yl)-2-formamidoacetamido]-3-cephem-4-carbonsäurenatriumsalz, IR(Nujol): 3305, 3190, 1762, 1660, 1605, 1517.

12.4. 3-(2-Methyl-1,3,4-thiazol-5-ylthiomethyl)-7β-[(2R,S)-2-(2-aminooxazol-4-yl)-2-formamidoacetamido]-3-cephem-4-carbonsäure-natriumsalz, IR(Nujol): 3315, 3200, 1765, 1668, 1610, 1522.

12.5. 3-(2-Methyl-1,3,4-thiadiazol-5-ylthiomethyl)-7β-[(2R,S)-2-(2-aminothiazol-4-yl)-2-formamidoacetamido]-3-cephem-4-carbonsäurenatriumsalz, IR(Nujol): 3310, 3197, 1760, 1665, 1602, 1515.

12.6. 3-(1-Carboxymethyl-1H-tetrazol-5-ylthiomethyl)-7β-[(2R,S)-2-(2-aminooxazol-4-yl)-2-formamidacetamido]-3-cephem-4-carbonsäuredinatriumsalz, IR(Nujol): 3320, 3202, 1768, 1610, 1522.

12.7. 3-(1-Carboxymethyl-1H-tetrazol-5-ylthiomethyl)-7β-[(2R,S)-2-(2-aminothiazol-4-yl)-2-formamidoacetamido]-3-cephem-4-carbonsäuredinatriumsalz, IR(Nujol): 3315, 3198, 1766, 1665, 1608, 1520.

12.8. 3-(1-Sulfomethyl-1H-tetrazol-5-ylthiomethyl)-7β-[(2R,S)-2-(2-aminooxazol-4-yl)-2-formamidoacetamido]-3-cephem-4-carbonsäuredinatriumsalz, IR(Nujol): 3315, 3200, 1765, 1668, 1605, 1520.

12.9. 2-(1-Sulfomethyl-1H-tetrazol-5-ylthiomethyl)-7β-[(2R,S)-2-(2-aminothiazol-4-yl)-2-formamidoacetamido]-3-cephem-4-carbonsäuredinatriumsalz, IR(Nujol): 3312, 3200, 1765, 1670, 1608, 1522.

12.10. 3-(3-Brompyridiniomethyl)-7β-[(2R,S)-2-(2-aminooxazol-4-yl)-2-formamidoacetamido]-3-cephem-4-carboxylat, IR(Nujol): 3310, 3195, 1765, 1665, 1608, 1515.

12.11. 3-(3-Brompyridiniomethyl)-7β-[(2R,S)-2-(2-aminothiazol-4-yl)-2-formamidoacetamido]-3-cephem-4-carboxylat, IR(Nujol): 3315, 3198, 1769, 1664, 1610, 1515.

12.12.3-(4-Carbamoylpyridiniomethyl)-7β-[2R,S)-2-(2-aminooxazol-4-yl)-2-formamidoacetamido]-3-cephem-4-carboxylat, IR(Nujol): 3320, 3200, 1765, 1665, 1605, 1518.

12.13.3-(4-Carbamoylpyridiniomethyl)-7β-[(2R,S)-2-(2-aminothiazol-4-yl)-2-formamidoacetamido]-3-cephem-4-carboxylat, IR(Nujol): 3315, 3198, 1765, 1667, 1610, 1515.

12.14. 7β-[(2R,S)-2-(5-amino-1,2,4-thiazol-3-yl)-2-formamidoacetamido]-3-cephem-4-carbonsäurenatriumsalz, IR(Nujol): 3308, 3202, 1755, 1663, 1595, 1517.

12.15.3-(2,3-Cyclopentenopyridiniomethyl)-7β-[(2R,S)-2-(2-aminooxazol-4-yl)-2-formamidoacetamido]-3-cephem-4-carboxylat, IR(Nujol): 3310, 3200, 1768, 1660, 1612, 1518.

12.16.3-(1,2,4-Thiadiazol-5-ylthiomethyl)-7β-[(2R,S)-2-(2-aminooxazol-4-yl)-2-formamidoacetamido]-3-cephem-4-carbonsäurenatriumsalz, IR(Nujol): 3312, 3195, 1760, 1655, 1600, 1520.

12.17.3-Azidomethyl-7β-[(2R,S)-2-(2-aminooxazol-4-yl)-2-formamidoacetamido]-3-cephem-4-carbonsäurenatriumsalz, IR(Nujol): 3310, 3200, 2113, 1765, 1670, 1608, 1520,

deren R- und S-Derivate, die Carbonsäuren und physiologisch spaltbaren Ester, z.B. Pivaloyloxymethylester oder 1-Aethoxycarbonyloxyäthylester.

Beispiel 16: Trockenampullen oder Vialen enthaltend 0,5 g, z.B. 3-Acetoxymethyl-7β-[(2R,S)-2-(2-aminothiazol-4-yl)-2-formamidoacetamido]-3-cephem-4-carbonsäurenatriumsalz, kann man folgendermassen herstellen:

<u>Zusammensetzung:</u> (für 1 Ampulle oder Viale)

| | |
|---|---|
| Wirkstoff | 0,5 g |
| Mannit | 0,05 g |

Eine sterile wässrige Lösung bestehend aus Wirkstoff und Mannit wird unter aseptischen Bedingungen in 5 ml-Ampullen oder 5 ml-Vialen eingefüllt, worauf diese verschlossen und geprüft werden.

Patentansprüche (für alle benamten Verstragsstaaten ausser Oesterreich)

1. 7β-Acylamido-3-cephem-4-carbonsäure-Verbindungen der Formel

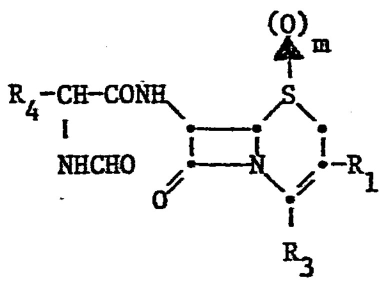

(I) ,

worin m null, eins oder zwei ist,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe, eine Azido- oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt,

und Salze von diesen Verbindungen.

2. 7β-Acylamido-3-cephem-4-carbonsäure-Verbindungen der Formel I, worin m null, eins oder zwei ist,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt, und Salze von diesen Verbindungen.

3. Verbindungen gemäss Anspruch 1 der Formel I, worin m null ist, $R_1$ Wasserstoff, Niederalkyl, Niederalkoxy, Halogen oder eine Gruppe der Formel $-CH_2-R_2$, worin $R_2$ Niederalkanoyloxy, Carbamoyloxy, unsubstituiertes oder substituiertes, monocyclisches, aromatisches oder bicyclisches, aromatisches oder partiell gesättigtes Heterocyclylthio, welches mindestens ein C-Atom enthält und mit einem seiner C-Atome mit dem Schwefel verbunden ist, Azido oder von einer tertiären, aliphatischen oder von einer aromatischen Stickstoffbase abgeleitetes Ammonio bedeutet, $R_3$ Carboxy oder unter physiologischen Bedingungen spaltbares, verestertes Carboxy und $R_4$ unsubstituiertes oder substituiertes, monocyclisches, aromatisches Oxaza-, Thiaza-, Diaza-, Thiadiaza-, Triaza-, oder Tetrazacyclyl darstellt, sowie pharmazeutisch annehmbare Salze von solchen Verbindungen mit salzbildenden Gruppen.

4. Verbindungen gemäss Anspruch 3 der Formel I, worin

m null ist und $R_1$ Wasserstoff, Niederalkyl, z.B. Methyl, Niederalkoxy, z.B. Methoxy, Halogen, z.B. Chlor, oder eine Gruppe der Formel $-CH_2-R_2$, worin $R_2$ Niederalkanoyloxy, z.B. Acetoxy, Carbamoyloxy, unsubstituiertes oder substituiertes, monocyclisches, aromatisches, fünf oder sechs Ringatome enthaltendes Heterocyclylthio mit 1-4 Stickstoffatomen oder mit einem Sauerstoff- oder Schwefelatom und gegebenenfalls 1-3 Stickstoffatomen oder bicyclisches, aromatisches, fünf oder sechs Atome pro Ring enthaltendes Heterocyclylthio mit 1-5 Stickstoffatomen und gegebenenfalls einem Sauerstoff- oder Schwefelatom, Azido oder eine von einem unsubstituierten oder substituierten aromatischen Heterocyclus mit 1-3 Stickstoffatomen und insgesamt 5 oder 6 Ringatomen gebildete Ammoniogruppe bedeutet, $R_3$ Carboxy oder Acyloxyniederalkoxycarbonyl und $R_4$ unsubstituiertes oder substituiertes Oxazolyl, Thiazolyl, Isothiazolyl, Imidazolyl, Thiadiazolyl, Triazolyl oder Tetrazolyl darstellt, sowie pharmazeutisch annehmbare Salze von solchen Verbindungen mit salzbildenden Gruppen.

5. Verbindungen gemäss Anspruch 4 der Formel I, worin m null ist und $R_1$ Wasserstoff oder eine Gruppe der Formel $-CH_2-R_2$, worin $R_2$ Niederalkanoyloxy, Carbamoyloxy, Diaza-, Triaza-, Tetraza-, Thiaza-, Thiadiaza-, Oxaza- oder Oxadiazacyclylthio, welche durch Niederalkyl, Diniederalkylaminoniederalkyl, Sulfoniederalkyl, Carboxyniederalkyl, Carbamoyl, Cyanniederalkyl, Tetrazolylniederalkyl oder durch Oxo substituiert sein können, Azido, Pyridinio, durch Niederalkyl, Carbamoyl, Niederalkylcarbamoyl, Hydroxyniederalkyl, Niederalkoxyniederalkyl, Cyanniederalkyl, Carboxyniederalkyl, Sulfoniederalkyl, Carboxyniederalkenyl, Carboxyniederalkylthio, Thiocarbamoyl, Brom, Chlor, Carboxy, Sulfo oder Cyan mono- oder disubstituiertes Pyridinio, durch Niederalkoxyiminoniederalkyl oder Niederalkylen, monosubstituiertes Pyridinio oder mit Benzol kondensiertes Pyridinio, $R_3$ Carboxy, Niederalkanoyloxyniederalkoxycarbonyl oder Niederalkoxycarbonyloxyniederalkoxycarbonyl, $R_4$ Aminooxazolyl, Aminothiazolyl, Aminothiadiazolyl, Hydroxythiadiazolyl oder Aminotriazolyl bedeutet, und pharmazeutisch verwendbare Salze von solchen Verbindungen.

6. Verbindungen gemäss Anspruch 5 der Formel I, worin m null ist und $R_1$ Wasserstoff oder eine Gruppe der Formel $-CH_2-R_2$, worin $R_2$ Niederalkanoyloxy, z.B. Acetoxy, Carbamoyloxy, Imidazolylthio, Triazolylthio, durch Niederalkyl substituiertes Triazolylthio, Tetrazolylthio, durch Niederalkyl, Diniederalkylaminoniederalkyl, Sulfoniederalkyl, Carboxyniederalkyl, Carbamoyl oder durch Tetrazolylniederalkyl substituiertes Tetrazolylthio, Thiazolylthio, durch Carboxyniederalkyl, und/oder Niederalkyl substituiertes Thiazolylthio, Isothiazolylthio, Thiadiazolylthio, durch Niederalkyl substituiertes Thiadiazolylthio, Thiatriazolylthio, Oxazolylthio, durch Niederalkyl substituiertes Oxazolylthio, durch Niederalkyl substituiertes Isoxazolylthio, Oxadiazolylthio, durch Niederalkyl substituiertes Oxadiazolylthio, durch Niederalkyl substituiertes 5,6-Dioxotetrahydro-as-triazin-3-

ylthio, Azido, Pyridinio, durch Hydroxyniederalkyl,Carboxy,Carboxyniederalkyl, Halogen, Carbamoyl oder Niederalkylen substituiertes oder mit Benzol kondensiertes Pyridinio, 2,3-Cyclopentenopyridinio oder Chinolinio, $R_3$ Carboxy, Niederalkanoyloxyniederalkoxycarbonyl oder Niederalkoxycarbonyloxyniederalkoxycarbonyl, und $R_4$ Aminooxazolyl, Aminothiazolyl, Aminothiadiazolyl oder Aminotriazolyl bedeutet, Stereoisomere, Mischungen von diesen Stereoisomeren, Hydrate und pharmazeutisch verwendbare Salze von solchen Verbindungen.

7. Verbindungen gemäss Anspruch 6 der Formel I, worin m null ist und $R_1$ Wasserstoff oder eine Gruppe der Formel $-CH_2-R_2$, worin $R_2$ Acetoxy, Carbamoyloxy, 1H-1,2,3-Triazol-4(5)-ylthio, 1H-Tetrazol-5-ylthio, 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, 1-Carboxymethyl-1H-tetrazol-5-ylthio, 1,2,3-Thiadiazol-4- oder -5-ylthio, 1,3,4-Thiadiazol-2-ylthio, 1,2,4-Thiadiazol-3- oder -5-ylthio, 1,2,5-Thiadiazol-3-ylthio, 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, Azido, Pyridinio, 3- oder 4-Hydroxymethylpyridinio, 4-Carboxypyridinio, 3- oder 4-Carboxymethylpyridinio, 3- oder 4-Chlorpyridinio, 3- oder 4-Brompyridinio, 3- oder 4-Carbamoylpyridinio, 2,3-Cyclopentenopyridinio oder Chinolinio bedeutet, $R_3$ Carboxy, Pivaloyloxymethoxycarbonyl, 1-Propionyloxyäthoxycarbonyl, 1-Aethoxycarbonyloxyäthoxycarbonyl oder tert-Butoxycarbonyloxymethoxycarbonyl, und $R_4$ 2-Aminooxazol-4-yl, 2-Aminothiazol-4-yl, 5-Amino-1,2,4-thiadiazol-3-yl bedeutet, Stereoisomere, Mischungen von diesen Stereoisomeren, Hydrate und pharmazeutisch verwendbare Salze von solchen Verbindungen.

8. 3-Acetoxymethyl-7β-[(2R,S)-2-(2-amino-4-oxazolyl)-2-formamidoacetamido]-3-cephem-4-carbonsäurenatriumsalz, gemäss Anspruch 7.

9. 3-Acetoxymethyl-7β-[(2R,S)-2-(2-aminothiazol-3-yl)-2-formamidoacetamido]-3-cephem-4-carbonsäurenatriumsalz, gemäss Anspruch 7.

10. 3-(1-Methyl-1H-tetrazol-5-ylthiomethyl)-7β-[(2R,S)-2-(2-aminothiazol-4-yl)-2-formamidoacetamido]-3-cephem-4-carbonsäurenatriumsalz, gemäss Anspruch 7.

11. 3-(1,2,3-Thiadiazol-5-ylthiomethyl)-7β-[(2S)-2-(2-aminothiazol-4-yl)-2-formamidoacetamido]-3-cephem-4-carbonsäurenatriumsalz, gemäss Anspruch 7.

12. Pharmazeutische Präparate enthaltend Verbindungen der Formel I, Hydrate oder pharmazeutisch verwendbare Salze von Verbindungen der Formel I mit salzbildenden Gruppen und Zusatzstoffe.

13. Verbindungen der Formel I, Hydrate oder pharmazeutisch verwendbare Salze von Verbindungen der Formel I zur Anwendung in einem Verfahren zur therapeutischen Behandlung des menschlichen oder tierischen Körpers.

14. Verbindungen der Formel I, Hydrate oder pharmazeutisch verwendbare Salze von Verbindungen der Formel I als antibiotisch wirksame Heilmittel.

15. Verwendung von Verbindungen der Formel I, Hydrate oder pharmazeutisch verwendbaren Salzen von Verbindungen der Formel I zur Herstellung von pharmazeutischen Präparaten.

16. Verfahren zur Herstellung von Verbindungen der Formel I, worin m, $R_1$, $R_3$ und $R_4$ die im Anspruch 1 genannten Bedeutungen haben, Stereoisomeren, Mischungen von diesen Steroisomeren, Hydraten und Salzen, dadurch gekennzeichnet, dass man

a) in einer Verbindung der Formel

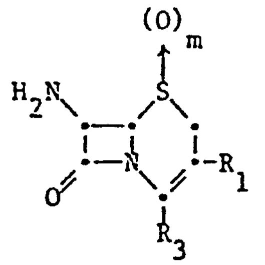

(II),

worin m und $R_1$ die unter Formel I genannten Bedeutungen haben, $R_3$ geschütztes Carboxy bedeutet, eine in $R_1$ vorhandene funktionelle Gruppe geschützt ist und die 7β-Aminogruppe gegebenenfalls durch eine die Acylierungsreaktion erlaubende Gruppe geschützt ist, die 7β-Aminogruppe durch Umsetzung mit einem den Acylrest einer Carbonsäure der Formel

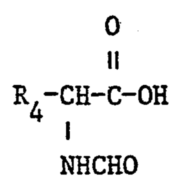

(III) ,

einführenden Acylierungsmittel, worin $R_4$ die unter Formel I genannte Bedeutung hat, und eine in $R_4$ vorhandene funktionelle Gruppe gegebenenfalls in geschützter Form vorliegt, acyliert oder

b) in einer Verbindung der Formel

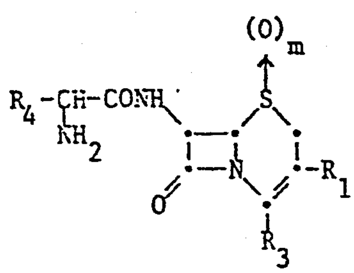

(IV) ,

worin m, $R_1$, $R_3$ und $R_4$ die unter Formel I genannten Bedeutungen haben, und eine in $R_1$ und/oder $R_4$ vorhandene funktionelle Gruppe gegebenenfalls geschützt ist, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt oder

c) eine 2-Cephem-Verbindung der Formel

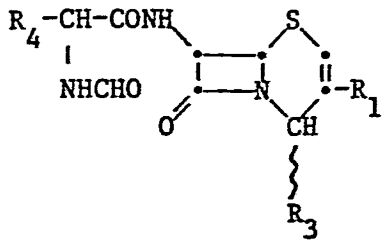

(V) ,

worin $R_1$, $R_3$ und $R_4$ die unter Formel I genannten Bedeutungen haben und eine in $R_1$ und/oder $R_4$ vorhandene funktionelle Gruppe gegebenenfalls in geschützter Form vorliegt, zur entsprechenden 3-Cephem-Verbindung der Formel I isomerisiert oder

d) zur Herstellung einer Verbindung der Formel I, worin $R_4$ 2-Amino-oxazol-4-yl oder 2-Aminothiazol-4-yl bedeutet, eine Verbindung der Formel

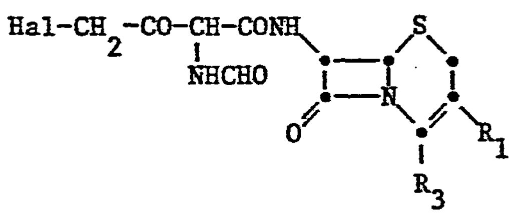

(VI),

worin Hal Halogen bedeutet, $R_1$ und $R_3$ die unter Formel I genannten Bedeutungen haben und eine in $R_1$ vorhandene funktionelle Gruppe gegebenenfalls geschützt ist, mit Harnstoff bzw. Thioharnstoff kondensiert oder

e) zur Herstellung einer Verbindung der Formel I, worin $R_4$ 5-Amino-1,2,4-thiadiazol-3-yl bedeutet, eine Verbindung der Formel:

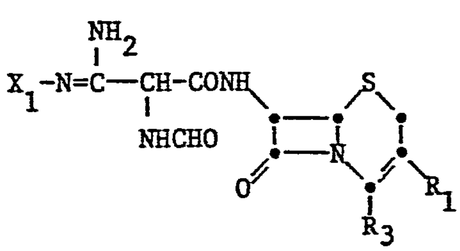

(VII),

worin $X_1$ Wasserstoff, Halogen oder Hydroxy bedeutet, $R_1$ und $R_3$ die unter Formel I genannten Bedeutungen haben und eine in $R_1$ vorhandene

funktionelle Gruppe gegebenenfalls geschützt ist, mit einem Salz der Rhodanwasserstoffsäure oder, falls $X_1$ Wasserstoff ist, mit Dirhodan umsetzt oder

f) zur Herstellung einer Verbindung der Formel I, worin $R_1$ eine Gruppe der Formel $-CH_2-R_2$ darstellt und $R_2$ veräthertes Mercapto bedeutet, eine Verbindung der Formel II, worin m und $R_3$ die unter Formel I genannten Bedeutungen haben, und $R_1$ die Gruppe der Formel $-CH_2-R_2$ darstellt, worin $R_2$ eine nukleofuge Abgangsgruppe bedeutet, mit einer Verbindung der Formel

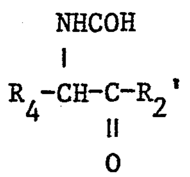

(VIII),

worin $R_4$ die unter Formel I genannten Bedeutungen hat, und $R_2'$ Niederalkylthio oder Heterocyclylthio bedeutet, umsetzt und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

17. Die nach dem Verfahren gemäss Anspruch 16 erhältlichen Verbindungen.

18. Verbindungen der Formel III, worin $R_4$ die unter in Anspruch 1, Formel I, genannten Bedeutungen hat.

19. Verfahren zur Herstellung von Verbindungen der Formel III, worin $R_4$ die in Anspruch 1 unter Formel I genannten Bedeutungen hat, dadurch gekennzeichnet, dass man in einem Aminosäureester der Formel

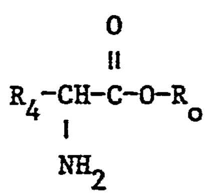 (IX) ,

worin $R_4$ die unter Formel I genannte Bedeutung hat und $R_o$ eine veresternde Gruppe darstellt und eine in $R_4$ vorhandene funktionelle Gruppe gegebenenfalls in geschützter Form vorliegt, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt und die veresternde Gruppe $R_o$ abspaltet und, wenn erwünscht, ein erhältliches Gemisch von 2R- und 2S-Verbindungen auftrennt und die 2R- oder 2S-Verbindung isoliert und/oder die erhältliche Carbonsäure in reaktionsfähige, funktionelle Derivate überführt.

Patentansprüche (für den Verstragsstaat Oesterreich)

1. Verfahren zur Herstellung von 7β-Acylamido-3-cephem-4-carbonsäure-Verbindungen der Formel

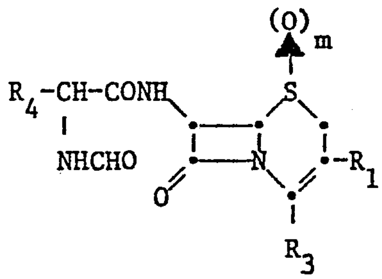

(I) ,

worin m null, eins oder zwei ist,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe, eine Azido- oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt,

und Salze von diesen Verbindungen, dadurch gekennzeichnet dass

a) in einer Verbindung der Formel

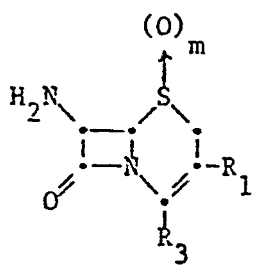

(II),

worin m und $R_1$ die unter Formel I genannten Bedeutungen haben, $R_3$ geschütztes Carboxy bedeutet, eine in $R_1$ vorhandene funktionelle Gruppe geschützt ist und die 7β-Aminogruppe gegebenenfalls durch eine die Acylierungsreaktion erlaubende Gruppe geschützt ist, die 7β-Amino-

gruppe durch Umsetzung mit einem den Acylrest einer Carbonsäure der Formel

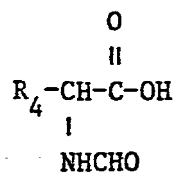

(III) ,

einführenden Acylierungsmittel, worin $R_4$ die unter Formel I genannte Bedeutung hat, und eine in $R_4$ vorhandene funktionelle Gruppe gegebenenfalls in geschützter Form vorliegt, acyliert oder

b) in einer Verbindung der Formel

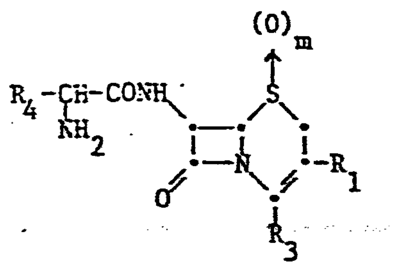

(IV) ,

worin m, $R_1$, $R_3$ und $R_4$ die unter Formel I genannten Bedeutungen haben, und eine in $R_1$ und/oder $R_4$ vorhandene funktionelle Gruppe gegebenenfalls geschützt ist, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt oder

c) eine 2-Cephem-Verbindung der Formel

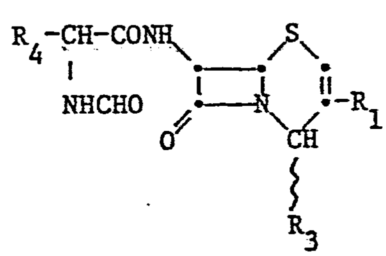

(V) ,

worin $R_1$, $R_3$ und $R_4$ die unter Formel I genannten Bedeutungen haben und eine in $R_1$ und/oder $R_4$ vorhandene funktionelle Gruppe gegebenenfalls in geschützter Form vorliegt, zur entsprechenden 3-Cephem-Verbindung der Formel I isomerisiert oder

d) zur Herstellung einer Verbindung der Formel I, worin $R_4$ 2-Amino-oxazol-4-yl oder 2-Aminothiazol-4-yl bedeutet, eine Verbindung der Formel

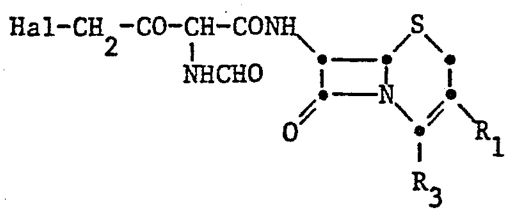

(VI),

worin Hal Halogen bedeutet, $R_1$ und $R_3$ die unter Formel I genannten Bedeutungen haben und eine in $R_1$ vorhandene funktionelle Gruppe gegebenenfalls geschützt ist, mit Harnstoff bzw. Thioharnstoff kondensiert oder

e) zur Herstellung einer Verbindung der Formel I, worin $R_4$ 5-Amino-1,2,4-thiadiazol-3-yl bedeutet, eine Verbindung der Formel:

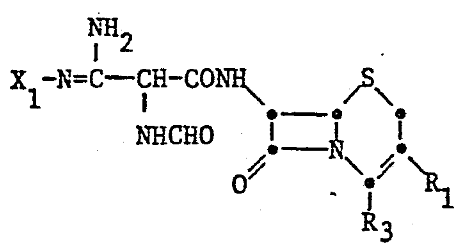

(VII),

worin $X_1$ Wasserstoff, Halogen oder Hydroxy bedeutet, $R_1$ und $R_3$ die unter Formel I genannten Bedeutungen haben und eine in $R_1$ vorhandene funktionelle Gruppe gegebenenfalls geschützt ist, mit einem Salz der Rhodanwasserstoffsäure oder, falls $X_1$ Wasserstoff ist, mit Dirhodan umsetzt oder

f) zur Herstellung einer Verbindung der Formel I, worin $R_1$ eine Gruppe der Formel $-CH_2-R_2$ darstellt und $R_2$ veräthertes Mercapto bedeutet, eine Verbindung der Formel II, worin m und $R_3$ die unter Formel I genannten Bedeutungen haben, und $R_1$ die Gruppe der Formel $-CH_2-R_2$ darstellt, worin $R_2$ eine nukleofuge Abgangsgruppe bedeutet, mit einer Verbindung der Formel

$$\begin{array}{c} NHCOH \\ | \\ R_4-CH-C-R_2' \\ \quad\;\; \| \\ \quad\;\; O \end{array} \qquad \text{(VIII),}$$

worin $R_4$ die unter Formel I genannten Bedeutungen hat, und $R_2'$ Niederalkylthio oder Heterocyclylthio bedeutet, umsetzt und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

2. Verfahren zur Herstellung von 7β-Acylamido-3-cephem-4-carbonsäure-Verbindungen der Formel I

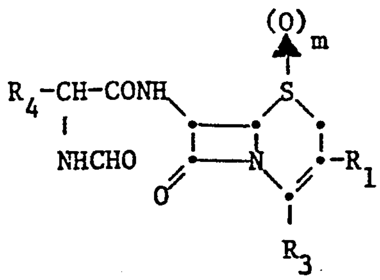

(I) ,

worin m null, eins oder zwei ist,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy, und

$R_4$ Heterocyclyl, das mit einem seiner C-Atome an das 2-C-Atom der 7β-Seitenkette gebunden ist, darstellt, und Salze von diesen von diesen Verbindungen, dadurch gekennzeichnet, dass man die in Anspruch 1 genannten Massnahmen ausführt.

3. Verfahren zur Herstellung von Verbindungen gemäss Anspruch 1 der Formel I, worin m null ist, $R_1$ Wasserstoff, Niederalkyl, Niederalkoxy, Halogen oder eine Gruppe der Formel $-CH_2-R_2$, worin $R_2$ Niederalkanoyloxy, Carbamoyloxy, unsubstituiertes oder substituiertes, monocyclisches, aromatisches oder bicyclisches, aromatisches oder partiell gesättigtes Heterocyclylthio, welches mindestens ein C-Atom enthält und mit einem seiner C-Atome mit dem Schwefel verbunden ist, Azido oder von einer tertiären, aliphatischen oder von einer aromatischen Stickstoffbase abgeleitetes Ammonio bedeutet, $R_3$ Carboxy oder unter physiologischen Bedingungen spaltbares, verestertes Carboxy und $R_4$ unsubstituiertes oder substituiertes, monocyclisches, aromatisches Oxaza-, Thiaza-, Diaza-, Thiadiaza-, Triaza-, oder Tetrazacyclyl darstellt, sowie pharmazeutisch annehmbaren Salzen von solchen Verbindungen mit

salzbildenden Gruppen, dadurch gekennzeichnet, dass man die in Anspruch 1 genannten Massnahmen ausführt.

4. Verfahren zur Herstellung von Verbindungen gemäss Anspruch 3 der Formel I, worin
m null ist und $R_1$ Wasserstoff, Niederalkyl, z.B. Methyl, Niederalkoxy, z.B. Methoxy, Halogen, z.B. Chlor, oder eine Gruppe der Formel $-CH_2-R_2$, worin $R_2$ Niederalkanoyloxy, z.B. Acetoxy, Carbamoyloxy, unsubstituiertes oder substituiertes, monocyclisches, aromatisches, fünf oder sechs Ringatome enthaltendes Heterocyclylthio mit 1-4 Stickstoffatomen oder mit einem Sauerstoff- oder Schwefelatom und gegebenenfalls 1-3 Stickstoffatomen oder bicyclisches, aromatisches, fünf oder sechs Atome pro Ring enthaltendes Heterocyclylthio mit 1-5 Stickstoffatomen und gegebenenfalls einem Sauerstoff- oder Schwefelatom, Azido oder eine von einem unsubstituierten oder substituierten aromatischen Heterocyclus mit 1-3 Stickstoffatomen und insgesamt 5 oder 6 Ringatomen gebildete Ammoniogruppe bedeutet, $R_3$ Carboxy oder Acyloxyniederalkoxycarbonyl und $R_4$ unsubstituiertes oder substituiertes Oxazolyl, Thiazolyl, Isothiazolyl, Imidazolyl, Thiadiazolyl, Triazolyl oder Tetrazolyl darstellt, sowie pharmazeutisch annehmbaren Salzen von solchen Verbindungen mit salzbildenden Gruppen, dadurch gekennzeichnet, dass man die in Anspruch 1 genannten Massnahmen ausführt.

5. Verfahren zur Herstellung von Verbindungen gemäss Anspruch 4 der Formel I, worin m null ist und
$R_1$ Wasserstoff oder eine Gruppe der Formel $-CH_2-R_2$, worin $R_2$ Niederalkanoyloxy, Carbamoyloxy, Diaza-, Triaza-, Tetraza-, Thiaza-, Thiadiaza-, Oxaza- oder Oxadiazacyclylthio, welche durch Niederalkyl Diniederalkylaminoniederalkyl, Sulfoniederalkyl, Carboxyniederalkyl, Carbamoyl, Cyanniederalkyl, Tetrazolylniederalkyl oder durch Oxo substituiert sein können, Azido, Pyridinio, durch Niederalkyl,

Carbamoyl, Niederalkylcarbamoyl, Hydroxyniederalkyl, Niederalkoxyniederalkyl, Cyanniederalkyl, Carboxyniederalkyl, Sulfoniederalkyl, Carboxyniederalkenyl, Carboxyniederalkylthio, Thiocarbamoyl, Brom, Chlor, Carboxy, Sulfo oder Cyan mono- oder disubstituiertes Pyridinio, durch Niederalkoxyiminoniederalkyl, oder Niederalkylen, monosubstituiertes Pyridinio oder mit Benzol kondensiertes Pyridinio, $R_3$ Carboxy, Niederalkanoyloxyniederalkoxycarbonyl oder Niederalkoxycarbonyloxyniederalkoxycarbonyl, $R_4$ Aminooxazolyl, Aminothiazolyl, Aminothiadiazolyl, Hydroxythiadiazolyl oder Aminotriazolyl bedeutet, und pharmazeutisch verwendbaren Salzen von solchen Verbindungen, dadurch gekennzeichnet, dass man die in Anspruch 1 genannten Massnahmen ausführt.

6. Verfahren zur Herstellung von Verbindungen gemäss Anspruch 5 der Formel I, worin m null ist und

$R_1$ Wasserstoff oder eine Gruppe der Formel $-CH_2-R_2$, worin $R_2$ Niederalkanoyloxy, z.B. Acetoxy, Carbamoyloxy, Imidazolylthio, Triazolylthio, durch Niederalkyl substituiertes Triazolylthio, Tetrazolylthio, durch Niederalkyl, Diniederalkylaminoniederalkyl, Sulfoniederalkyl, Carboxyniederalkyl, Carbamoyl oder durch Tetrazolylniederalkyl substituiertes Tetrazolylthio, Thiazolylthio, durch Carboxyniederalkyl, und/oder Niederalkyl substituiertes Thiazolylthio, Isothiazolylthio, Thiadiazolylthio, durch Niederalkyl substituiertes Thiadiazolylthio, Thiatriazolylthio, Oxazolylthio, durch Niederalkyl substituiertes Oxazolylthio, durch Niederalkyl substituiertes Isoxazolylthio, Oxadiazolylthio, durch Niederalkyl substituiertes Oxadiazolylthio, durch Niederalkyl substituiertes 5,6-Dioxotetrahydro-as-triazin-3-ylthio, Azido, Pyridinio, durch Hydroxyniederalkyl, Carboxy, Carboxyniederalkyl, Halogen, Carbamoyl oder Niederalkylen substituiertes oder mit Benzol kondensiertes Pyridinio, 2,3-Cyclopentenopyridinio oder Chinolinio, $R_3$ Carboxy, Niederalkanoyloxyniederalkoxycarbonyl oder Niederalkoxycarbonyloxyniederalkoxycarbonyl, und $R_4$ Aminooxazolyl, Aminothiazolyl, Aminothiadiazolyl oder Aminotriazolyl bedeutet,

Stereoisomeren, Mischungen von diesen Stereoisomeren, Hydraten und pharmazeutisch verwendbaren Salzen von solchen Verbindungen, dadurch gekennzeichnet, dass man die in Anspruch 1 genannten Massnahmen ausführt.

7. Verfahren zur Herstellung von Verbindungen gemäss Anspruch 6 der Formel I, worin m null ist und $R_1$ Wasserstoff oder eine Gruppe der Formel $-CH_2-R_2$, worin $R_2$ Acetoxy, Carbamoyloxy, 1H-1,2,4-Triazol-4(5)-ylthio, 1H-Tetrazol-5-ylthio, 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, 1-Carboxymethyl-1H-tetrazol-5-ylthio, 1,2,3-Thiadiazol-4- oder -5-ylthio, 1,3,4-Thiadiazol-2-ylthio, 1,2,4-Thiadiazol-3- oder -5-ylthio, 1,2,5-Thiadiazol-3-ylthio, 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, Azido, Pyridinio, 3- oder 4-Hydroxymethylpyridinio, 4-Carboxypyridinio, 3- oder 4-Carboxymethylpyridinio, 3- oder 4-Chlorpyridinio, 3- oder 4-Brompyridinio, 3- oder 4-Carbamoylpyridinio, 2,3-Cyclopentenopyridinio oder Chinolinio bedeutet, $R_3$ Carboxy, Pivaloyloxymethoxycarbonyl, 1-Propionyloxyäthoxycarbonyl, 1-Aethoxycarbonyloxyäthoxycarbonyl oder tert-Butoxycarbonyloxymethoxycarbonyl, und $R_4$ 2-Aminooxazol-4-yl, 2-Aminothiazol-4-yl, 5-Amino-1,2,4-thiadiazol-3-yl bedeutet, Stereoisomeren, Mischungen von diesen Stereoisomeren, Hydraten und pharmazeutisch verwendbaren Salzen von solchen Verbindungen, dadurch gekennzeichnet, dass man die in Anspruch 1 genannten Massnahmen durchführt.

8. Verfahren zur Herstellung von 3-Acetoxymethyl-7β-[(2R,S)-2-(2-amino-4-oxazolyl)-2-formamidoacetamido]-3-cephem-4-carbonsäure-natriumsalz, gemäss Anspruch 7, dadurch gekennzeichnet, dass man die in Anspruch 1 genannten Massnahmen durchführt.

9. Verfahren zur Herstellung von 3-Acetoxymethyl-7β-[(2R,S)-2-(2-aminothiazol-3-yl)-2-formamidoacetamido]-3-cephem-4-carbonsäure-natriumsalz, gemäss Anspruch 7, dadurch gekennzeichnet, dass man die in Anspruch 1 genannten Massnahmen durchführt.

10. Verfahren zur Herstellung von 3-(1-Methyl-1H-tetrazol-5-ylthiomethyl)-7β-[(2R,S)-2-(2-aminothiazol-4-yl)-2-formamidoacetamido]-3-cephem-4-carbonsäurenatriumsalz, gemäss Anspruch 7, dadurch gekennzeichnet, dass man die in Anspruch 1 genannten Massnahmen durchführt.

11. Verfahren zur Herstellung von 3-(1,2,3-Thiadiazol-5-ylthiomethyl)-7β-[(2S)-2-(2-aminothiazol-4-yl)-2-formamidoacetamido]-3-cephem-4-carbonsäurenatriumsalz, gemäss Anspruch 7, dadurch gekennzeichnet, dass man die in Anspruch 1 genannten Massnahmen durchführt.

12. Verfahren zur Herstellung von pharmazeutischen Präparaten enthaltend Verbindungen gemäss Anspruch 1 der Formel I, dadurch gekennzeichnet, dass man Verbindungen der Formel I oder Salz davon nach dem Verfahren gemäss Anspruch 1 herstellt und diese mit einem pharmazeutischen Trägermaterial mischt.

13. Verfahren zur Herstellung von Verbindungen der Formel III, worin $R_4$ die in Anspruch 1 unter Formel I genannten Bedeutungen hat, dadurch geknnzeichnet, dass man in einem Aminosäureester der Formel

$$R_4-\underset{\displaystyle NH_2}{\underset{|}{CH}}-\overset{\displaystyle O}{\overset{\|}{C}}-O-R_o \qquad (IX) \quad ,$$

worin $R_4$ die unter Formel I genannte Bedeutung hat und $R_o$ eine veresternde Gruppe darstellt und eine in $R_4$ vorhandene funktionelle Gruppe gegebenenfalls in geschützter Form vorliegt, und die 2-Aminogruppe gegebenenfalls durch eine die Formylierungsreaktion erlaubende Gruppe geschützt ist, die 2-Aminogruppe durch Umsetzung mit einem Formylierungsmittel in die 2-Formamidogruppe umwandelt und die veresterte Gruppe $R_o$ abspaltet und, wenn erwünscht, ein erhältliches Gemisch von 2R- und 2S-Verbindungen auftrennt und die 2R- oder 2S-Verbindung isoliert und/oder die erhältliche Carbonsäure in reaktionsfähige, funktionelle Derivate überführt.

FO 7.4/RS/mg*/